(19) **Europäisches Patentamt / European Patent Office / Office européen des brevets**

(11) **EP 4 778 939 A1**

(12) **EUROPEAN PATENT APPLICATION**
published in accordance with Art. 153(4) EPC

(43) Date of publication:
**22.07.2026 Bulletin 2026/30**

(21) Application number: **24864727.3**

(22) Date of filing: **13.09.2024**

(51) International Patent Classification (IPC):
***C07K 16/28*** (2006.01)  ***A61K 39/395*** (2006.01)

(52) Cooperative Patent Classification (CPC):
**A61K 39/395; C07K 16/28**

(86) International application number:
**PCT/CN2024/118675**

(87) International publication number:
**WO 2025/056013 (20.03.2025 Gazette 2025/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.09.2023 CN 202311177674**

(72) Inventors:
• **SHAN, Shuang**
  **Shanghai 200245 (CN)**
• **TIAN, Chenmin**
  **Shanghai 200245 (CN)**

(74) Representative: **Potter Clarkson**
  **Chapel Quarter**
  **Mount Street**
  **Nottingham NG1 6HQ (GB)**

(71) Applicants:
• **Jiangsu Hengrui Pharmaceuticals Co., Ltd.**
  **Lianyungang, Jiangsu 222047 (CN)**
• **Shanghai Hengrui Pharmaceutical Co., Ltd.**
  **Shanghai 200245 (CN)**

Remarks:
The complete document including Reference Table(s) and the Sequence Listing(s) can be downloaded from the EPO website

(54) **PHARMACEUTICAL COMPOSITION COMPRISING BISPECIFIC ANTIBODY SPECIFICALLY BINDING TO GPRC5D AND CD3**

(57) A pharmaceutical composition comprising a bispecific antibody specifically binding to GPRC5D and CD3. Specifically, the provided pharmaceutical composition comprises the bispecific antibody specifically binding to GPRC5D and CD3, and a buffer agent.

EP 4 778 939 A1

**Description**

**TECHNICAL FIELD**

The present disclosure pertains to the field of pharmaceutical formulations and particularly relates to a pharmaceutical composition comprising a bispecific antibody specifically binding to GPRC5D and CD3 and use thereof.

**BACKGROUND**

The statements herein merely provide background information related to the present disclosure and may not necessarily constitute the prior art.

Multiple myeloma (MM) is the second most common hematological malignancy, with a 5-year survival rate of about 53.9%, and the incidence of this disease is expected to increase as the global population ages.

GPRC5D, a G protein-coupled orphan receptor and a seven-transmembrane protein, is specifically highly expressed in multiple myeloma patients and is associated with patients' poor prognoses, while it is hardly expressed in most normal tissues. In addition, the expression level of GPR5CD in both newly diagnosed MM and relapsed/refractory MM patients is significantly higher than that in normal individuals. Furthermore, in various aberrant cell types, there is no difference in GPRC5D expression between patients with 1(q)gain and those with 13(q)del. This association of GPRC5D overexpression with cancer suggests the possibility of GPRC5D serving as an excellent therapeutic target for cancer.

There are currently a number of clinically available therapeutic agents for MM, including immunomodulatory agents (IMIDs), protease inhibitors (PIs), and mAb drugs. These drugs do ameliorate the clinical condition of MM patients, but each of these therapies has its drawbacks. For example, IMIDs can cause birth defects, neurotoxicity, and severe thrombosis, and PIs are associated with a high risk of peripheral neurotoxicity. mAbs against CD38 and SLAMF7 can lead to severe infections and anemia due to the reduction of erythroid and white blood cells, and resistance to these mAbs may also develop due to antigen loss. Currently, no therapy can completely cure MM, and patients will eventually relapse. Therefore, the development of a new targeted immunotherapy is of great clinical significance.

**SUMMARY**

The present disclosure provides a pharmaceutical composition comprising a bispecific antibody specifically binding to GPRC5D and CD3. The pharmaceutical composition has therapeutic activity. In addition, the pharmaceutical composition also has the advantages of good stability and the like.

In some embodiments, the present disclosure provides a pharmaceutical composition comprising a bispecific antibody specifically binding to GPRC5D and CD3 and a buffer, wherein:

the bispecific antibody specifically binding to GPRC5D and CD3 comprises one antigen-binding moiety 1 specifically binding to GPRC5D and one antigen-binding moiety 2 specifically binding to CD3, the antigen-binding moiety 1 specifically binding to GPRC5D comprises a heavy chain variable region GPRC5D-VH and a light chain variable region GPRC5D-VL, and the antigen-binding moiety 2 specifically binding to CD3 comprises a heavy chain variable region CD3-VH and a light chain variable region CD3-VL, wherein:

(i) the GPRC5D-VH comprises: a GPRC5D-HCDR1 comprising the amino acid sequence of SEQ ID NO: 11, a GPRC5D-HCDR2 comprising the amino acid sequence of SEQ ID NO: 12, and a GPRC5D-HCDR3 comprising the amino acid sequence of SEQ ID NO: 13; and the GPRC5D-VL comprises: a GPRC5D-LCDR1 comprising the amino acid sequence of SEQ ID NO: 43, 14, 41, 42, or 44, a GPRC5D-LCDR2 comprising the amino acid sequence of SEQ ID NO: 15, and a GPRC5D-LCDR3 comprising the amino acid sequence of SEQ ID NO: 16; or the GPRC5D-VH comprises: a GPRC5D-HCDR1 comprising the amino acid sequence of SEQ ID NO: 5, a GPRC5D-HCDR2 comprising the amino acid sequence of SEQ ID NO: 31, 6, or 30, and a GPRC5D-HCDR3 comprising the amino acid sequence of SEQ ID NO: 7; and the GPRC5D-VL comprises: a GPRC5D-LCDR1 comprising the amino acid sequence of SEQ ID NO: 27, 8, 26, 28, or 29, a GPRC5D-LCDR2 comprising the amino acid sequence of SEQ ID NO: 9, and a GPRC5D-LCDR3 comprising the amino acid sequence of SEQ ID NO: 10; and

(ii) the CD3-VH comprises: a CD3-HCDR1 comprising the amino acid sequence of SEQ ID NO: 51, a CD3-HCDR2 comprising the amino acid sequence of SEQ ID NO: 52, and a CD3-HCDR3 comprising the amino acid sequence of SEQ ID NO: 53; and the CD3-VL comprises: a CD3-LCDR1 comprising the amino acid sequence of SEQ ID NO: 54, a CD3-LCDR2 comprising the amino acid sequence of SEQ ID NO: 55, and a CD3-LCDR3 comprising the amino acid sequence of SEQ ID NO: 56;

the buffer is a histidine buffer, an acetate buffer, a citrate buffer, a succinate buffer, or a phosphate buffer.

In some embodiments, provided is the pharmaceutical composition according to the above, wherein the buffer is histidine-histidine hydrochloride buffer or acetic acid-sodium acetate buffer.

In some embodiments, provided is the pharmaceutical composition according to any one of the above, wherein the buffer is a histidine-histidine hydrochloride buffer.

In some embodiments, provided is the pharmaceutical composition according to any one of the above, wherein the pharmaceutical composition has a pH of 5.0 to 7.0. In some embodiments, the pharmaceutical composition has a pH of 5.0 to 6.5. In some embodiments, the pharmaceutical composition has a pH of 5.0 to 6.0. In some embodiments, the pharmaceutical composition has a pH of 5.0 to 5.5. In some embodiments, the pharmaceutical composition has a pH of 5.5 to 6.0. In some embodiments, the pharmaceutical composition has a pH of about 5.5. In some embodiments, the pharmaceutical composition has a pH of about 5.6. In some embodiments, the pharmaceutical composition has a pH of about 5.7. When a point value is referred to in the present disclosure, it should be understood that the point value includes a margin of error. This margin of error is due to factors such as laboratory environment, personnel operations, instruments, methodology, and measurement errors. Taking the pH as an example, when the measurement is about 5.5, it should be understood that a margin of error is included. As an example, when the formulation is measured using an industrial pH meter, "about 5.5" represents $5.5 \pm 0.2$ (i.e., pH of 5.3 to 5.7).

In some embodiments, the pharmaceutical composition has a pH of 5.0, 5.1, 5.2, 5.3, 5.4, 5.5, 5.6, 5.7, 5.8, 5.9, 6.0, 6.1, 6.2, 6.3, 6.4, or 6.5, or any range between these point values. In some embodiments, the pharmaceutical composition has a pH of 5.5. In some embodiments, the pharmaceutical composition has a pH of 5.6. In some embodiments, the pharmaceutical composition has a pH of 5.7.

Generally, the pH of a pharmaceutical composition obtained by buffer exchange is almost consistent with the pH of the buffer. Also, it is well known to those skilled in the art that in the process of pharmaceutical formulation, there may sometimes be a pH drift, but the pH drift of the pharmaceutical formulation is generally small (within a range of $\pm 0.3$). In some embodiments, the pH drift of the pharmaceutical formulation is within a range of $\pm 0.2$. In some embodiments, the pH drift of the pharmaceutical formulation is within a range of $\pm 0.1$. As an example, "drift at pH 5.5" means that the pH drift is within the range of $5.5 \pm 0.3$ (i.e., pH of 5.2 to 5.8), or the pH drift is within the range of $5.5 \pm 0.2$ (i.e., pH of 5.3 to 5.7), or the pH drift is within the range of $5.5 \pm 0.1$ (i.e., pH of 5.4 to 5.6).

In some embodiments, provided is the pharmaceutical composition according to any one of the above, wherein the bispecific antibody specifically binding to GPRC5D and CD3 is at a concentration of 1 mg/mL to 250 mg/mL. In some embodiments, the bispecific antibody specifically binding to GPRC5D and CD3 is at a concentration of 1 mg/mL to 200 mg/mL. In some embodiments, the bispecific antibody specifically binding to GPRC5D and CD3 is at a concentration of 1 mg/mL to 150 mg/mL. In some embodiments, the bispecific antibody specifically binding to GPRC5D and CD3 is at a concentration of 60 mg/mL to 150 mg/mL. In some embodiments, the bispecific antibody specifically binding to GPRC5D and CD3 is at a concentration of 80 mg/mL to 120 mg/mL. In some embodiments, the bispecific antibody specifically binding to GPRC5D and CD3 is at a concentration of 90 mg/mL to 110 mg/mL. In some embodiments, the bispecific antibody specifically binding to GPRC5D and CD3 is at a concentration of about 100 mg/mL. In some embodiments, the bispecific antibody specifically binding to GPRC5D and CD3 is at a concentration of 60 mg/mL to 200 mg/mL. In some embodiments, the bispecific antibody specifically binding to GPRC5D and CD3 is at a concentration of 80 mg/mL to 200 mg/mL. In some embodiments, the bispecific antibody specifically binding to GPRC5D and CD3 is at a concentration of 100 mg/mL to 200 mg/mL. In some embodiments, the bispecific antibody specifically binding to GPRC5D and CD3 is at a concentration of 120 mg/mL to 150 mg/mL. In some embodiments, the bispecific antibody specifically binding to GPRC5D and CD3 is at a concentration of about 1 mg/mL. In some embodiments, the bispecific antibody specifically binding to GPRC5D and CD3 is at a concentration of about 2 mg/mL. In some embodiments, the bispecific antibody specifically binding to GPRC5D and CD3 is at a concentration of about 5 mg/mL. In some embodiments, the bispecific antibody specifically binding to GPRC5D and CD3 is at a concentration of about 60 mg/mL. In some embodiments, the bispecific antibody specifically binding to GPRC5D and CD3 is at a concentration of about 150 mg/mL. In some embodiments, the bispecific antibody specifically binding to GPRC5D and CD3 is at a concentration of 1 mg/mL, 2 mg/mL, 5 mg/mL, 10 mg/mL, 20 mg/mL, 30 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL, 105 mg/mL, 110 mg/mL, 120 mg/mL, 130 mg/mL, 140 mg/mL, 150 mg/mL, 160 mg/mL, 170 mg/mL, 180 mg/mL, 190 mg/mL, 200 mg/mL, 210 mg/mL, 220 mg/mL, 230 mg/mL, 240 mg/mL, or 250 mg/mL, or any range between these point values. In some embodiments, the bispecific antibody specifically binding to GPRC5D and CD3 is at a concentration of 1 mg/mL. In some embodiments, the bispecific antibody specifically binding to GPRC5D and CD3 is at a concentration of 2 mg/mL. In some embodiments, the bispecific antibody specifically binding to GPRC5D and CD3 is at a concentration of 5 mg/mL. In some embodiments, the bispecific antibody specifically binding to GPRC5D and CD3 is at a concentration of 60 mg/mL. In some embodiments, the bispecific antibody specifically binding to GPRC5D and CD3 is at a concentration of 100 mg/mL. In some embodiments, the bispecific antibody specifically binding to GPRC5D and CD3 is at a concentration of 150 mg/mL.

In some embodiments, provided is the pharmaceutical composition according to any one of the above, wherein the pharmaceutical composition comprises a surfactant. In some embodiments, the surfactant is a nonionic surfactant. In some embodiments, the surfactant is selected from the group consisting of poloxamer (e.g., poloxamer 188), polysorbate (e.g., polysorbate 20 or polysorbate 80), poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocamidopropyl-betaine, linoleamidopropyl-betaine, myristamidopropyl betaine, palmitamidopropyl betaine, isostearamidopropyl-betaine, myristamidopropyl-dimethylamine, palmitamidopropyl-dimethylamine, isostearamidopropyl-dimethylamine, sodium methyl cocoyl, sodium methyl oleyl taurate, polyethylene glycol, polypropylene glycol, copolymers of ethylene and propylene glycol, and the like. In some embodiments, the surfactant is polysorbate or poloxamer. In some embodiments, the surfactant is polysorbate 80 or poloxamer 188. In some embodiments, the surfactant is polysorbate 80.

In some embodiments, provided is the pharmaceutical composition according to any one of the above, wherein the surfactant is at a concentration of 0.01 mg/mL to 8.0 mg/mL. In some embodiments, provided is the pharmaceutical composition according to any one of the above, wherein the surfactant is at a concentration of 0.01 mg/mL to 3.0 mg/mL. In some embodiments, provided is the pharmaceutical composition according to any one of the above, wherein the surfactant is at a concentration of 0.01 mg/mL to 2.5 mg/mL. In some embodiments, provided is the pharmaceutical composition according to any one of the above, wherein the surfactant is at a concentration of 0.01 mg/mL to 2.0 mg/mL. In some embodiments, provided is the pharmaceutical composition according to any one of the above, wherein the surfactant is at a concentration of 0.01 mg/mL to 1.0 mg/mL. In some embodiments, the surfactant is at a concentration of 0.1 mg/mL to 0.8 mg/mL. In some embodiments, the surfactant is at a concentration of 0.2 mg/mL to 0.8 mg/mL. In some embodiments, the surfactant is at a concentration of 0.2 mg/mL to 0.6 mg/mL. In some embodiments, the surfactant is at a concentration of 0.32 mg/mL to 0.48 mg/mL. In some embodiments, the surfactant is at a concentration of 0.36 mg/mL to 0.44 mg/mL. In some embodiments, the surfactant is at a concentration of about 0.2 mg/mL. In some embodiments, the surfactant is at a concentration of about 0.4 mg/mL. In some embodiments, the surfactant is at a concentration of about 0.6 mg/mL. In some embodiments, the surfactant is at a concentration of 0.01 mg/mL, 0.05 mg/mL, 0.1 mg/mL, 0.15 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, or 1.0 mg/mL, or any range between these point values. In some embodiments, the surfactant is at a concentration of 0.4 mg/mL.

In some embodiments, the surfactant is 0.01 mg/mL to 1.0 mg/mL polysorbate 80. In some embodiments, the surfactant is 0.1 mg/mL to 0.8 mg/mL polysorbate 80. In some embodiments, the surfactant is 0.2 mg/mL to 0.6 mg/mL polysorbate 80. In some embodiments, the surfactant is 0.32 mg/mL to 0.48 mg/mL polysorbate 80. In some embodiments, the surfactant is 0.36 mg/mL to 0.44 mg/mL polysorbate 80. In some embodiments, the surfactant is about 0.4 mg/mL polysorbate 80. In some embodiments, the surfactant is 0.2 mg/mL polysorbate 80. In some embodiments, the surfactant is 0.4 mg/mL polysorbate 80. In some embodiments, the surfactant is 0.6 mg/mL polysorbate 80.

In some embodiments, the surfactant is 0.5 mg/mL to 8.0 mg/mL PF68. In some embodiments, the surfactant is 0.5 mg/mL to 3.0 mg/mL PF68. In some embodiments, the surfactant is 2.0 mg/mL PF68.

In some embodiments, provided is the pharmaceutical composition according to any one of the above, which comprises a sugar. In some embodiments, the sugar is selected from the group consisting of conventional compositions $(CH_2O)_n$ and derivatives thereof, including monosaccharides, disaccharides, trisaccharides, polysaccharides, sugar alcohols, reducing sugars, non-reducing sugars, and the like. The sugar may be selected from the group consisting of sucrose, trehalose, glucose, lactose, fructose, maltose, dextran, glycerin, erythritol, glycerol, arabitol, sylitol, sorbitol, mannitol, melibiose, melezitose, raffinose, mannotriose, stachyose, maltose, lactulose, maltulose, glucitol, maltitol, lactitol, iso-maltulose, and the like.

In some embodiments, the sugar is sucrose, trehalose, mannitol, or sorbitol. In some embodiments, the sugar is sucrose or trehalose. In some embodiments, the sugar is sucrose.

In some embodiments, the sugar is at a concentration of 10 mg/mL to 100 mg/mL. In some embodiments, the sugar is at a concentration of 30 mg/mL to 100 mg/mL. In some embodiments, the sugar is at a concentration of 64 mg/mL to 96 mg/mL. In some embodiments, the sugar is at a concentration of 68 mg/mL to 92 mg/mL. In some embodiments, the sugar is at a concentration of 72 mg/mL to 88 mg/mL. In some embodiments, the sugar is at a concentration of about 30 mg/mL. In some embodiments, the sugar is at a concentration of about 80 mg/mL. In some embodiments, the sugar is at a concentration of about 100 mg/mL. In some embodiments, non-limiting examples of the concentration of the sugar include 10 mg/mL, 20 mg/mL, 30 mg/mL, 35 mg/mL, 37.5 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL, and any one range between these point values. In some embodiments, the sugar is at a concentration of 30 mg/mL.

In some embodiments, the sugar is at a concentration of 80 mg/mL. In some embodiments, the sugar is at a concentration of 100 mg/mL.

In some embodiments, the sugar is 10 mg/mL to 100 mg/mL sucrose. In some embodiments, the sugar is 30 mg/mL to 100 mg/mL sucrose. In some embodiments, the sugar is 64 mg/mL to 96 mg/mL sucrose. In some embodiments, the sugar is 68 mg/mL to 92 mg/mL sucrose. In some embodiments, the sugar is 72 mg/mL to 88 mg/mL sucrose. In some embodiments, the sugar is about 30 mg/mL sucrose. In some embodiments, the sugar is about 80 mg/mL sucrose. In some embodiments, the sugar is about 100 mg/mL sucrose.

In some embodiments, provided is the pharmaceutical composition according to any one of the above, wherein the buffer is at a concentration of 5 mM to 100 mM. In some embodiments, the buffer is at a concentration of 5 mM to 50 mM. In some embodiments, the buffer is at a concentration of 10 mM to 50 mM. In some embodiments, the buffer is at a concentration of 5 mM to 30 mM. In some embodiments, the buffer is at a concentration of 5 mM to 25 mM. In some embodiments, the buffer is at a concentration of 5 mM to 20 mM. In some embodiments, the buffer is at a concentration of 8 mM to 20 mM. In some embodiments, the buffer is at a concentration of 14 mM to 20 mM. In some embodiments, the buffer is at a concentration of about 5 mM. In some embodiments, the buffer is at a concentration of about 14 mM. In some embodiments, the buffer is at a concentration of about 20 mM. In some embodiments, the buffer is at a concentration of about 50 mM. In some embodiments, the buffer is at a concentration of 5 mM, 6 mM, 7 mM, 8 mM, 9 mM, 10 mM, 11 mM, 12 mM, 13 mM, 14 mM, 15 mM, 16 mM, 17 mM, 18 mM, 19 mM, 20 mM, 25 mM, 30 mM, 40 mM, 50 mM, 60 mM, 70 mM, 80 mM, 90 mM, or 100 mM, or any one range between these point values. In some embodiments, the buffer is at a concentration of 5 mM. In some embodiments, the buffer is at a concentration of 14 mM. In some embodiments, the buffer is at a concentration of 20 mM. In some embodiments, the buffer is at a concentration of 50 mM.

In some embodiments, the buffer is 5 mM to 100 mM histidine-histidine hydrochloride buffer. In some embodiments, the buffer is 5 mM to 50 mM histidine-histidine hydrochloride buffer. In some embodiments, the buffer is 5 mM to 30 mM histidine-histidine hydrochloride buffer. In some embodiments, the buffer is 5 mM to 25 mM histidine-histidine hydrochloride buffer. In some embodiments, the buffer is 5 mM to 20 mM histidine-histidine hydrochloride buffer. In some embodiments, the buffer is 8 mM to 20 mM histidine-histidine hydrochloride buffer. In some embodiments, the buffer is about 5 mM histidine-histidine hydrochloride buffer. In some embodiments, the buffer is about 14 mM histidine-histidine hydrochloride buffer. In some embodiments, the buffer is about 20 mM histidine-histidine hydrochloride buffer. In some embodiments, the buffer is about 50 mM histidine-histidine hydrochloride buffer. In some embodiments, the buffer is 5 mM histidine-histidine hydrochloride buffer. In some embodiments, the buffer is 14 mM histidine-histidine hydrochloride buffer. In some embodiments, the buffer is 20 mM histidine-histidine hydrochloride buffer. In some embodiments, the buffer is 50 mM histidine-histidine hydrochloride buffer.

In some embodiments, provided is the pharmaceutical composition according to any one of the above, wherein the pharmaceutical composition further comprises an auxiliary material. In some embodiments, the auxiliary material is an ethylenediaminetetraacetic acid disodium salt or a hydrate thereof, arginine hydrochloride, glycine, methionine, proline, histidine, phenylalanine, glutamic acid, aspartic acid, sodium chloride, or calcium chloride. In some embodiments, the auxiliary material is ethylenediaminetetraacetic acid disodium salt dihydrate (also known as "edetate disodium" or "EDTA-2Na").

In some embodiments, provided is the pharmaceutical composition according to any one of the above, wherein the auxiliary material is at a concentration of 0.01 mg/mL to 1 mg/mL. In some embodiments, the auxiliary material is at a concentration of 0.01 mg/mL to 0.8 mg/mL. In some embodiments, the auxiliary material is at a concentration of 0.01 mg/mL to 0.6 mg/mL. In some embodiments, the auxiliary material is at a concentration of 0.01 mg/mL to 0.4 mg/mL. In some embodiments, the auxiliary material is at a concentration of 0.01 mg/mL to 0.2 mg/mL. In some embodiments, the auxiliary material is at a concentration of 0.08 mg/mL to 0.12 mg/mL. In some embodiments, the auxiliary material is at a concentration of 0.09 mg/mL to 0.11 mg/mL. In some embodiments, the auxiliary material is at a concentration of about 0.01 mg/mL. In some embodiments, the auxiliary material is at a concentration of about 0.1 mg/mL. In some embodiments, the auxiliary material is at a concentration of about 1 mg/mL. In some embodiments, the auxiliary material is at a concentration of 0.01 mg/mL, 0.03 mg/mL, 0.05 mg/mL, 0.08 mg/mL, 0.09 mg/mL, 0.1 mg/mL, 0.11 mg/mL, 0.12 mg/mL, 0.13 mg/mL, 0.14 mg/mL, 0.15 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1 mg/mL, or any one range between these point values. In some embodiments, provided is the pharmaceutical composition according to any one of the above, wherein the auxiliary material is at a concentration of 0.01 mg/mL. In some embodiments, provided is the pharmaceutical composition according to any one of the above, wherein the auxiliary material is at a concentration of 0.1 mg/mL. In some embodiments, provided is the pharmaceutical composition according to any one of the above, wherein the auxiliary material is at a concentration of 1 mg/mL.

In some embodiments, provided is the pharmaceutical composition according to any one of the above, wherein the auxiliary material is 0.01 mg/mL to 1 mg/mL ethylenediaminetetraacetic acid disodium salt dihydrate. In some embodiments, the auxiliary material is 0.01 mg/mL to 0.2 mg/mL ethylenediaminetetraacetic acid disodium salt dihydrate. In some embodiments, the auxiliary material is 0.08 mg/mL to 0.12 mg/mL ethylenediaminetetraacetic acid disodium salt dihydrate. In some embodiments, the auxiliary material is 0.09 mg/mL to 0.11 mg/mL ethylenediaminetetraacetic acid disodium salt dihydrate. In some embodiments, the auxiliary material is about 0.01 mg/mL ethylenediaminetetraacetic acid disodium salt dihydrate. In some embodiments, the auxiliary material is about 0.1 mg/mL ethylenediaminetetraacetic acid disodium salt dihydrate. In some embodiments, the auxiliary material is about 1 mg/mL ethylenediaminetetraacetic acid disodium salt dihydrate. In some embodiments, the auxiliary material is 0.01 mg/mL ethylenediaminetetraacetic acid disodium salt dihydrate. In some embodiments, the auxiliary material is 0.1 mg/mL ethylenediaminetetraacetic acid disodium salt dihydrate. In some embodiments, the auxiliary material is 1 mg/mL ethylenediaminetetraacetic acid disodium salt dihydrate.

In some embodiments, provided is the pharmaceutical composition according to any one of the above, wherein in the bispecific antibody specifically binding to GPRC5D and CD3, the GPRC5D-VH comprises: a GPRC5D-HCDR1 comprising the amino acid sequence of SEQ ID NO: 11, a GPRC5D-HCDR2 comprising the amino acid sequence of SEQ ID NO: 12, and a GPRC5D-HCDR3 comprising the amino acid sequence of SEQ ID NO: 13; and the GPRC5D-VL comprises: a GPRC5D-LCDR1 comprising the amino acid sequence of SEQ ID NO: 43, a GPRC5D-LCDR2 comprising the amino acid sequence of SEQ ID NO: 15, and a GPRC5D-LCDR3 comprising the amino acid sequence of SEQ ID NO: 16; and

the CD3-VH comprises: a CD3-HCDR1 comprising the amino acid sequence of SEQ ID NO: 51, a CD3-HCDR2 comprising the amino acid sequence of SEQ ID NO: 52, and a CD3-HCDR3 comprising the amino acid sequence of SEQ ID NO: 53; and the CD3-VL comprises: a CD3-LCDR1 comprising the amino acid sequence of SEQ ID NO: 54, a CD3-LCDR2 comprising the amino acid sequence of SEQ ID NO: 55, and a CD3-LCDR3 comprising the amino acid sequence of SEQ ID NO: 56.

In some embodiments, provided is the pharmaceutical composition according to any one of the above, wherein in the bispecific antibody specifically binding to GPRC5D and CD3, the GPRC5D-VH comprises: a GPRC5D-HCDR1 comprising the amino acid sequence of SEQ ID NO: 5, a GPRC5D-HCDR2 comprising the amino acid sequence of SEQ ID NO: 31, and a GPRC5D-HCDR3 comprising the amino acid sequence of SEQ ID NO: 7; and the GPRC5D-VL comprises: a GPRC5D-LCDR1 comprising the amino acid sequence of SEQ ID NO: 27, a GPRC5D-LCDR2 comprising the amino acid sequence of SEQ ID NO: 9, and a GPRC5D-LCDR3 comprising the amino acid sequence of SEQ ID NO: 10;

the CD3-VH comprises: a CD3-HCDR1 comprising the amino acid sequence of SEQ ID NO: 51, a CD3-HCDR2 comprising the amino acid sequence of SEQ ID NO: 52, and a CD3-HCDR3 comprising the amino acid sequence of SEQ ID NO: 53; and the CD3-VL comprises: a CD3-LCDR1 comprising the amino acid sequence of SEQ ID NO: 54, a CD3-LCDR2 comprising the amino acid sequence of SEQ ID NO: 55, and a CD3-LCDR3 comprising the amino acid sequence of SEQ ID NO: 56.

In some embodiments, provided is the pharmaceutical composition according to any one of the above, wherein in the bispecific antibody specifically binding to GPRC5D and CD3, the GPRC5D-VH comprises: a GPRC5D-HCDR1 set forth in SEQ ID NO: 11, a GPRC5D-HCDR2 set forth in SEQ ID NO: 12, and a GPRC5D-HCDR3 set forth in SEQ ID NO: 13; and the GPRC5D-VL comprises: a GPRC5D-LCDR1 set forth in SEQ ID NO: 43, a GPRC5D-LCDR2 set forth in SEQ ID NO: 15, and a GPRC5D-LCDR3 set forth in SEQ ID NO: 16; and

the CD3-VH comprises: a CD3-HCDR1 set forth in SEQ ID NO: 51, a CD3-HCDR2 set forth in SEQ ID NO: 52, and a CD3-HCDR3 set forth in SEQ ID NO: 53; and the CD3-VL comprises: a CD3-LCDR1 set forth in SEQ ID NO: 54, a CD3-LCDR2 set forth in SEQ ID NO: 55, and a CD3-LCDR3 set forth in SEQ ID NO: 56.

In some embodiments, provided is the pharmaceutical composition according to any one of the above, wherein in the bispecific antibody specifically binding to GPRC5D and CD3, the GPRC5D-VH comprises: a GPRC5D-HCDR1 set forth in SEQ ID NO: 5, a GPRC5D-HCDR2 set forth in SEQ ID NO: 31, and a GPRC5D-HCDR3 set forth in SEQ ID NO: 7; and the GPRC5D-VL comprises: a GPRC5D-LCDR1 set forth in SEQ ID NO: 27, a GPRC5D-LCDR2 set forth in SEQ ID NO: 9, and a GPRC5D-LCDR3 set forth in SEQ ID NO: 10;

the CD3-VH comprises: a CD3-HCDR1 set forth in SEQ ID NO: 51, a CD3-HCDR2 set forth in SEQ ID NO: 52, and a CD3-HCDR3 set forth in SEQ ID NO: 53; and the CD3-VL comprises: a CD3-LCDR1 set forth in SEQ ID NO: 54, a CD3-LCDR2 set forth in SEQ ID NO: 55, and a CD3-LCDR3 set forth in SEQ ID NO: 56.

In some embodiments, provided is the pharmaceutical composition according to any one of the above, wherein in the bispecific antibody specifically binding to GPRC5D and CD3, the GPRC5D-HCDR1, the GPRC5D-HCDR2, the GPRC5D-HCDR3, the GPRC5D-LCDR1, the GPRC5D-LCDR2, the GPRC5D-LCDR3, the CD3-HCDR1, the CD3-HCDR2, the CD3-HCDR3, the CD3-LCDR1, the CD3-LCDR2, and the CD3-LCDR3 are defined according to the Kabat, IMGT, Chothia, AbM, or Contact numbering scheme, preferably according to the Kabat numbering scheme.

In some embodiments, provided is the pharmaceutical composition according to any one of the above, wherein in the bispecific antibody specifically binding to GPRC5D and CD3,

(i) the GPRC5D-VH comprises the amino acid sequence of SEQ ID NO: 34, 32, or 33, or an amino acid sequence having at least 90% sequence identity to SEQ ID NO: 34, 32, or 33, and the GPRC5D-VL comprises the amino acid sequence of SEQ ID NO: 39, 35, 36, 37, 38, or 40, or an amino acid sequence having at least 90% sequence identity to SEQ ID NO: 39, 35, 36, 37, 38, or 40; or

the GPRC5D-VH comprises the amino acid sequence of SEQ ID NO: 3 or an amino acid sequence having at least 90% sequence identity to SEQ ID NO: 3, and the GPRC5D-VL comprises the amino acid sequence of SEQ ID NO:

4 or an amino acid sequence having at least 90% sequence identity to SEQ ID NO: 4; the CD3-VH comprises the amino acid sequence of SEQ ID NO: 57 or an amino acid sequence having at least 90% sequence identity to SEQ ID NO: 57, and the CD3-VL comprises the amino acid sequence of SEQ ID NO: 58 or an amino acid sequence having at least 90% sequence identity to SEQ ID NO: 58; or

(ii) the GPRC5D-VH comprises the amino acid sequence of SEQ ID NO: 19, 17, or 18, or an amino acid sequence having at least 90% sequence identity to SEQ ID NO: 19, 17, or 18, and the GPRC5D-VL comprises the amino acid sequence of SEQ ID NO: 23, 20, 21, 22, 24, or 25, or an amino acid sequence having at least 90% sequence identity to SEQ ID NO: 23, 20, 21, 22, 24, or 25; or

the GPRC5D-VH comprises the amino acid sequence of SEQ ID NO: 1 or an amino acid sequence having at least 90% sequence identity to SEQ ID NO: 1, and the GPRC5D-VL comprises the amino acid sequence of SEQ ID NO: 2 or an amino acid sequence having at least 90% sequence identity to SEQ ID NO: 2; and the CD3-VH comprises the amino acid sequence of SEQ ID NO: 57 or an amino acid sequence having at least 90% sequence identity to SEQ ID NO: 57, and the CD3-VL comprises the amino acid sequence of SEQ ID NO: 58 or an amino acid sequence having at least 90% sequence identity to SEQ ID NO: 58.

In some embodiments, provided is the pharmaceutical composition according to any one of the above, wherein in the bispecific antibody specifically binding to GPRC5D and CD3,

(i) the GPRC5D-VH comprises the amino acid sequence of SEQ ID NO: 34, and the GPRC5D-VL comprises the amino acid sequence of SEQ ID NO: 39; and the CD3-VH comprises the amino acid sequence of SEQ ID NO: 57, and the CD3-VL comprises the amino acid sequence of SEQ ID NO: 58; or
(ii) the GPRC5D-VH comprises the amino acid sequence of SEQ ID NO: 19, and the GPRC5D-VL comprises the amino acid sequence of SEQ ID NO: 23; and the CD3-VH comprises the amino acid sequence of SEQ ID NO: 57, and the CD3-VL comprises the amino acid sequence of SEQ ID NO: 58.

In some embodiments, provided is the pharmaceutical composition according to any one of the above, wherein in the bispecific antibody specifically binding to GPRC5D and CD3,

(i) the amino acid sequence of the GPRC5D-VH is set forth in SEQ ID NO: 34, and the amino acid sequence of the GPRC5D-VL is set forth in SEQ ID NO: 39; and the amino acid sequence of the CD3-VH is set forth in SEQ ID NO: 57, and the amino acid sequence of the CD3-VL is set forth in SEQ ID NO: 58; or
(ii) the amino acid sequence of the GPRC5D-VH is set forth in SEQ ID NO: 19, and the amino acid sequence of the GPRC5D-VL is set forth in SEQ ID NO: 23; and the amino acid sequence of the CD3-VH is set forth in SEQ ID NO: 57, and the amino acid sequence of the CD3-VL is set forth in SEQ ID NO: 58.

In some embodiments, provided is the pharmaceutical composition according to any one of the above, wherein the antigen-binding moiety specifically binding to GPRC5D is a Fab, and the antigen-binding moiety specifically binding to CD3 is a substituted Fab; the substituted Fab results from substitution of the original CH1 and CL of a Fab with an Obscurin chain and a Titin chain, respectively; preferably, the Obscurin chain comprises the amino acid sequence of SEQ ID NO: 83, and the Titin chain comprises the amino acid sequence of SEQ ID NO: 84.

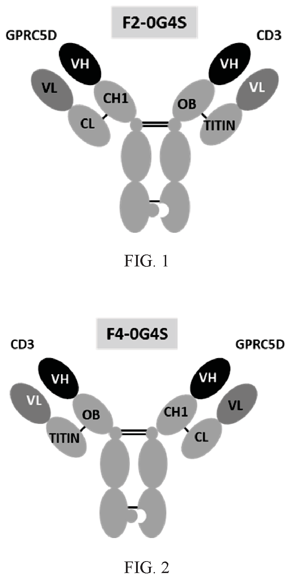

In some embodiments, provided is the pharmaceutical composition according to any one of the above, wherein the bispecific antibody specifically binding to GPRC5D and CD3 has a structure shown as formula 2-0G4S or formula 4-0G4S, wherein the structural schematic diagram of formula 2-0G4S is shown in FIG. 1, and the structural schematic diagram of formula 4-0G4S is shown in FIG. 2.

In some embodiments, provided is the pharmaceutical composition according to any one of the above, wherein the bispecific antibody specifically binding to GPRC5D and CD3 comprises one antigen-binding moiety 1 specifically binding to GPRC5D and one antigen-binding moiety 2 specifically binding to CD3, the antigen-binding moiety 1 specifically binding to GPRC5D comprises a heavy chain variable region GPRC5D-VH and a light chain variable region GPRC5D-VL, the antigen-binding moiety 2 specifically binding to CD3 comprises a heavy chain variable region CD3-VH and a light chain variable region CD3-VL, and the bispecific antibody has a structure shown in FIG. 2, wherein:

the GPRC5D-VH comprises: a GPRC5D-HCDR1 comprising the amino acid sequence of SEQ ID NO: 11, a GPRC5D-HCDR2 comprising the amino acid sequence of SEQ ID NO: 12, and a GPRC5D-HCDR3 comprising the amino acid sequence of SEQ ID NO: 13; and the GPRC5D-VL comprises: a GPRC5D-LCDR1 comprising the amino acid sequence of SEQ ID NO: 43, 14, 41, 42, or 44, a GPRC5D-LCDR2 comprising the amino acid sequence of SEQ ID NO: 15, and a GPRC5D-LCDR3 comprising the amino acid sequence of SEQ ID NO: 16; and
the CD3-VH comprises: a CD3-HCDR1 comprising the amino acid sequence of SEQ ID NO: 51, a CD3-HCDR2 comprising the amino acid sequence of SEQ ID NO: 52, and a CD3-HCDR3 comprising the amino acid sequence of SEQ ID NO: 53; and the CD3-VL comprises: a CD3-LCDR1 comprising the amino acid sequence of SEQ ID NO: 54, a CD3-LCDR2 comprising the amino acid sequence of SEQ ID NO: 55, and a CD3-LCDR3 comprising the amino acid sequence of SEQ ID NO: 56.

In some embodiments, provided is the pharmaceutical composition according to any one of the above, wherein the bispecific antibody specifically binding to GPRC5D and CD3 comprises one antigen-binding moiety 1 specifically binding to GPRC5D and one antigen-binding moiety 2 specifically binding to CD3, the antigen-binding moiety 1 specifically binding to GPRC5D comprises a heavy chain variable region GPRC5D-VH and a light chain variable region GPRC5D-VL, the antigen-binding moiety 2 specifically binding to CD3 comprises a heavy chain variable region CD3-VH and a light chain variable region CD3-VL, and the bispecific antibody has a structure shown in FIG. 1,

wherein: the GPRC5D-VH comprises: a GPRC5D-HCDR1 comprising the amino acid sequence of SEQ ID NO: 5, a GPRC5D-HCDR2 comprising the amino acid sequence of SEQ ID NO: 31, 6, or 30, and a GPRC5D-HCDR3 comprising the amino acid sequence of SEQ ID NO: 7; and the GPRC5D-VL comprises: a GPRC5D-LCDR1 comprising the amino acid sequence of SEQ ID NO: 27, 8, 26, 28, or 29, a GPRC5D-LCDR2 comprising the amino acid sequence of SEQ ID NO: 9, and a GPRC5D-LCDR3 comprising the amino acid sequence of SEQ ID NO: 10; and the CD3-VH comprises: a CD3-HCDR1 comprising the amino acid sequence of SEQ ID NO: 51, a CD3-HCDR2 comprising the amino acid sequence of SEQ ID NO: 52, and a CD3-HCDR3 comprising the amino acid sequence of SEQ ID NO: 53; and the CD3-VL comprises: a CD3-LCDR1 comprising the amino acid sequence of SEQ ID NO: 54, a CD3-LCDR2 comprising the amino acid sequence of SEQ ID NO: 55, and a CD3-LCDR3 comprising the amino acid sequence of SEQ ID NO: 56.

In some embodiments, provided is the pharmaceutical composition according to any one of the above, wherein:

the bispecific antibody specifically binding to GPRC5D and CD3 comprises one antigen-binding moiety 1 specifically binding to GPRC5D and one antigen-binding moiety 2 specifically binding to CD3, the antigen-binding moiety 1 specifically binding to GPRC5D comprises a heavy chain variable region GPRC5D-VH and a light chain variable region GPRC5D-VL, the antigen-binding moiety 2 specifically binding to CD3 comprises a heavy chain variable region CD3-VH and a light chain variable region CD3-VL, and the bispecific antibody has a structure shown in FIG. 2, wherein:

the GPRC5D-VH comprises: a GPRC5D-HCDR1 set forth in SEQ ID NO: 11, a GPRC5D-HCDR2 set forth in SEQ ID NO: 12, and a GPRC5D-HCDR3 set forth in SEQ ID NO: 13; and the GPRC5D-VL comprises: a GPRC5D-LCDR1 set forth in SEQ ID NO: 43, a GPRC5D-LCDR2 set forth in SEQ ID NO: 15, and a GPRC5D-LCDR3 set forth in SEQ ID NO: 16; and
the CD3-VH comprises: a CD3-HCDR1 set forth in SEQ ID NO: 51, a CD3-HCDR2 set forth in SEQ ID NO: 52, and a CD3-HCDR3 set forth in SEQ ID NO: 53; and the CD3-VL comprises: a CD3-LCDR1 set forth in SEQ ID NO: 54, a CD3-LCDR2 set forth in SEQ ID NO: 55, and a CD3-LCDR3 set forth in SEQ ID NO: 56.

In some embodiments, provided is the pharmaceutical composition according to any one of the above, wherein:

the bispecific antibody specifically binding to GPRC5D and CD3 comprises one antigen-binding moiety 1 specifically binding to GPRC5D and one antigen-binding moiety 2 specifically binding to CD3, the antigen-binding moiety 1 specifically binding to GPRC5D comprises a heavy chain variable region GPRC5D-VH and a light chain variable region GPRC5D-VL, the antigen-binding moiety 2 specifically binding to CD3 comprises a heavy chain variable region CD3-VH and a light chain variable region CD3-VL, and the bispecific antibody has a structure shown in FIG. 1, wherein the GPRC5D-VH comprises: a GPRC5D-HCDR1 set forth in SEQ ID NO: 5, a GPRC5D-HCDR2 set forth in SEQ ID NO: 31, and a GPRC5D-HCDR3 set forth in SEQ ID NO: 7; and the GPRC5D-VL comprises: a GPRC5D-LCDR1 set forth in SEQ ID NO: 27, a GPRC5D-LCDR2 set forth in SEQ ID NO: 9, and a GPRC5D-LCDR3 set forth in SEQ ID NO: 10;
the CD3-VH comprises: a CD3-HCDR1 set forth in SEQ ID NO: 51, a CD3-HCDR2 set forth in SEQ ID NO: 52, and a CD3-HCDR3 set forth in SEQ ID NO: 53; and the CD3-VL comprises: a CD3-LCDR1 set forth in SEQ ID NO: 54, a CD3-LCDR2 set forth in SEQ ID NO: 55, and a CD3-LCDR3 set forth in SEQ ID NO: 56.

In some embodiments, provided is the pharmaceutical composition according to any one of the above, wherein:

the bispecific antibody specifically binding to GPRC5D and CD3 comprises one antigen-binding moiety 1 specifically binding to GPRC5D and one antigen-binding moiety 2 specifically binding to CD3, the antigen-binding moiety 1 specifically binding to GPRC5D comprises a heavy chain variable region GPRC5D-VH and a light chain variable region GPRC5D-VL, the antigen-binding moiety 2 specifically binding to CD3 comprises a heavy chain variable region CD3-VH and a light chain variable region CD3-VL, and the bispecific antibody has a structure shown in FIG. 2, wherein the amino acid sequence of the GPRC5D-VH is set forth in SEQ ID NO: 34, and the amino acid sequence of the GPRC5D-VL is set forth in SEQ ID NO: 39; and
the amino acid sequence of the CD3-VH is set forth in SEQ ID NO: 57, and the amino acid sequence of the CD3-VL is set forth in SEQ ID NO: 58.

In some embodiments, provided is the pharmaceutical composition according to any one of the above, wherein the bispecific antibody specifically binding to GPRC5D and CD3 comprises one antigen-binding moiety 1 specifically binding to GPRC5D and one antigen-binding moiety 2 specifically binding to CD3, the antigen-binding moiety 1 specifically binding to GPRC5D comprises a heavy chain variable region GPRC5D-VH and a light chain variable region GPRC5D-VL, the antigen-binding moiety 2 specifically binding to CD3 comprises a heavy chain variable region CD3-VH and a light chain variable region CD3-VL, and the bispecific antibody has a structure shown in FIG. 1, wherein the amino acid sequence of the GPRC5D-VH is set forth in SEQ ID NO: 19, and the amino acid sequence of the GPRC5D-VL is set forth in SEQ ID NO: 23; and the amino acid sequence of the CD3-VH is set forth in SEQ ID NO: 57, and the amino acid sequence of the CD3-VL is set forth in SEQ ID NO: 58.

In some embodiments, provided is the pharmaceutical composition according to any one of the above, wherein:

the bispecific antibody specifically binding to GPRC5D and CD3 comprises one antigen-binding moiety 1 specifically binding to GPRC5D and one antigen-binding moiety 2 specifically binding to CD3, the antigen-binding moiety 1 specifically binding to GPRC5D comprises a heavy chain variable region GPRC5D-VH and a light chain variable region GPRC5D-VL, the antigen-binding moiety 2 specifically binding to CD3 comprises a heavy chain variable region CD3-VH and a light chain variable region CD3-VL, and the bispecific antibody has a structure shown in FIG. 2, wherein the amino acid sequence of the GPRC5D-VH is set forth in SEQ ID NO: 34, and the amino acid sequence of the GPRC5D-VL is set forth in SEQ ID NO: 39; and
the amino acid sequence of the CD3-VH is set forth in SEQ ID NO: 57, and the amino acid sequence of the CD3-VL is set forth in SEQ ID NO: 58;
the Obscurin chain comprises the amino acid sequence of SEQ ID NO: 83, and the Titin chain comprises the amino acid sequence of SEQ ID NO: 84.

In some embodiments, provided is the pharmaceutical composition according to any one of the above, wherein:

the bispecific antibody specifically binding to GPRC5D and CD3 comprises one antigen-binding moiety 1 specifically binding to GPRC5D and one antigen-binding moiety 2 specifically binding to CD3, the antigen-binding moiety 1 specifically binding to GPRC5D comprises a heavy chain variable region GPRC5D-VH and a light chain variable region GPRC5D-VL, the antigen-binding moiety 2 specifically binding to CD3 comprises a heavy chain variable region CD3-VH and a light chain variable region CD3-VL, and the bispecific antibody has a structure shown in FIG. 1, wherein the amino acid sequence of the GPRC5D-VH is set forth in SEQ ID NO: 19, and the amino acid sequence of the GPRC5D-VL is set forth in SEQ ID NO: 23; and the amino acid sequence of the CD3-VH is set forth in SEQ ID NO: 57, and the amino acid sequence of the CD3-VL is set forth in SEQ ID NO: 58;
the Obscurin chain comprises the amino acid sequence of SEQ ID NO: 83, and the Titin chain comprises the amino acid sequence of SEQ ID NO: 84.

In some embodiments, provided is the pharmaceutical composition according to any one of the above, wherein:

the bispecific antibody specifically binding to GPRC5D and CD3 comprises one first chain set forth in SEQ ID NO: 61, one second chain set forth in SEQ ID NO: 60, one third chain set forth in SEQ ID NO: 67, and one fourth chain set forth in SEQ ID NO: 68; or
comprises one first chain set forth in SEQ ID NO: 65, one second chain set forth in SEQ ID NO: 66, one third chain set forth in SEQ ID NO: 59, and one fourth chain set forth in SEQ ID NO: 60.

In some embodiments, provided is the pharmaceutical composition according to any one of the above, wherein: the bispecific antibody specifically binding to GPRC5D and CD3 comprises one first chain set forth in SEQ ID NO: 61, one second chain set forth in SEQ ID NO: 60, one third chain set forth in SEQ ID NO: 67, and one fourth chain set forth in SEQ ID NO: 68.

In some embodiments, provided is the pharmaceutical composition according to any one of the above, which comprises the following components:

(a) 1 mg/mL to 250 mg/mL said bispecific antibody specifically binding to GPRC5D and CD3,
(b) 0.01 mg/mL to 1.0 mg/mL surfactant,
(c) 10 mg/mL to 100 mg/mL sugar,
(d) 0.01 mg/mL to 1 mg/mL ethylenediaminetetraacetic acid disodium salt or a hydrate thereof, and
(e) 5 mM to 100 mM buffer, the pharmaceutical composition having a pH of 5.0 to 6.5.

In some embodiments, provided is the pharmaceutical composition according to any one of the above, which comprises the following components:

(a) 1 mg/mL to 200 mg/mL said bispecific antibody specifically binding to GPRC5D and CD3,
(b) 0.1 mg/mL to 0.8 mg/mL polysorbate 80,
(c) 30 mg/mL to 100 mg/mL sucrose,
(d) 0.01 mg/mL to 1 mg/mL ethylenediaminetetraacetic acid disodium salt dihydrate, and
(e) 5 mM to 50 mM histidine-histidine hydrochloride buffer or acetic acid-sodium acetate buffer, the pharmaceutical composition having a pH of 5.0 to 6.0.

In some embodiments, provided is the pharmaceutical composition according to any one of the above, which comprises the following components:

(a) 1 mg/mL to 150 mg/mL said bispecific antibody specifically binding to GPRC5D and CD3,
(b) 0.1 mg/mL to 1.0 mg/mL polysorbate 80,
(c) 30 mg/mL to 100 mg/mL sucrose,
(d) 0.01 mg/mL to 1 mg/mL ethylenediaminetetraacetic acid disodium salt dihydrate, and
(e) 5 mM to 50 mM histidine-histidine hydrochloride buffer, the pharmaceutical composition having a pH of 5.0 to 6.0.

In some embodiments, provided is the pharmaceutical composition according to any one of the above, which comprises the following components:

(a) 60 mg/mL to 150 mg/mL said bispecific antibody specifically binding to GPRC5D and CD3,
(b) 0.1 mg/mL to 0.8 mg/mL polysorbate 80,
(c) 30 mg/mL to 100 mg/mL sucrose,
(d) 0.01 mg/mL to 0.2 mg/mL ethylenediaminetetraacetic acid disodium salt dihydrate, and
(e) 5 mM to 50 mM histidine-histidine hydrochloride buffer or acetic acid-sodium acetate buffer, the pharmaceutical composition having a pH of 5.0 to 6.0.

In some embodiments, provided is the pharmaceutical composition according to any one of the above, which comprises the following components:

(a) 60 mg/mL to 150 mg/mL said bispecific antibody specifically binding to GPRC5D and CD3,
(b) 0.2 mg/mL to 0.6 mg/mL polysorbate 80,
(c) 64 mg/mL to 96 mg/mL sucrose,
(d) 0.01 mg/mL to 0.2 mg/mL ethylenediaminetetraacetic acid disodium salt dihydrate, and
(e) 5 mM to 30 mM histidine-histidine hydrochloride buffer or acetic acid-sodium acetate buffer, the pharmaceutical composition having a pH of 5.0 to 6.0.

In some embodiments, provided is the pharmaceutical composition according to any one of the above, which comprises the following components:

(a) 80 mg/mL to 120 mg/mL said bispecific antibody specifically binding to GPRC5D and CD3,
(b) 0.32 mg/mL to 0.48 mg/mL polysorbate 80,
(c) 64 mg/mL to 96 mg/mL sucrose,
(d) 0.08 mg/mL to 0.12 mg/mL ethylenediaminetetraacetic acid disodium salt dihydrate, and
(e) 8 mM to 20 mM histidine-histidine hydrochloride buffer, the pharmaceutical composition having a pH of 5.5 to 6.0.

In some embodiments, provided is the pharmaceutical composition according to any one of the above, which comprises the following components:

(a) 90 mg/mL to 110 mg/mL said bispecific antibody specifically binding to GPRC5D and CD3,
(b) 0.36 mg/mL to 0.44 mg/mL polysorbate 80,
(c) 72 mg/mL to 88 mg/mL sucrose,
(d) 0.09 mg/mL to 0.11 mg/mL ethylenediaminetetraacetic acid disodium salt dihydrate, and
(e) 8 mM to 20 mM histidine-histidine hydrochloride buffer, the pharmaceutical composition having a pH of 5.5 to 6.0.

In some embodiments, provided is the pharmaceutical composition according to any one of the above, which comprises the following components:

(a) about 2 mg/mL said bispecific antibody specifically binding to GPRC5D and CD3,
(b) about 0.4 mg/mL polysorbate 80,
(c) about 80 mg/mL sucrose,
(d) about 0.1 mg/mL ethylenediaminetetraacetic acid disodium salt dihydrate, and
(e) about 14 mM histidine-histidine hydrochloride buffer, the pharmaceutical composition having a pH of about 5.6.

In some embodiments, provided is the pharmaceutical composition according to any one of the above, which comprises the following components:

(a) about 60 mg/mL said bispecific antibody specifically binding to GPRC5D and CD3,
(b) about 0.4 mg/mL polysorbate 80,
(c) about 80 mg/mL sucrose,
(d) about 0.1 mg/mL ethylenediaminetetraacetic acid disodium salt dihydrate, and
(e) about 14 mM histidine-histidine hydrochloride buffer, the pharmaceutical composition having a pH of about 5.6.

In some embodiments, provided is the pharmaceutical composition according to any one of the above, which comprises the following components:

(a) about 100 mg/mL said bispecific antibody specifically binding to GPRC5D and CD3,
(b) about 0.4 mg/mL polysorbate 80,
(c) about 80 mg/mL sucrose,
(d) about 0.1 mg/mL ethylenediaminetetraacetic acid disodium salt dihydrate, and
(e) about 14 mM histidine-histidine hydrochloride buffer, the pharmaceutical composition having a pH of about 5.6.

In some embodiments, provided is the pharmaceutical composition according to any one of the above, which comprises the following components:

(a) 2 mg/mL said bispecific antibody specifically binding to GPRC5D and CD3,
(b) 0.4 mg/mL polysorbate 80,
(c) 80 mg/mL sucrose,
(d) 0.1 mg/mL ethylenediaminetetraacetic acid disodium salt dihydrate, and
(e) 14 mM histidine-histidine hydrochloride buffer, the pharmaceutical composition having a pH of 5.6.

In some embodiments, provided is the pharmaceutical composition according to any one of the above, which comprises the following components:

(a) 60 mg/mL said bispecific antibody specifically binding to GPRC5D and CD3,
(b) 0.4 mg/mL polysorbate 80,
(c) 80 mg/mL sucrose,
(d) 0.1 mg/mL ethylenediaminetetraacetic acid disodium salt dihydrate, and
(e) 14 mM histidine-histidine hydrochloride buffer, the pharmaceutical composition having a pH of 5.6.

In some embodiments, provided is the pharmaceutical composition according to any one of the above, which comprises the following components:

(a) 100 mg/mL said bispecific antibody specifically binding to GPRC5D and CD3,

(b) 0.4 mg/mL polysorbate 80,
(c) 80 mg/mL sucrose,
(d) 0.1 mg/mL ethylenediaminetetraacetic acid disodium salt dihydrate, and
(e) 14 mM histidine-histidine hydrochloride buffer, the pharmaceutical composition having a pH of 5.6.

In some embodiments, provided is the pharmaceutical composition according to any one of the above, which comprises the following components:

(a) about 2 mg/mL said bispecific antibody specifically binding to GPRC5D and CD3,
(b) about 0.4 mg/mL polysorbate 80,
(c) about 80 mg/mL sucrose,
(d) about 0.1 mg/mL ethylenediaminetetraacetic acid disodium salt dihydrate, and
(e) about 20 mM histidine-histidine hydrochloride buffer, the pharmaceutical composition having a pH of about 5.5.

In some embodiments, provided is the pharmaceutical composition according to any one of the above, which comprises the following components:

(a) about 60 mg/mL said bispecific antibody specifically binding to GPRC5D and CD3,
(b) about 0.4 mg/mL polysorbate 80,
(c) about 80 mg/mL sucrose,
(d) about 0.1 mg/mL ethylenediaminetetraacetic acid disodium salt dihydrate, and
(e) about 20 mM histidine-histidine hydrochloride buffer, the pharmaceutical composition having a pH of about 5.5.

In some embodiments, provided is the pharmaceutical composition according to any one of the above, which comprises the following components:

(a) about 100 mg/mL said bispecific antibody specifically binding to GPRC5D and CD3,
(b) about 0.4 mg/mL polysorbate 80,
(c) about 80 mg/mL sucrose,
(d) about 0.1 mg/mL ethylenediaminetetraacetic acid disodium salt dihydrate, and
(e) about 20 mM histidine-histidine hydrochloride buffer, the pharmaceutical composition having a pH of about 5.5.

In some embodiments, provided is the pharmaceutical composition according to any one of the above, which comprises the following components:

(a) 2 mg/mL said bispecific antibody specifically binding to GPRC5D and CD3,
(b) 0.4 mg/mL polysorbate 80,
(c) 80 mg/mL sucrose,
(d) 0.1 mg/mL ethylenediaminetetraacetic acid disodium salt dihydrate, and
(e) 20 mM histidine-histidine hydrochloride buffer, the pharmaceutical composition having a pH of 5.5.

In some embodiments, provided is the pharmaceutical composition according to any one of the above, which comprises the following components:

(a) 60 mg/mL said bispecific antibody specifically binding to GPRC5D and CD3,
(b) 0.4 mg/mL polysorbate 80,
(c) 80 mg/mL sucrose,
(d) 0.1 mg/mL ethylenediaminetetraacetic acid disodium salt dihydrate, and
(e) 20 mM histidine-histidine hydrochloride buffer, the pharmaceutical composition having a pH of 5.5.

In some embodiments, provided is the pharmaceutical composition according to any one of the above, which comprises the following components:

(a) 100 mg/mL said bispecific antibody specifically binding to GPRC5D and CD3,
(b) 0.4 mg/mL polysorbate 80,
(c) 80 mg/mL sucrose,
(d) 0.1 mg/mL ethylenediaminetetraacetic acid disodium salt dihydrate, and
(e) 20 mM histidine-histidine hydrochloride buffer, the pharmaceutical composition having a pH of 5.5.

In some embodiments, provided is the pharmaceutical composition according to the above, which is a liquid formulation. In some embodiments, the solvent in the liquid formulation is water.

The present disclosure further provides a freeze-dried formulation, wherein the freeze-dried formulation is capable of forming the pharmaceutical composition according to any one of the above after being reconstituted.

The present disclosure further provides a freeze-dried formulation that is a formulation in a freeze-dried form of the pharmaceutical composition according to any one of the above. The present disclosure further provides a method for preparing a freeze-dried formulation, the method comprising a step of lyophilizing the pharmaceutical composition according to any one of the above. In some embodiments, the lyophilization according to any one of the above comprises steps of pre-freezing, primary drying, and secondary drying in sequence.

The present disclosure further provides a freeze-dried formulation, wherein the formulation is obtained by lyophilizing the pharmaceutical composition according to any one of the above.

The present disclosure further provides a reconstituted solution, wherein the reconstituted solution is prepared by reconstituting the freeze-dried formulation according to any one of the above.

The present disclosure further provides a reconstituted solution that is a formulation in a reconstituted form of the freeze-dried formulation according to any one of the above.

In some embodiments, provided is the reconstituted solution according to any one of the above, the components and contents of which are identical to those of the aforementioned pharmaceutical composition.

In some embodiments, provided is the reconstituted solution according to any one of the above, which comprises the following components:

(a) 1 mg/mL to 250 mg/mL said bispecific antibody specifically binding to GPRC5D and CD3,
(b) 0.01 mg/mL to 1.0 mg/mL surfactant,
(c) 10 mg/mL to 100 mg/mL sugar,
(d) 0.01 mg/mL to 1 mg/mL ethylenediaminetetraacetic acid disodium salt or a hydrate thereof, and
(e) 5 mM to 100 mM buffer, the pharmaceutical composition having a pH of 5.0 to 6.5.

In some embodiments, provided is the reconstituted solution according to any one of the above, which comprises the following components:

(a) 1 mg/mL to 200 mg/mL said bispecific antibody specifically binding to GPRC5D and CD3,
(b) 0.1 mg/mL to 0.8 mg/mL polysorbate 80,
(c) 30 mg/mL to 100 mg/mL sucrose,
(d) 0.01 mg/mL to 1 mg/mL ethylenediaminetetraacetic acid disodium salt dihydrate, and
(e) 5 mM to 50 mM histidine-histidine hydrochloride buffer or acetic acid-sodium acetate buffer, the pharmaceutical composition having a pH of 5.0 to 6.0.

In some embodiments, provided is the reconstituted solution according to any one of the above, which comprises the following components:

(a) 1 mg/mL to 150 mg/mL said bispecific antibody specifically binding to GPRC5D and CD3,
(b) 0.1 mg/mL to 1.0 mg/mL polysorbate 80,
(c) 30 mg/mL to 100 mg/mL sucrose,
(d) 0.01 mg/mL to 1 mg/mL ethylenediaminetetraacetic acid disodium salt dihydrate, and
(e) 5 mM to 50 mM histidine-histidine hydrochloride buffer, the pharmaceutical composition having a pH of 5.0 to 6.0.

In some embodiments, provided is the reconstituted solution according to any one of the above, which comprises the following components:

(a) 60 mg/mL to 150 mg/mL said bispecific antibody specifically binding to GPRC5D and CD3,
(b) 0.1 mg/mL to 0.8 mg/mL polysorbate 80,
(c) 30 mg/mL to 100 mg/mL sucrose,
(d) 0.01 mg/mL to 0.2 mg/mL ethylenediaminetetraacetic acid disodium salt dihydrate, and
(e) 5 mM to 50 mM histidine-histidine hydrochloride buffer or acetic acid-sodium acetate buffer, the pharmaceutical composition having a pH of 5.0 to 6.0.

In some embodiments, provided is the reconstituted solution according to any one of the above, which comprises the following components:

(a) 60 mg/mL to 150 mg/mL said bispecific antibody specifically binding to GPRC5D and CD3,

(b) 0.2 mg/mL to 0.6 mg/mL polysorbate 80,

(c) 64 mg/mL to 96 mg/mL sucrose,

(d) 0.01 mg/mL to 0.2 mg/mL ethylenediaminetetraacetic acid disodium salt dihydrate, and

(e) 5 mM to 30 mM histidine-histidine hydrochloride buffer or acetic acid-sodium acetate buffer, the pharmaceutical composition having a pH of 5.0 to 6.0.

In some embodiments, provided is the reconstituted solution according to any one of the above, which comprises the following components:

(a) 80 mg/mL to 120 mg/mL said bispecific antibody specifically binding to GPRC5D and CD3,

(b) 0.32 mg/mL to 0.48 mg/mL polysorbate 80,

(c) 64 mg/mL to 96 mg/mL sucrose,

(d) 0.08 mg/mL to 0.12 mg/mL ethylenediaminetetraacetic acid disodium salt dihydrate, and

(e) 8 mM to 20 mM histidine-histidine hydrochloride buffer, the pharmaceutical composition having a pH of 5.5 to 6.0.

In some embodiments, provided is the reconstituted solution according to any one of the above, which comprises the following components:

(a) 90 mg/mL to 110 mg/mL said bispecific antibody specifically binding to GPRC5D and CD3,

(b) 0.36 mg/mL to 0.44 mg/mL polysorbate 80,

(c) 72 mg/mL to 88 mg/mL sucrose,

(d) 0.09 mg/mL to 0.11 mg/mL ethylenediaminetetraacetic acid disodium salt dihydrate, and

(e) 8 mM to 20 mM histidine-histidine hydrochloride buffer, the pharmaceutical composition having a pH of 5.5 to 6.0.

In some embodiments, provided is the reconstituted solution according to any one of the above, which comprises the following components:

(a) about 2 mg/mL said bispecific antibody specifically binding to GPRC5D and CD3,

(b) about 0.4 mg/mL polysorbate 80,

(c) about 80 mg/mL sucrose,

(d) about 0.1 mg/mL ethylenediaminetetraacetic acid disodium salt dihydrate, and

(e) about 14 mM histidine-histidine hydrochloride buffer, the pharmaceutical composition having a pH of about 5.6.

In some embodiments, provided is the reconstituted solution according to any one of the above, which comprises the following components:

(a) about 60 mg/mL said bispecific antibody specifically binding to GPRC5D and CD3,

(b) about 0.4 mg/mL polysorbate 80,

(c) about 80 mg/mL sucrose,

(d) about 0.1 mg/mL ethylenediaminetetraacetic acid disodium salt dihydrate, and

(e) about 14 mM histidine-histidine hydrochloride buffer, the pharmaceutical composition having a pH of about 5.6.

In some embodiments, provided is the reconstituted solution according to any one of the above, which comprises the following components:

(a) about 100 mg/mL said bispecific antibody specifically binding to GPRC5D and CD3,

(b) about 0.4 mg/mL polysorbate 80,

(c) about 80 mg/mL sucrose,

(d) about 0.1 mg/mL ethylenediaminetetraacetic acid disodium salt dihydrate, and

(e) about 14 mM histidine-histidine hydrochloride buffer, the pharmaceutical composition having a pH of about 5.6.

In some embodiments, provided is the reconstituted solution according to any one of the above, which comprises the following components:

(a) 2 mg/mL said bispecific antibody specifically binding to GPRC5D and CD3,

(b) 0.4 mg/mL polysorbate 80,

(c) 80 mg/mL sucrose,

(d) 0.1 mg/mL ethylenediaminetetraacetic acid disodium salt dihydrate, and
(e) 14 mM histidine-histidine hydrochloride buffer, the pharmaceutical composition having a pH of 5.6.

In some embodiments, provided is the reconstituted solution according to any one of the above, which comprises the following components:

(a) 60 mg/mL said bispecific antibody specifically binding to GPRC5D and CD3,
(b) 0.4 mg/mL polysorbate 80,
(c) 80 mg/mL sucrose,
(d) 0.1 mg/mL ethylenediaminetetraacetic acid disodium salt dihydrate, and
(e) 14 mM histidine-histidine hydrochloride buffer, the pharmaceutical composition having a pH of 5.6.

In some embodiments, provided is the reconstituted solution according to any one of the above, which comprises the following components:

(a) 100 mg/mL said bispecific antibody specifically binding to GPRC5D and CD3,
(b) 0.4 mg/mL polysorbate 80,
(c) 80 mg/mL sucrose,
(d) 0.1 mg/mL ethylenediaminetetraacetic acid disodium salt dihydrate, and
(e) 14 mM histidine-histidine hydrochloride buffer, the pharmaceutical composition having a pH of 5.6.

In some embodiments, provided is the reconstituted solution according to any one of the above, which comprises the following components:

(a) about 2 mg/mL said bispecific antibody specifically binding to GPRC5D and CD3,
(b) about 0.4 mg/mL polysorbate 80,
(c) about 80 mg/mL sucrose,
(d) about 0.1 mg/mL ethylenediaminetetraacetic acid disodium salt dihydrate, and
(e) about 20 mM histidine-histidine hydrochloride buffer, the pharmaceutical composition having a pH of about 5.5.

In some embodiments, provided is the reconstituted solution according to any one of the above, which comprises the following components:

(a) about 60 mg/mL said bispecific antibody specifically binding to GPRC5D and CD3,
(b) about 0.4 mg/mL polysorbate 80,
(c) about 80 mg/mL sucrose,
(d) about 0.1 mg/mL ethylenediaminetetraacetic acid disodium salt dihydrate, and
(e) about 20 mM histidine-histidine hydrochloride buffer, the pharmaceutical composition having a pH of about 5.5.

In some embodiments, provided is the reconstituted solution according to any one of the above, which comprises the following components:

(a) about 100 mg/mL said bispecific antibody specifically binding to GPRC5D and CD3,
(b) about 0.4 mg/mL polysorbate 80,
(c) about 80 mg/mL sucrose,
(d) about 0.1 mg/mL ethylenediaminetetraacetic acid disodium salt dihydrate, and
(e) about 20 mM histidine-histidine hydrochloride buffer, the pharmaceutical composition having a pH of about 5.5.

In some embodiments, provided is the reconstituted solution according to any one of the above, which comprises the following components:

(a) 2 mg/mL said bispecific antibody specifically binding to GPRC5D and CD3,
(b) 0.4 mg/mL polysorbate 80,
(c) 80 mg/mL sucrose,
(d) 0.1 mg/mL ethylenediaminetetraacetic acid disodium salt dihydrate, and
(e) 20 mM histidine-histidine hydrochloride buffer, the pharmaceutical composition having a pH of 5.5.

In some embodiments, provided is the reconstituted solution according to any one of the above, which comprises the following components:

(a) 60 mg/mL said bispecific antibody specifically binding to GPRC5D and CD3,
(b) 0.4 mg/mL polysorbate 80,
(c) 80 mg/mL sucrose,
(d) 0.1 mg/mL ethylenediaminetetraacetic acid disodium salt dihydrate, and
(e) 20 mM histidine-histidine hydrochloride buffer, the pharmaceutical composition having a pH of 5.5.

In some embodiments, provided is the reconstituted solution according to any one of the above, which comprises the following components:

(a) 100 mg/mL said bispecific antibody specifically binding to GPRC5D and CD3,
(b) 0.4 mg/mL polysorbate 80,
(c) 80 mg/mL sucrose,
(d) 0.1 mg/mL ethylenediaminetetraacetic acid disodium salt dihydrate, and
(e) 20 mM histidine-histidine hydrochloride buffer, the pharmaceutical composition having a pH of 5.5.

In some embodiments, provided is the pharmaceutical composition or the reconstituted solution according to any one of the above, which is a formulation for intravenous, subcutaneous, intraperitoneal, or intramuscular injection. In some embodiments, provided is the pharmaceutical composition or the reconstituted solution according to any one of the above, which is a formulation for intravenous injection. In some embodiments, provided is the pharmaceutical composition or the reconstituted solution according to any one of the above, which is a formulation for subcutaneous injection.

In some embodiments, provided is the pharmaceutical composition or the reconstituted solution according to any one of the above, which is suitable for intravenous, subcutaneous, intraperitoneal, or intramuscular injection. In some embodiments, provided is the pharmaceutical composition or the reconstituted solution according to any one of the above, which is suitable for intravenous injection. In some embodiments, provided is the pharmaceutical composition or the reconstituted solution according to any one of the above, which is suitable for subcutaneous injection.

In some embodiments, provided is the pharmaceutical composition or the reconstituted solution or the freeze-dried formulation according to any one of the above, which is used for manufacturing a medicament for intravenous, subcutaneous, intraperitoneal, or intramuscular injection. In some embodiments, provided is the pharmaceutical composition or the reconstituted solution or the freeze-dried formulation according to any one of the above, which is used for manufacturing a medicament for intravenous injection. In some embodiments, provided is the pharmaceutical composition or the reconstituted solution or the freeze-dried formulation according to any one of the above, which is used for manufacturing a medicament for subcutaneous injection.

The present disclosure further provides a kit comprising at least one container, wherein each container independently contains the pharmaceutical composition according to any one of the above, the freeze-dried formulation according to any one of the above, or the reconstituted solution according to any one of the above.

In some embodiments, the present disclosure further provides a method for diagnosing, treating, and alleviating a disorder in a subject, and the method comprises administering to the subject an effective amount of the pharmaceutical composition according to any one of the above, the freeze-dried formulation according to any one of the above, the reconstituted solution according to any one of the above, or the kit according to any one of the above.

In some embodiments, the present disclosure further provides a method for treating or preventing a disease, and the method comprises administering to a subject a therapeutically effective amount of the pharmaceutical composition according to any one of the above, the freeze-dried formulation according to any one of the above, the reconstituted solution according to any one of the above, or the kit according to any one of the above.

In some embodiments, the present disclosure further provides the pharmaceutical composition according to any one of the above, the freeze-dried formulation according to any one of the above, the reconstituted solution according to any one of the above, or the kit according to any one of the above for use in the treatment or prevention of a disease.

In one aspect, the present disclosure further provides use of the pharmaceutical composition according to any one of the above, the freeze-dried formulation according to any one of the above, the reconstituted solution according to any one of the above, or the kit according to any one of the above in the manufacture of a medicament for preventing or treating a disease.

In some embodiments, the disease according to any one of the above is selected from the group consisting of a proliferative disease (including tumors and cancer) and an autoimmune disease.

In some embodiments, the disease according to any one of the above is a hematological malignancy or a solid tumor. In some embodiments, the hematological malignancy is selected from the group consisting of multiple myeloma, relapsed/refractory multiple myeloma, smoldering multiple myeloma, monoclonal gammopathy (MGUS) (e.g., monoclonal gammopathy of undetermined diagnostic significance), acute lymphoblastic leukemia (ALL), diffuse large B-cell lymphoma (DLBCL), Burkitt's lymphoma (BL), follicular lymphoma (FL), mantle cell lymphoma (MCL), Waldenstrom's macroglobulinemia, plasma cell leukemia, light chain amyloidosis (AL), precursor B-cell lymphoblastic leukemia, acute myelogenous leukemia (AML), myelodysplastic syndrome (MDS), chronic lymphocytic leukemia (CLL), B-cell malignancy, chronic myelogenous leukemia (CML), hairy cell leukemia (HCL), blastic plasmacytoid dendritic cell neoplasm, Hodgkin's lymphoma, non-Hodgkin's lymphoma, marginal zone B-cell lymphoma (MZBL), mucosa-associated lymphoid tissue lymphoma (MALT), plasma cell leukemia, and anaplastic large cell lymphoma (ALCL). In some embodiments, the solid tumor is selected from the group consisting of ovarian cancer, lung cancer (non-small cell lung cancer and small cell lung cancer), gastric cancer, prostate cancer, renal cancer, liver cancer, colorectal cancer (such as colon cancer and rectal cancer), esophageal cancer, bladder cancer, cervical cancer, and melanoma.

In some embodiments, the disease is selected from the group consisting of multiple myeloma, relapsed/refractory multiple myeloma, smoldering multiple myeloma, monoclonal gammopathy (MGUS) (e.g., monoclonal gammopathy of undetermined diagnostic significance), acute lymphoblastic leukemia (ALL), diffuse large B-cell lymphoma (DLBCL), Burkitt's lymphoma (BL), follicular lymphoma (FL), mantle cell lymphoma (MCL), Waldenstrom's macroglobulinemia, plasma cell leukemia, light chain amyloidosis (AL), precursor B-cell lymphoblastic leukemia, acute myelogenous leukemia (AML), myelodysplastic syndrome (MDS), chronic lymphocytic leukemia (CLL), B-cell malignancy, chronic myelogenous leukemia (CML), hairy cell leukemia (HCL), blastic plasmacytoid dendritic cell neoplasm, Hodgkin's lymphoma, non-Hodgkin's lymphoma, marginal zone B-cell lymphoma (MZBL), mucosa-associated lymphoid tissue lymphoma (MALT), plasma cell leukemia, anaplastic large cell lymphoma (ALCL), ovarian cancer, lung cancer, gastric cancer, prostate cancer, renal cancer, liver cancer, colorectal cancer, esophageal cancer, bladder cancer, cervical cancer, and melanoma.

In some embodiments, the disease is multiple myeloma.

In some embodiments, the disease is relapsed/refractory multiple myeloma.

In some embodiments, the aforementioned disease is a disease associated with GPRC5D; in some embodiments, the aforementioned disease is a disease expressing GPRC5D.

## BRIEF DESCRIPTION OF THE DRAWINGS

FIG. 1 is a structural schematic diagram of formula 2-0G4S; and
FIG. 2 is a structural schematic diagram of formula 4-0G4S.

## DETAILED DESCRIPTION

**Terminology**

In order to facilitate the understanding of the present disclosure, certain technical and scientific terms are specifically defined below. Unless otherwise explicitly defined herein, all other technical and scientific terms used herein have the meanings generally understood by those of ordinary skill in the art to which the present disclosure pertains.

The three-letter and single-letter codes for amino acids used in the present disclosure are as described in J. Biol. Chem., 243, p3558 (1968).

The term "antibody" is used in the broadest sense and encompasses a variety of antibody structures, including but not limited to monoclonal antibodies, polyclonal antibodies, monospecific antibodies, multispecific antibodies (e.g., bispecific antibodies), full-length antibodies, and antibody fragments (or antigen-binding fragments, or antigen-binding moieties), as long as they exhibit the desired antigen binding activity. "Natural antibody" refers to a naturally occurring immunoglobulin molecule. For example, a natural IgG antibody is a heterotetrameric protein of about 150,000 Daltons composed of two light chains and two heavy chains linked by a disulfide bond. From the N-terminus to the C-terminus, each heavy chain comprises one variable region (VH), also known as variable heavy domain or heavy chain variable region, followed by three constant domains (CH1, CH2, and CH3). Similarly, from the N-terminus to the C-terminus, each light chain comprises one variable region (VL), also known as variable light domain or light chain variable domain, followed by one constant light domain (light chain constant region, CL). The term "bispecific antibody" refers to an antibody (including an antibody or an antigen-binding fragment thereof, such as a single-chain antibody) capable of specifically binding to two different antigens or two different antigenic epitopes of the same antigen. Bispecific antibodies of various structures have been disclosed in the prior art. The bispecific antibodies can be classified into IgG-like bispecific antibodies and antibody-fragment-type bispecific antibodies according to the integrity of IgG molecules. The bispecific antibodies can be classified into bivalent, trivalent, tetravalent, or higher-valency bispecific antibodies according to the number of the antigen-binding regions. The bispecific antibodies can be classified into symmetric bispecific antibodies and asymmetric bispecific antibodies according to whether their structures are symmetric. Among them, the fragment-type bispecific antibodies, such as Fab fragments lacking Fc fragments, are formed by combining 2 or more Fab fragments into one molecule and have relatively low immunogenicity, low molecular weight, and relatively high tumor tissue permeability. Typical antibody structures of this type include F(ab')2, scFv-Fab, (scFv)2-Fab, and the like. The IgG-like bispecific antibodies (e.g., antibodies having Fc fragments) have large relative molecular weight. The Fc fragments facilitate the purification of the antibodies and increase their solubility and stability, and the Fc portions may further bind to the receptor FcRn and increase the serum half-life of the antibodies. Typical structural models of bispecific antibodies include bispecific antibodies such as KiH, CrossMAb, Triomab quadroma, FcΔAdp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP-DART, CODV-Fab-TL, HLE-BiTE, F(ab)2-CrossMAb, IgG-(scFv)2, Bs4Ab, DVD-Ig, Tetravalent-DART-Fc, (scFv)4-Fc, CODV-Ig, mAb2, and F(ab)4-CrossMAb (see Aran F. Labrijn et al., Nature Reviews Drug Discovery, volume 18, pages 585-608 (2019); Chen S1 et al., J Immunol Res., Feb. 11, 2019; 2019:4516041).

The term "variable region" or "variable domain" refers to an antigen-binding domain in an antigen-binding molecule. Herein, in the antigen-binding moiety 1 specifically binding to HGFR, the heavy chain variable region is denoted by M-VH, and the light chain variable region is denoted by M-VL; in the antigen-binding moiety 2 specifically binding to EGFR, the heavy chain variable region is denoted by E-VH, and the light chain variable region is denoted by E-VL. The VH and VL each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDRs: HCDR1, HCDR2, and HCDR3; a VL comprises 3 CDRs: LCDR1, LCDR2, and LCDR3. Herein, the 3 CDRs in the M-VH are denoted by M-HCDR1, M-HCDR2, and M-HCDR3, respectively; the 3 CDRs in the M-VL are denoted by M-LCDR1, M-LCDR2, and M-LCDR3, respectively; the 3 CDRs in the E-VH are denoted by E-HCDR1, E-HCDR2, and E-HCDR3, respectively; the 3 CDRs in the E-VL are denoted by E-LCDR1, E-LCDR2, and E-LCDR3, respectively. Each VH and VL is composed of three CDRs and four FRs arranged from the amino terminus to the carboxyl terminus in the following order: FR1, CDR1, FR2, CDR2, FR3, CDR3, and FR4. A single VH or VL may be sufficient to provide antigen binding specificity.

The amino acid sequence boundaries of the CDRs can be determined by a variety of well-known schemes, for example, the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains[J]. 2001), and the ImMunoGenTics (IMGT) numbering scheme (Lefranc, M.P. et al., Dev. Comp. Immunol., 27, 55-77 (2003); Front Immunol., Oct. 16, 2018; 9:2278), and the like. The corresponding relationships between the various numbering schemes are well known to those skilled in the art. The numbering schemes of the present disclosure are shown in Table 1 below.

Table 1. The relationships between CDR numbering schemes

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the variable region and CDR sequences in examples of the present disclosure. Although the Kabat numbering scheme is employed to define amino acid residues in specific embodiments, corresponding technical solutions for other numbering schemes are to be considered as equivalent technical solutions.

The term "antibody fragment" refers to a molecule different from an intact antibody, which comprises a moiety of an intact antibody that retains the antigen binding ability of the intact antibody. Examples of antibody fragments include, but are not limited to, Fv, Fab, Fab', Fab'-SH, F(ab')$_2$, single-domain antibodies, single-chain Fab (scFab), diabodies, linear antibodies, single-chain antibody molecules (e.g., scFv), and multispecific antibodies formed from antibody fragments.

A Fab refers to a protein consisting of VH and CH1 (Fab heavy chain) and VL and CL (Fab light chain) of an immunoglobulin.

An Fv refers to an antigen-binding domain consisting of VH and VL of an immunoglobulin.

In some embodiments in the present disclosure, the first Fab has the structure of a Fab. In a substituted Fab, the CH1 and CL are substituted with a Titin chain or an Obscurin chain. The term "Fc region" or "fragment crystallizable region" is used to define the C-terminal region of the heavy chain of an antibody, including native Fc regions and engineered Fc regions. In some embodiments, the Fc region comprises two identical or different subunits. In some embodiments, the Fc region of the human IgG heavy chain is defined as extending from the amino acid residue at position Cys226 or from Pro230 to its carboxyl terminus. Suitable native sequence Fc regions for the antibodies described herein include human IgG1, IgG2 (IgG2A and IgG2B), IgG3, and IgG4. Unless otherwise stated, the numbering scheme for the Fc region is the EU index.

The term "Titin chain" refers to a fragment of a Titin protein that is 78-118 amino acids in length and comprises a peptide fragment of a Titin Ig-like 152 domain or a functional variant thereof. The Titin chain is capable of forming a dimerized complex with the Obscurin chain.

The term "Obscurin chain" refers to a fragment of an Obscurin protein that is 87-117 amino acids in length and comprises a peptide fragment of an Obscurin Ig-like 1 domain or a functional variant thereof, or a fragment of an Obscurin-like 1 protein that is 78-118 amino acids in length and comprises a peptide fragment of an Obscurin-like Ig-like 1 domain or a functional variant thereof. The Obscurin chain is capable of binding to the Titin chain to form a dimerized complex. The Titin chain and the Obscurin chain of the present disclosure can be used to substitute the CH1 and CL in the Fab to form a substituted Fab (Fab-S). This substitution does not affect the binding of an antigen-binding molecule to an antigen.

The term "capable of specifically binding", "specifically binding", or "binding" means that an antibody is capable of binding to a certain antigen or an epitope thereof with higher affinity than to other antigens or epitopes. Generally, an antibody binds to an antigen or an epitope thereof with an equilibrium dissociation constant (KD) of about $1 \times 10^{-7}$ M or less (e.g., about $1 \times 10^{-8}$ M or less). In some embodiments, the KD for the binding of an antibody to an antigen is 10% or less (e.g., 1%) of the KD for the binding of the antibody to a non-specific antigen (e.g., BSA or casein). KD may be determined using known methods, for example, by a FACS or BIACORE® surface plasmon resonance assay. However, an antibody that specifically binds to an antigen or an epitope thereof may have cross-reactivity to other related antigens, e.g., to corresponding antigens from other species (homologous), such as humans or monkeys, e.g., *Macaca fascicularis* (cynomolgus, cyno), *Pan troglodytes* (chimpanzee, chimp), or *Callithrix jacchus* (commonmarmoset, marmoset).

The term "antigen-binding moiety" refers to a polypeptide molecule specifically binding to a target antigen. Antigen-binding moieties include the antibodies and the fragments thereof described herein. A specific antigen-binding moiety includes an antigen-binding domain of an antibody, which comprises an antibody heavy chain variable region and an antibody light chain variable region. The term "antigen-binding moiety specifically binding to HGFR" refers to a moiety that is capable of binding to HGFR or an epitope thereof with sufficient affinity. In certain examples, the antigen-binding moiety specifically binding to HGFR has an equilibrium dissociation constant (KD) as follows: < about 1 µM, < about 100 nM, or < about 10 nM or less, as determined by the Biacore method. In certain examples, the antigen-binding moiety specifically binding to HGFR binds to conserved epitopes in HGFRs from different species. The term "antigen-binding moiety specifically binding to EGFR" refers to a moiety that is capable of binding to EGFR or an epitope thereof with sufficient affinity, such that a molecule comprising the moiety can be used as a diagnostic agent and/or a therapeutic agent targeting EGFR. In certain examples, the antigen-binding moiety specifically binding to EGFR has an equilibrium dissociation constant (KD) as follows: < about 1 µM, < about 100 nM, or < about 10 nM or less, as determined by the Biacore method. In certain examples, the antigen-binding moiety specifically binding to EGFR binds to conserved epitopes in EGFRs from different species. Antigen-binding moieties include antibody fragments as defined herein, e.g., a Fab, a substituted Fab, or an Fv.

In this context, ordinal numerals in "antigen-binding moiety 1", "antigen-binding moiety 2", "linker 1", and "linker 2" are used for the purpose of only distinguishing different technical features and chemical entities without limiting any order, level, or quantity.

The term "linker" refers to a linker unit that links two polypeptide fragments. Herein, the linkers present in the same structural formula or different structural formulas may be identical or different. The linker may be a peptide linker comprising one or more amino acids, typically about 1-30, 2-24, or 3-15 amino acids. The linkers used herein may be identical or different. When "-" appears in a structural formula, it means that the units on both sides are directly linked by a covalent bond. When the term "bond" appears in a structural unit, it means that the units on both sides of the unit are directly linked.

Unless otherwise stated, the "Kabat" numbering scheme is applied to the variable region and CDR sequences in examples of the present disclosure.

"Pharmaceutical composition" means comprising one or more bispecific antibodies described herein, as well as other components such as physiologically/pharmaceutically acceptable carriers and excipients. The pharmaceutical composition is intended to promote administration to an organism and facilitate the absorption of the active ingredient so that it can exert its biological activity. In the present disclosure, "pharmaceutical composition" and "formulation" are not mutually exclusive.

"Effective amount" comprises an amount sufficient to ameliorate or prevent a symptom or a sign of a medical disorder. An effective amount also means an amount sufficient to allow or facilitate diagnosis. The effective amount for a particular subject or veterinary subject may vary depending on factors such as the disorder to be treated, the general health of the subject, the method, route, and dose of administration, and the severity of side effects. An effective amount may be the maximum dose or administration regimen that avoids significant side effects or toxic effects.

"Pharmaceutically acceptable carrier" or "pharmaceutically acceptable excipient" includes any material that, when combined with an active ingredient, allows the ingredient to retain biological activity and is non-reactive with the immune system of a subject. Examples include, but are not limited to, any standard pharmaceutical carrier, such as phosphate-buffered saline, water, emulsions such as oil/water emulsions, and various types of wetting agents. In some examples, the diluent for aerosol or parenteral administration is phosphate-buffered saline (PBS) or normal (0.9%) saline. Compositions comprising such carriers are formulated by well-known conventional methods.

It should be understood by those skilled in the art that when used with reference to numerical ranges, cutoff values, or specific values, "about" may mean within 1 or more than 1 standard deviation. Alternatively, "about" may mean a range up to 20% apart (i.e., ±20%). Since many of the numerical values used herein are determined experimentally, those skilled in the art will appreciate that such determinations can and typically do vary from experiment to experiment. Because of this inherent difference, it is believed that the values used herein should not be unduly limited. Thus, the term "about" is used to encompass variations of ±20% or less, variations of ±10% or less, variations of ±5% or less, variations of ±1% or less, variations of ±0.5% or less, or variations of ±0.1% or less from a specified value.

"Buffer" refers to a buffer that resists changes in pH by the action of its acid-base conjugate components. Examples of buffers that maintain the pH within an appropriate range include acetate, succinate, gluconate, histidine, oxalate, lactate, phosphate, citrate, tartrate, fumarate, glycylglycine, and other organic acid buffers.

"Histidine buffer" is a buffer comprising histidine. Examples of histidine buffers include histidine-histidine hydrochloride, histidine-histidine acetate, histidine-histidine phosphate, histidine-histidine sulfate, and the like. The histidine-histidine hydrochloride buffer is preferred. The histidine-histidine hydrochloride buffer may be prepared from histidine and hydrochloric acid, or from histidine and histidine hydrochloride.

"Citrate buffer" is a buffer comprising citrate ions. Examples of citrate buffers include citric acid-sodium citrate, citric acid-potassium citrate, citric acid-calcium citrate, citric acid-magnesium citrate, and the like. The preferred citrate buffer is citric acid-sodium citrate.

"Succinate buffer" is a buffer comprising succinate ions. Examples of succinate buffers include succinic acid-sodium succinate, succinic acid-potassium succinate, succinic acid-calcium succinate, and the like. The preferred succinate buffer is succinic acid-sodium succinate. Illustratively, the succinic acid-sodium succinate may be prepared from succinic acid and sodium hydroxide, or from succinic acid and sodium succinate.

"Phosphate buffer" is a buffer comprising phosphate ions. Examples of phosphate buffers include citric acid-disodium hydrogen phosphate, disodium hydrogen phosphate-sodium dihydrogen phosphate, disodium hydrogen phosphate-potassium dihydrogen phosphate, disodium hydrogen phosphate-citric acid, and the like. The preferred phosphate buffer is citric acid-disodium hydrogen phosphate.

"Acetate buffer" is a buffer comprising acetate ions. Examples of acetate buffers include acetic acid-sodium acetate, histidine-histidine acetate, acetic acid-potassium acetate, acetic acid-calcium acetate, acetic acid-magnesium acetate, and the like. The preferred acetate buffer is acetic acid-sodium acetate.

"Poloxamer", a block copolymer of ethylene oxide and propylene oxide, is water-soluble and used as a surfactant in pharmaceutical formulations. Examples of poloxamers include poloxamer 188.

"Edetate disodium" (CAS No. 6381-92-6; the edetate disodium described in the present disclosure is purchased from SIGMA). The official website of the company shows the following aliases: ethylenediaminetetraacetic acid disodium salt dihydrate, EDTA disodium salt, EDTA-Na$_2$, ethylenediaminetetraacetic acid Na$_2$, ethylenediaminetetraacetate disodium salt dihydrate, disodium ethylenediaminetetraacetate dihydrate, and sodium calcium edetate. Therefore, "edetate disodium", "ethylenediaminetetraacetic acid disodium salt dihydrate", "EDTA-2Na", and disodium edetate are used interchangeably in the present disclosure and are essentially the substance under CAS No. 6381-92-6.

"Freeze-dried formulation" refers to a formulation or a pharmaceutical composition obtained by lyophilizing a pharmaceutical composition or a formulation in liquid or solution form *in vacuo.*

The pharmaceutical composition described herein can achieve a stable effect. That is, the antibody in the pharmaceutical composition substantially retains its physical and/or chemical stability and/or biological activity after storage; preferably, the pharmaceutical composition substantially retains its physical and chemical stability as well as its biological activity after storage. The storage period is generally selected based on a predetermined shelf life of the pharmaceutical composition. There are a variety of analytical techniques currently available for determining protein stability, and the stability after storage for a selected period of time at a selected temperature can be determined.

A stable formulation is one in which no significant change is observed under the following conditions: stored at refrigeration temperature (2-8 °C) for at least 1 month, at least 3 months, preferably 6 months, more preferably 1 year, and even more preferably up to 2 years. In addition, stable liquid formulations include liquid formulations that exhibit desirable characteristics after storage at temperatures including 25 °C for periods including 1 month, 3 months, or 6 months. They also include formulations that exhibit the desired characteristics after storage at a temperature of 40 °C for periods including 4 weeks, 1 month, 3 months, or 6 months. Typical examples for stability are as follows: generally, no more than about 10%, preferably no more than about 5%, of antibodies aggregate or are degraded as determined by SEC-HPLC. The formulation is a pale yellow, nearly colorless and clear liquid, or a colorless and clear liquid, or is clear to slightly opalescent, by visual analysis. The concentration, pH, weight, and osmolality of the formulation vary by no more than ±10%, preferably by no more than ±5%. Generally, aggregation of no more than about 10%, preferably no more than about 5% is formed in the formulation.

An antibody "retains its physical stability" in a pharmaceutical formulation if it shows no significant increase in aggregation, precipitation, and/or denaturation upon visual inspection of color and/or clarity, or as determined by UV light scattering, size exclusion chromatography (SEC), and dynamic light scattering (DLS). Changes in protein conformation can be evaluated by fluorescence spectroscopy (which determines the protein tertiary structure) and by FTIR spectroscopy (which determines the protein secondary structure).

An antibody "retains its chemical stability" in a pharmaceutical formulation if it shows no significant chemical change. Chemical stability can be evaluated by detecting and quantifying chemically changed proteins. Degradation processes that often change the chemical structure of proteins include hydrolysis or clipping (evaluated by methods such as size exclusion chromatography and CE-SDS), oxidation (evaluated by methods such as peptide mapping in conjunction with mass spectroscopy or MALDI/TOF/MS), deamidation (evaluated by methods such as ion-exchange chromatography, capillary isoelectric focusing, peptide mapping, and isoaspartic acid measurement), and isomerization (evaluated by measuring the isoaspartic acid content, peptide mapping, etc.).

An antibody "retains its biological activity" in a pharmaceutical formulation if the biological activity of the antibody at a given time is within a predetermined range of the biological activity exhibited during the preparation of the pharmaceutical formulation.

"Treatment" or "treat" (and grammatical variations thereof) refers to clinical intervention applied to the treated individual, which may be performed either for prophylaxis or during the course of clinical pathology. Desirable effects of the treatment include, but are not limited to, preventing the occurrence or recurrence of a disease, palliating symptoms, palliating/diminishing any direct or indirect pathological consequences of the disease, preventing metastasis, decreasing the rate of disease progression, ameliorating or palliating the disease state, and regressing or improving prognosis. In some embodiments, the antigen-binding molecule of the present disclosure is used to delay the development of or slow the progression of disease.

The details of one or more embodiments of the present disclosure are set forth in the specification above. Although any methods and materials similar or identical to those described herein can be used in the practice or testing of the present disclosure, the preferred methods and materials are described below. Other features, objects, and advantages of the present disclosure will be apparent from the specification and the claims. In the specification and claims, singular forms include plural referents unless otherwise indicated clearly in the context. Unless otherwise defined, all technical and scientific terms used herein have the meanings generally understood by those of ordinary skill in the art to which the present disclosure pertains. All the patents and publications cited in the specification are incorporated by reference. The following examples are set forth in order to more fully illustrate the preferred embodiments of the present disclosure. These examples should not be construed in any way as limiting the scope of the present disclosure, which is defined by the claims.

**Examples-Preparation of Bispecific Antibodies Specifically Binding to GPRC5D and CD3**

PCT/CN2023/081237 (filing date: March 14, 2023; Priority Patent Application No.: CN202210245308.7) is incorporated herein by reference in its entirety.

Example 1. Construction and Identification of Cell Lines Expressing Recombinant Human GPRC5D and Cynomolgus Monkey GPRC5D

To screen for antibodies that can bind well to the cell surface GPRC5D, CHOK1-humanGPRC5D, HEK293-humanGPRC5D, CHOK1-cynoGPRC5D, and HEK293-cynoGPRC5D cell strains expressing human GPRC5D or cynomolgus monkey GPRC5D were constructed in the present disclosure. The full-length gene of human GPRC5D or cynomolgus monkey GPRC5D was cloned into the mammalian cell expression vector pCDH. The pVSV-G, pCMV-dR8.91, and pCDH-humanGPRC5D or pCDH-cynoGPRC5D plasmids were co-transfected into HEK293T cells (ATCC® CRL-11268) to package the virus. After 48 h of transfection, the virus was collected and used to infect CHOK1 cells (ATCC® CCL-61) and HEK293 cells (ATCC® CRL-1573). After 72 h of infection, monoclonal cells highly expressing human GPRC5D or cynomolgus monkey GPRC5D were obtained by flow cytometry sorting.

**Example 2. Preparation of Mouse Anti-Human GPRC5D Monoclonal Antibodies**

SJL mice were cross-immunized with CHOK1-humanGPRC5D and CHOK1-cynoGPRC5D cells. After 7 rounds of immunization, blood was collected to measure the serum antibody titers. Mice with high and plateauing serum antibody titers were selected for spleen cell fusion. The fused hybridoma cells were plated in a 96-well cell culture plate and cultured in a 5% $CO_2$ incubator at 37 °C. The cell culture supernatants were tested using mirrorball and flow cytometry sorting (FACS). The positive clones obtained by screening were expanded, cryopreserved, and subcloned two to three times until single cell clones were obtained. The selected hybridoma clones were further subjected to serum-free cell culture to prepare and purify antibodies. The hybridoma antibodies obtained were tested by FACS for their binding to CHOK1-humanGPRC5D, CHOK1-cynoGPRC5D, and CHOK1 cells (see Test Example 1 of the present disclosure). Hybridoma cell strains mAb1 and mAb24, which showed good human-monkey cross-binding activity and no non-specific binding, were selected.

The sequences of the monoclonal antibodies from hybridoma cell strains mAb1 and mAb24 were cloned as follows: Hybridoma cells in the logarithmic growth phase were collected, and RNA was extracted using Trizol (Invitrogen, Cat# 15596-018) and reverse-transcribed into cDNA. PCR amplification was performed using the cDNA as a template, and the products were sent to a sequencing company for sequencing. The amino acid sequences of the variable regions of the antibodies corresponding to the obtained DNA sequences are as follows:

The amino acid sequence of mAb 1 heavy chain variable region:

EVQLVESGGGLVKPGGSLKLSCAASGFTFS<u>DYGMH</u>WVRQAPEKGLEWVA<u>FISS GSSTIYYADTVKG</u>RFTISRDNAKNTLFLQMTSLRSEDTAIYYCTR<u>LGPNWYFDV</u> WGTGTTVTVSS

SEQ ID NO: 1

The amino acid sequence of mAb 1 light chain variable region:

DVVMTQTPLSLSVSLGDQASISC<u>RSSQSLVHSNGNTYLY</u>WYLQKPGQSPKLLIY <u>KVSNRFS</u>GVPDRFSGSGSGTDFTLKISRVEAEDLGVYFC<u>SQSTHVPPLT</u>FGAGTK LELK

SEQ ID NO: 2

The amino acid sequence of mAb24 heavy chain variable region:

QVQLQQSDAELVKPGGSVKISCKVSGYTFT<u>DYPIH</u>WMKQRPEQGLEWMG<u>YVF PRDGSTKYNEKFRV</u>KATWTADKSSSTVYMQLNSLTSDDSAVYFCSR<u>ELTAY</u>W GQGTTLRVSS

SEQ ID NO: 3

The amino acid sequence of mAb24 light chain variable region:

DVVLTQTPLTLSVTIGQPASISC<u>KSSQSLLHSNGKTYLN</u>WLLQRPGQSPRRLIY<u>L VSKLDS</u>GVPDRFAGSGSGTDFTLKITRVEAEDLGVYYC<u>AQGTHFPRT</u>FGGGSKL EIR

SEQ ID NO: 4

Note: The underlined parts are CDRs according to the Kabat numbering scheme, and the rest are FRs.

Table 2. CDRs of GPRC5D antibodies

| Antibody | Heavy chain variable region | | | Light chain variable region | |
|---|---|---|---|---|---|
| mAb1 | HCDR1 | DYGMH (SEQ ID NO: 5) | LCDR1 | RSSQSLVHSNGNTYLY (SEQ ID NO: 8) | |
| | HCDR2 | FISSGSSTIYYADTVKG (SEQ ID NO: 6) | LCDR2 | KVSNRFS (SEQ ID NO: 9) | |
| | HCDR3 | LGPNWYFDV (SEQ ID NO: 7) | LCDR3 | SQSTHVPPLT (SEQ ID NO: 10) | |
| mAb24 | HCDR1 | DYPIH (SEQ ID NO: 11) | LCDR1 | KSSQSLLHSNGKTYLN (SEQ ID NO: 14) | |
| | HCDR2 | YVFPRDGSTKYNEKFRV (SEQ ID NO: 12) | LCDR2 | LVSKLDS (SEQ ID NO: 15) | |
| | HCDR3 | ELTAY (SEQ ID NO: 13) | LCDR3 | AQGTHFPRT (SEQ ID NO: 16) | |
| Note: The CDRs in the table are determined according to the Kabat numbering scheme. | | | | | |

**Example 3. Humanization Design for Anti-Human GPRC5D Monoclonal Antibodies**

The humanization of murine monoclonal antibodies was carried out using methods disclosed in many publications in the art. In brief, based on the typical structures of the VH/VL CDRs of the murine antibodies obtained, a human germline database was searched for homologous sequences of the light chain variable regions (VL) and the heavy chain variable regions (VH); the CDRs of the murine antibodies were grafted onto a human template, and some residues of the VL and VH were mutated; the constant regions of the murine antibodies were substituted with a human constant region to obtain the final humanized molecules.

1. Humanization of mAb1 antibody

IGHV3-21*01 was used for the FR1, FR2, and FR3 of the humanized heavy chain variable region of mAb1, and IGHJ6*01 was used for the FR4; IGKV2-29*02 was used for the FR1, FR2, and FR3 of the light chain variable region, and IGKJ2*01 was used for the FR4. Optionally, the amino acids at positions 49, 56, 57, and/or 93 in the heavy chain variable region of the humanized antibody were substituted; and/or the amino acids at positions 2, 28, 29, and/or 45 in the light chain variable region of the humanized antibody were substituted.

Table 3. Amino acid substitutions in the humanized antibody of mAb1

| hu1 VL | | hu1 VH | |
|---|---|---|---|
| L1 | None | H1 | S49A, A93T |
| L2 | I2V, Q45K | H2 | S49A, T56N, A93T |
| L3 | I2V, N28S, Q45K | H3 | S49A, I57Q, A93T |
| L4 | I2V, N28Q, Q45K | | |
| L5 | I2V, N28T, Q45K | | |
| L6 | I2V, G29V, Q45K | | |
| Note: Illustratively, Q45K indicates that the Q at position 45 is mutated back to K according to the Kabat numbering scheme. This applies similarly to other mutations. | | | |

The sequences of the antibody variable regions are as follows (the CDRs are underlined; this applies hereinafter):

>hu1H1 (SEQ ID NO: 17):

EVQLVESGGGLVKPGGSLRLSCAASGFTFS<u>DYGMH</u>WVRQAPGKGLEWVA<u>FISS GSSTIYYADTVKG</u>RFTISRDNAKNSLYLQMNSLRAEDTAVYYCTR<u>LGPNWYFD V</u>WGQGTTVTVSS

> hu1H2 (SEQ ID NO: 18):

EVQLVESGGGLVKPGGSLRLSCAASGFTFS<u>DYGMH</u>WVRQAPGKGLEWVA<u>FISS GSSNIYYADTVKG</u>RFTISRDNAKNSLYLQMNSLRAEDTAVYYCTR<u>LGPNWYFD V</u>WGQGTTVTVSS

> hu1H3 (SEQ ID NO: 19):

EVQLVESGGGLVKPGGSLRLSCAASGFTFS<u>DYGMH</u>WVRQAPGKGLEWVA<u>FISS GSSTQYYADTVKG</u>RFTISRDNAKNSLYLQMNSLRAEDTAVYYCTR<u>LGPNWYF DV</u>WGQGTTVTVSS

> hu1L1 (SEQ ID NO: 20):

DIVMTQTPLSLSVTPGQPASISC<u>RSSQSLVHSNGNTYLY</u>WYLQKPGQSPQLLIY<u>K VSNRFS</u>GVPDRFSGSGSGTDFTLKISRVEAEDVGVYYC<u>SQSTHVPPLT</u>FGQGTK LEIK

> hu1L2 SEQ ID NO: 21):

DVVMTQTPLSLSVTPGQPASISC<u>RSSQSLVHSNGNTYLY</u>WYLQKPGQSPKLLIY <u>KVSNRFS</u>GVPDRFSGSGSGTDFTLKISRVEAEDVGVYYC<u>SQSTHVPPLT</u>FGQGT KLEIK

> hu1L3 (SEQ ID NO: 22):

DVVMTQTPLSLSVTPGQPASISC<u>RSSQSLVHSSGNTYLY</u>WYLQKPGQSPKLLIY <u>KVSNRFS</u>GVPDRFSGSGSGTDFTLKISRVEAEDVGVYYC<u>SQSTHVPPLT</u>FGQGT KLEIK

> hu1L4 (SEQ ID NO: 23):

DVVMTQTPLSLSVTPGQPASISC<u>RSSQSLVHSQGNTYLY</u>WYLQKPGQSPKLLIY <u>KVSNRFS</u>GVPDRFSGSGSGTDFTLKISRVEAEDVGVYYC<u>SQSTHVPPLT</u>FGQGT KLEIK

> hu1L5 (SEQ ID NO: 24):

DVVMTQTPLSLSVTPGQPASISC<u>RSSQSLVHSTGNTYLY</u>WYLQKPGQSPKLLIY <u>KVSNRFS</u>GVPDRFSGSGSGTDFTLKISRVEAEDVGVYYC<u>SQSTHVPPLT</u>FGQGT

KLEIK

> hu1L6 SEQ ID NO: 25):

DVVMTQTPLSLSVTPGQPASISCRSSQSLVHSNVNTYLYWYLQKPGQSPKLLIY KVSNRFSGVPDRFSGSGSGTDFTLKISRVEAEDVGVYYCSQSTHVPPLTFGQGT KLEIK

The amino acid sequences of substituted CDRs in the humanized antibodies are shown in the table below.

Table 4. Amino acid sequences of substituted CDRs in hu1

| Name | Amino acid sequence | Sequence No. |
|---|---|---|
| > hu1L3 LCDR1 | RSSQSLVHSSGNTYLY | SEQ ID NO: 26 |
| > hu1L4 LCDR1 | RSSQSLVHSQGNTYLY | SEQ ID NO: 27 |
| > hu1L5 LCDR1 | RSSQSLVHSTGNTYLY | SEQ ID NO: 28 |
| > hu1L6 LCDR1 | RSSQSLVHSNVNTYLY | SEQ ID NO: 29 |
| > hu1H2 HCDR2 | FISSGSSNIYYADTVKG | SEQ ID NO: 30 |
| > hu1H3 HCDR2 | FISSGSSTQYYADTVKG | SEQ ID NO: 31 |

2. Humanization of mAb24 antibody

IGHV1-46*01 was used for the FR1, FR2, and FR3 of the humanized heavy chain variable region of the mAb24 antibody, and IGHJ6*01 was used for the FR4; IGKV2-30*02 was used for the FR1, FR2, and FR3 of the light chain variable region, and IGKJ4*01 was used for the FR4. Optionally, the amino acids at positions 1, 24, 37, 69, 71, 73, and/or 93 in the heavy chain variable region of the humanized antibody were substituted; and/or the amino acids at positions 4, 28, 29, 36, and/or 37 in the light chain variable region of the humanized antibody were substituted.

Table 5. Amino acid substitutions in the humanized antibody of mAb24

| hu24 VL | | hu24 VH | |
|---|---|---|---|
| L1 | None | H1 | Q1E, R71A, A93S |
| L2 | M4L, F36L, Q37L | H2 | Q1E, M69W, R71A, T73K, A93S |
| L3 | M4L, G29V, F36L, Q37L | H3 | Q1E, A24V, V37M, M69W, R71A, T73K, A93S |
| L4 | M4L, G29L, F36L, Q37L | | |
| L5 | M4L, N28S, F36L, Q37L | | |
| L6 | M4L, N28T, F36L, Q37L | | |
| Note: Illustratively, M4L indicates that the M at position 4 is mutated back to L according to the Kabat numbering scheme. This applies similarly to other mutations. | | | |

The sequences of the antibody variable regions are as follows:

>hu24H1 (SEQ ID NO: 32):

EVQLVQSGAEVKKPGASVKVSCKASGYTFTDYPIHWVRQAPGQGLEWMGYVF PRDGSTKYNEKFRVRVTMTADTSTSTVYMELSSLRSEDTAVYYCSRELTAYWG QGTTVTVSS

>hu24H2 (SEQ ID NO: 33):

EVQLVQSGAEVKKPGASVKVSCKASGYTFT<u>DYPIH</u>WVRQAPGQGLEWMG<u>YVF PRDGSTKYNEKFRV</u>RVTWTADKSTSTVYMELSSLRSEDTAVYYCSR<u>ELTAY</u>W GQGTTVTVSS

>hu24H3 (SEQ ID NO: 34):

EVQLVQSGAEVKKPGASVKVSCKVSGYTFT<u>DYPIH</u>WMRQAPGQGLEWMG<u>YV FPRDGSTKYNEKFRV</u>RVTWTADKSTSTVYMELSSLRSEDTAVYYCSR<u>ELTAY</u>W GQGTTVTVSS

>hu24L1 (SEQ ID NO: 35):

DVVMTQSPLSLPVTLGQPASISC<u>KSSQSLLHSNGKTYLN</u>WFQQRPGQSPRRLIY<u>L VSKLDS</u>GVPDRFSGSGSGTDFTLKISRVEAEDVGVYYC<u>AQGTHFPRT</u>FGGGTKV EIK

>hu24L2 (SEQ ID NO: 36):

DVVLTQSPLSLPVTLGQPASISC<u>KSSQSLLHSNGKTYLN</u>WLLQRPGQSPRRLIY<u>L VSKLDS</u>GVPDRFSGSGSGTDFTLKISRVEAEDVGVYYC<u>AQGTHFPRT</u>FGGGTKV EIK

>hu24L3 (SEQ ID NO: 37):

DVVLTQSPLSLPVTLGQPASISC<u>KSSQSLLHSNVKTYLN</u>WLLQRPGQSPRRLIY<u>L VSKLDS</u>GVPDRFSGSGSGTDFTLKISRVEAEDVGVYYC<u>AQGTHFPRT</u>FGGGTKV EIK

>hu24L4 (SEQ ID NO: 38):

DVVLTQSPLSLPVTLGQPASISC<u>KSSQSLLHSNLKTYLN</u>WLLQRPGQSPRRLIY<u>L VSKLDS</u>GVPDRFSGSGSGTDFTLKISRVEAEDVGVYYC<u>AQGTHFPRT</u>FGGGTKV EIK

>hu24L5 (SEQ ID NO: 39):

DVVLTQSPLSLPVTLGQPASISC<u>KSSQSLLHSSGKTYLN</u>WLLQRPGQSPRRLIY<u>L VSKLDS</u>GVPDRFSGSGSGTDFTLKISRVEAEDVGVYYC<u>AQGTHFPRT</u>FGGGTKV EIK

>hu24L6 (SEQ ID NO: 40):

DVVLTQSPLSLPVTLGQPASISC<u>KSSQSLLHSTGKTYLN</u>WLLQRPGQSPRRLIY<u>L VSKLDS</u>GVPDRFSGSGSGTDFTLKISRVEAEDVGVYYC<u>AQGTHFPRT</u>FGGGTKV EIK

The amino acid sequences of substituted CDRs in the humanized antibodies are shown in the table below.

Table 6. Amino acid sequences of substituted CDRs in hu24

| Name | Amino acid sequence | Sequence No. |
|------|--------------------|--------------| 
| >hu24L3 LCDR1 | KSSQSLLHSNVKTYLN | SEQ ID NO: 41 |
| >hu24L4 LCDR1 | KSSQSLLHSNLKTYLN | SEQ ID NO: 42 |
| >hu24L5 LCDR1 | KSSQSLLHSSGKTYLN | SEQ ID NO: 43 |
| >hu24L6 LCDR1 | KSSQSLLHSTGKTYLN | SEQ ID NO: 44 |

**Example 4. Construction of Chimeric Antibodies**

The variable region sequences of the murine anti-GPRC5D antibodies mAb1 and mAb24 were each combined with a heavy chain constant region set forth in SEQ ID NO: 45 and a light chain constant region set forth in SEQ ID NO: 46 to obtain chimeric antibodies Chi-1 and Chi-24. Illustratively, Chi-1 represents a chimeric antibody comprising the murine heavy and light chain variable regions of mAb1 and the constant regions. This applies similarly to other chimeric antibodies. The heavy and light chain constant region sequences of the chimeric antibodies are as follows:

> The heavy chain constant region sequence (SEQ ID NO: 45):

ASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHT
CPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQ
PENNYKTTPPVLDSDGSFFLYSKLTVDKSRWQQGNVFSCSVMHEALHNHYTQ
KSLSLSPGK

> CL (SEQ ID NO: 46):

RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC

The heavy chain sequence of Chi-1 (SEQ ID NO: 47):

EVQLVESGGGLVKPGGSLKLSCAASGFTFS<u>DYGMH</u>WVRQAPEKGLEWVA<u>FISS
GSSTIYYADT</u>VKGRFTISRDNAKNTLFLQMTSLRSEDTAIYYCTR<u>LGPNWYFDV</u>
WGTGTTVTVSS*ASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCD
KTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEK
TISKAKGQPREPQVYTLPPSRDELTKNQVSLTCLVKGFYPSDIAVEWESNGQPENNY
KTTPPVLDSDGSFFLYSKLTVDKSRWQQGNVFSCSVMHEALHNHYTQKSLSLSPGK*

The light chain sequence of Chi-1 (SEQ ID NO: 48):

DVVMTQTPLSLSVSLGDQASISCRSSQSLVHSNGNTYLYWYLQKPGQSPKLLIY
KVSNRFSGVPDRFSGSGSGTDFTLKISRVEAEDLGVYFCSQSTHVPPLTFGAGTK
LELK*RTVAAPSVFIFPPSDEQLKSGTASVVCLLNNFYPREAKVQWKVDNALQSGNSQ*
*ESVTEQDSKDSTYSLSSTLTLSKADYEKHKVYACEVTHQGLSSPVTKSFNRGEC*

The heavy chain sequence of Chi-24 (SEQ ID NO: 49):

QVQLQQSDAELVKPGGSVKISCKVSGYTFTDYPIHWMKQRPEQGLEWMGYVF

PRDGSTKYNEKFRVKATWTADKSSSTVYMQLNSLTSDDSAVYFCSRELTAYW
GQGTTLRVSS*ASTKGPSVFPLAPSSKSTSGGTAALGCLVKDYFPEPVTVSWNSGALT*
*SGVHTFPAVLQSSGLYSLSSVVTVPSSSLGTQTYICNVNHKPSNTKVDKKVEPKSCDK*
*THTCPPCPAPEAAGGPSVFLFPPKPKDTLMISRTPEVTCVVVDVSHEDPEVKFNWYV*
*DGVEVHNAKTKPREEQYNSTYRVVSVLTVLHQDWLNGKEYKCKVSNKALPAPIEKTI*
*SKAKGQPREPQVYTLPPSRDELTKNQVSLTCLVKGFYPSDIAVEWESNGQPENNYKT*
*TPPVLDSDGSFFLYSKLTVDKSRWQQGNVFSCSVMHEALHNHYTQKSLSLSPGK*

The light chain sequence of Chi-24 (SEQ ID NO: 50):

DVVLTQTPLTLSVTIGQPASISCKSSQSLLHSNGKTYLNWLLQRPGQSPRRLIYL
VSKLDSGVPDRFAGSGSGTDFTLKITRVEAEDLGVYYCAQGTHFPRTFGGGSKL
EIR*RTVAAPSVFIFPPSDEQLKSGTASVVCLLNNFYPREAKVQWKVDNALQSGNSQES*
*VTEQDSKDSTYSLSSTLTLSKADYEKHKVYACEVTHQGLSSPVTKSFNRGEC*

**Example 5. Preparation and Identification of Anti-GPRC5D/CD3 Bispecific Antibodies**

The CD3-binding molecules of the present disclosure may be derived from any suitable antibody. Particularly suitable antibodies are described, for example, in international application No. PCT/CN2019/123548 (incorporated herein by reference in its entirety). Specifically, S107E was used in the examples of the present disclosure.

Table 7. CDRs of S107E

| No. | Heavy chain | | Light chain | |
|---|---|---|---|---|
| S107E | HCDR1 | KYAMN (SEQ ID NO: 51) | LCDR1 | GSSTGAVTSGNYPN (SEQ ID NO: 54) |
| | HCDR2 | RIRSKYNNYATYYADSVKD (SEQ ID NO: 52) | LCDR2 | GTKFLAP (SEQ ID NO: 55) |
| | HCDR3 | HGNFGNEYISYWAY (SEQ ID NO: 53) | LCDR3 | VLWYSNRWV (SEQ ID NO: 56) |

The sequences of the variable regions of S107E are as follows:

> S107E-VH (SEQ ID NO: 57):

EVQLVESGGGLVQPGGSLKLSCAASGFTFNKYAMNWVRQAPGKGLEWVARIR
SKYNNYATYYADSVKDRFTISRDDSKNTAYLQMNNLKTEDTAVYYCVRHGNF
GNEYISYWAYWGQGTLVTVSS

> S107E-VL (SEQ ID NO: 58):

QTVVTQEPSLTVSPGGTVTLTC<u>GSSTGAVTSGNYPN</u>WVQQKPGQAPRGLIG<u>GT
KFLAP</u>GTPARFSGSLLGGKAALTLSGVQPEDEAEYYC<u>VLWYSNRWV</u>FGGGTK
LTVL

Humanized anti-GPRC5D antibody variable regions were combined with the variable regions of the anti-CD3 antibody S107E, IgG$_1$(CH1), Obscurin, Titin, and an IgG1 mutant IgG1(AA, L234A/L235A). Two different forms of bispecific antibodies were formed, and they were formula 2-0G4S and formula 4-0G4S.

Formula 2-0G4S (i.e., F2-0G4S) is an asymmetrically structured molecule comprising four chains:

chain 1: VH(anti-GPRCSD)-IgG$_1$(CH1)-IgG$_1$Fc(Knob);
chain 2: VL(anti-GPRC5D)-CL;
chain 3: (Ob(A0B2)-Hole): VH(S107E)-Ob(A0B2)-IgG$_1$Fc(Hole); and
chain 4: (VL-Titin(A0B2)): VL(S107E)-Titin(A0B2); a schematic diagram of this formula is shown in FIG. 1 (OB stands for Obscurin);
wherein:

> Ob(A0B2)-Hole (SEQ ID NO: 59):

EVQLVESGGGLVQPGGSLKLSCAASGFTFN<u>KYAMN</u>WVRQAPGKGLEWVA<u>RIR
SKYNNYATYYADSVKD</u>RFTISRDDSKNTAYLQMNNLKTEDTAVYYCVR<u>HGNF
GNEYISYWAY</u>WGQGTLVTVSS<u>SGAPRFLTCPKASVVSVGKDATLSSQIVGNPFP
QVSWEKDKQPVTAGVRFRLAQDGDLYRLKILDLQLSDSGQYVSRARNAHGEA
FACLGLQVDAEA</u>*DKTHTCPPCPAPE<u>AA</u>GGPSVFLFPPKPKDTLMISRTPEVTCVVV
DVSHEDPEVKFNWYVDGVEVHNAKTKPREEQYNSTYRVVSVLTVLHQDWLNGKEY
KCKVSNKALPAPIEKTISKAKGQPREPQV<u>C</u>TLPPSRDELTKNQVSL<u>S</u>C<u>A</u>VKGFYPSDI
AVEWESNGQPENNYKTTPPVLDSDGSFFL<u>V</u>SKLTVDKSRWQQGNVFSCSVMHEALH
NHYTQKSLSLSPGK*

Note: The CD3-binding domain CDRs obtained according to the Kabat numbering scheme are marked with "____", the heavy chain constant regions are "italicized", Obscurin(A0B2) is marked with "__", and the mutation positions (L234A, L235A, Y349C, T366S, L368A, and Y407V) are marked with "__".
> VL-Titin(A0B2) (SEQ ID NO: 60):

QTVVTQEPSLTVSPGGTVTLTC<u>GSSTGAVTSGNYPN</u>WVQQKPGQAPRGLIG<u>GT
KFLAP</u>GTPARFSGSLLGGKAALTLSGVQPEDEAEYYC<u>VLWYSNRWV</u>FGGGTK
LTVL<u>GIPPKIECLPIDISIDEGKVLTVASCFTGEPTPEVTWSTGGRKIHSQEQGRFH
IENTDDSTTLTIKDVQKQDGGLYTLTLRNEFGSDSATVNIHIRSI</u>

Note: The CD3-binding domain CDRs obtained according to the Kabat numbering scheme are marked with "__", and Titin(A0B2) was marked with "____".

Formula 4-0G4S (i.e., F4-0G4S) is an asymmetrically structured molecule comprising four chains:

chain 1: (Ob(A0B2)-Knob): VH(S107E)-Ob(A0B2)-IgG$_1$Fc(Knob);
chain 2: (VL-Titin(A0B2)): VL(S107E)-Titin(A0B2);
chain 3: VH(anti-GPRC5D)-IgG$_1$(CH1)-IgG$_1$Fc(Hole); and
chain 4: VL(anti-GPRC5D)-CL;
a schematic diagram of this formula is shown in FIG. 2 (OB stands for Obscurin).
> Ob(A0B2)-Knob (SEQ ID NO: 61):

EVQLVESGGGLVQPGGSLKLSCAASGFTFNKYAMNWVRQAPGKGLEWVARIR

SKYNNYATYYADSVKDRFTISRDDSKNTAYLQMNNLKTEDTAVYYCVRHGNF
GNEYISYWAYWGQGTLVTVSSSGAPRFLTCPKASVVSVGKDATLSSQIVGNPFP
QVSWEKDKQPVTAGVRFRLAQDGDLYRLKILDLQLSDSGQYVSRARNAHGEA
FACLGLQVDAEA*DKTHTCPPCPAPEAAGGPSVFLFPPKPKDTLMISRTPEVTCVVV
DVSHEDPEVKFNWYVDGVEVHNAKTKPREEQYNSTYRVVSVLTVLHQDWLNGKEY
KCKVSNKALPAPIEKTISKAKGQPREPQVYTLPPCRDELTKNQVSLWCLVKGFYPSDI
AVEWESNGQPENNYKTTPPVLDSDGSFFLYSKLTVDKSRWQQGNVFSCSVMHEALH
NHYTQKSLSLSPGK*

Note: The CD3-binding domain CDRs obtained according to the Kabat numbering scheme are marked with "____", the heavy chain constant regions are "italicized", Obscurin(A0B2) is marked with "__", and the mutation positions (L234A, L235A, S354C, and T366W) are marked with "__".
> VL-Titin(A0B2) (SEQ ID NO: 60):

QTVVTQEPSLTVSPGGTVTLTCGSSTGAVTSGNYPNWVQQKPGQAPRGLIGGT
KFLAPGTPARFSGSLLGGKAALTLSGVQPEDEAEYYCVLWYSNRWVFGGGTK
LTVLGIPPKIECLPIDISIDEGKVLTVASCFTGEPTPEVTWSTGGRKIHSQEQGRFH
IENTDDSTTLTIKDVQKQDGGLYTLTLRNEFGSDSATVNIHIRSI

Note: The CD3-binding domain CDRs obtained according to the Kabat numbering scheme are marked with "__", and Titin(A0B2) is marked with "___".
wherein:

> IgG$_1$(CH1) (SEQ ID NO: 62):

ASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSC

> IgG$_1$Fc(Knob) (SEQ ID NO: 63):

DKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVYTLPPCRDELTKNQVSLWCLVKGFYPSDIAVE
WESNGQPENNYKTTPPVLDSDGSFFLYSKLTVDKSRWQQGNVFSCSVMHEAL
HNHYTQKSLSLSPGK

> IgG$_1$Fc(Hole) (SEQ ID NO: 64):

DKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVCTLPPSRDELTKNQVSLSCAVKGFYPSDIAVEW
ESNGQPENNYKTTPPVLDSDGSFFLVSKLTVDKSRWQQGNVFSCSVMHEALHN
HYTQKSLSLSPGK

> Obscurin(A0B2) (SEQ ID NO: 83):

SGAPRFLTCPKASVVSVGKDATLSSQIVGNPFPQVSWEKDKQPVTAGVRFRLA
QDGDLYRLKILDLQLSDSGQYVSRARNAHGEAFACLGLQVDAEA

> Titin(A0B2) (SEQ ID NO: 84):

GIPPKIECLPIDISIDEGKVLTVASCFTGEPTPEVTWSTGGRKIHSQEQGRFHIENT
DDSTTLTIKDVQKQDGGLYTLTLRNEFGSDSATVNIHIRSI

Table 8. Bispecific antibodies of the present disclosure

| Antibody No. | Chain 1 | Chain 2 | Chain 3 | Chain 4 |
|---|---|---|---|---|
| F2-0G4S-1 | hu1H3-CH1-Knob (SEQ ID NO: 65) | hu1L4-CL (SEQ ID NO: 66) | Ob(A0B2)-Hole (SEQ ID NO: 59) | VL-Titin(A0B2) (SEQ ID NO: 60) |
| F4-0G4S-1 | Ob(A0B2)-Knob (SEQ ID NO: 61) | VL-Titin(A0B2) (SEQ ID NO: 60) | hu24H3-CH1-Hole (SEQ ID NO: 67) | hu24L5-CL (SEQ ID NO: 68) |

The specific amino acid sequences are as follows:

the chain 1 of F2-0G4S-1 (SEQ ID NO: 65):

EVQLVESGGGLVKPGGSLRLSCAASGFTFS<u>DYGMH</u>WVRQAPGKGLEWVA<u>FISS
GSSTQYYADTVKG</u>RFTISRDNAKNSLYLQMNSLRAEDTAVYYCTR<u>LGPNWYF
DV</u>WGQGTTVTVSS*ASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPCRDELTKNQVSLWCLVKGFYPSDIAVEWESNGQPE
NNYKTTPPVLDSDGSFFLYSKLTVDKSRWQQGNVFSCSVMHEALHNHYTQKSLSLSP
GK*

the chain 2 of F2-0G4S-1 (SEQ ID NO: 66):

DVVMTQTPLSLSVTPGQPASISC<u>RSSQSLVHSQGNTYLY</u>WYLQKPGQSPKLLIY
<u>KVSNRFS</u>GVPDRFSGSGSGTDFTLKISRVEAEDVGVYYC<u>SQSTHVPPLT</u>FGQGT
KLEIK*RTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGEC*

the chain 3 of F2-0G4S-1 (SEQ ID NO: 59)
the chain 4 of F2-0G4S-1 (SEQ ID NO: 60)
the chain 1 of F4-0G4S-1 (SEQ ID NO: 61)
the chain 2 of F4-0G4S-1 (SEQ ID NO: 60)
the chain 3 of F4-0G4S-1 (SEQ ID NO: 67):

EVQLVQSGAEVKKPGASVKVSCKVSGYTFT<u>DYPIH</u>WMRQAPGQGLEWMG<u>YV</u>
<u>FPRDGSTKYNEKFRV</u>RVTWTADKSTSTVYMELSSLRSEDTAVYYCSR<u>ELTAY</u>W
GQGTTVTVSS*ASTKGPSVFPLAPSSKSTSGGTAALGCLVKDYFPEPVTVSWNSGALT*
*SGVHTFPAVLQSSGLYSLSSVVTVPSSSLGTQTYICNVNHKPSNTKVDKKVEPKSCDK*
*THTCPPCPAPEAAGGPSVFLFPPKPKDTLMISRTPEVTCVVVDVSHEDPEVKFNWYV*
*DGVEVHNAKTKPREEQYNSTYRVVSVLTVLHQDWLNGKEYKCKVSNKALPAPIEKTI*
*SKAKGQPREPQVCTLPPSRDELTKNQVSLSCAVKGFYPSDIAVEWESNGQPENNYKT*
*TPPVLDSDGSFFLVSKLTVDKSRWQQGNVFSCSVMHEALHNHYTQKSLSLSPGK*

the chain 4 of F4-0G4S-1 (SEQ ID NO: 68):

DVVLTQSPLSLPVTLGQPASISC<u>KSSQSLLHSSGKTYLN</u>WLLQRPGQSPRRLIY<u>L</u>

<u>VSKLDS</u>GVPDRFSGSGSGTDFTLKISRVEAEDVGVYYC<u>AQGTHFPRT</u>FGGGTKV
EIK*RTVAAPSVFIFPPSDEQLKSGTASVVCLLNNFYPREAKVQWKVDNALQSGNSQES*
*VTEQDSKDSTYSLSSTLTLSKADYEKHKVYACEVTHQGLSSPVTKSFNRGEC*

The positive control molecule used in the present disclosure was JNJ-64407564. The sequences are as follows (from WO2018017786):

>the heavy chain (anti-GPRC5D) (SEQ ID NO: 69):

<u>QVQLVQSGAEVKKPGASVKVSCKASGYSFTGYTMNWVRQAPGQGLEWMGLI
NPYNSDTNYAQKLQGRVTMTTDTSTSTAYMELRSLRSDDTAVYYCARVALRV
ALDYWGQGTLVTVSS</u>ASTKGPSVFPLAPCSRSTSESTAALGCLVKDYFPEPVTV
SWNSGALTSGVHTFPAVLQSSGLYSLSSVVTVPSSSLGTKTYTCNVDHKPSNTK
VDKRVESKYGPPCPPCPAPEAAGGPSVFLFPPKPKDTLMISRTPEVTCVVVDVS
QEDPEVQFNWYVDGVEVHNAKTKPREEQFNSTYRVVSVLTVLHQDWLNGKE
YKCKVSNKGLPSSIEKTISKAKGQPREPQVYTLPPSQEEMTKNQVSLTCLVKGF
YPSDIAVEWESNGQPENNYKTTPPVLDSDGSFFLYSRLTVDKSRWQEGNVFSCS
VMHEALHNHYTQKSLSLSLGK

> the light chain (anti-GPRC5D) (SEQ ID NO: 70):

<u>DIQMTQSPSSLSASVGDRVTITCKASQNVATHVGWYQQKPGKAPKRLIYSASY
RYSGVPSRFSGSGSGTEFTLTISNLQPEDFATYYCQQYNRYPYTFGQGTKLEIK</u>*R*
*TVAAPSVFIFPPSDEQLKSGTASVVCLLNNFYPREAKVQWKVDNALQSGNSQESVTE*
*QDSKDSTYSLSSTLTLSKADYEKHKVYACEVTHQGLSSPVTKSFNRGEC*

> the heavy chain (anti-CD3E) (SEQ ID NO: 71):

EVQLVESGGGLVQPGGSLRLSCAASGFTFNTYAMNWVRQAPGKGLEWVARIR
SKYNNYATYYAASVKGRFTISRDDSKNSLYLQMNSLKTEDTAVYYCARHGNF
GNSYVSWFAYWGQGTLVTVSSASTKGPSVFPLAPCSRSTSESTAALGCLVKDY
FPEPVTVSWNSGALTSGVHTFPAVLQSSGLYSLSSVVTVPSSSLGTKTYTCNVD
HKPSNTKVDKRVESKYGPPCPPCPAPEAAGGPSVFLFPPKPKDTLMISRTPEVTC
VVVDVSQEDPEVQFNWYVDGVEVHNAKTKPREEQFNSTYRVVSVLTVLHQD
WLNGKEYKCKVSNKGLPSSIEKTISKAKGQPREPQVYTLPPSQEEMTKNQVSLT
CLVKGFYPSDIAVEWESNGQPENNYKTTPPVLDSDGSFLLYSKLTVDKSRWQE
GNVFSCSVMHEALHNHYTQKSLSLSLGK

> the light chain (anti-CD3E) SEQ ID NO: 72):

QTVVTQEPSLTVSPGGTVTLTCRSSTGAVTTSNYANWVQQKPGQAPRGLIGGT
NKRAPGTPARFSGSLLGGKAALTLSGVQPEDEAEYYCALWYSNLWVFGGGTK
LTVLGQPKAAPSVTLFPPSSEELQANKATLVCLISDFYPGAVTVAWKADSSPVK
AGVETTTPSKQSNNKYAASSYLSLTPEQWKSHRSYSCQVTHEGSTVEKTVAPT
ECS

> the heavy chain variable region of the humanized anti-GPRC5D antibody in JNJ-64407564 (SEQ ID NO: 73):

QVQLVQSGAEVKKPGASVKVSCKASGYSFTGYTMNWVRQAPGQGLEWMGLI
NPYNSDTNYAQKLQGRVTMTTDTSTSTAYMELRSLRSDDTAVYYCARVALRV

ALDYWGQGTLVTVSS

> the light chain variable region of the humanized anti-GPRC5D antibody in JNJ-64407564 (SEQ ID NO: 74):

DIQMTQSPSSLSASVGDRVTITCKASQNVATHVGWYQQKPGKAPKRLIYSASY
RYSGVPSRFSGSGSGTEFTLTISNLQPEDFATYYCQQYNRYPYTFGQGTKLEIK

> the heavy chain of JNJ7564-huIgG1 (SEQ ID NO: 75):

QVQLVQSGAEVKKPGASVKVSCKASGYSFTGYTMNWVRQAPGQGLEWMGLI
NPYNSDTNYAQKLQGRVTMTTDTSTSTAYMELRSLRSDDTAVYYCARVALRV
ALDYWGQGTLVTVSS*ASTKGPSVFPLAPSSKSTSGGTAALGCLVKDYFPEPVTVSW*
*NSGALTSGVHTFPAVLQSSGLYSLSSVVTVPSSSLGTQTYICNVNHKPSNTKVDKKVEP*
*KSCDKTHTCPPCPAPEAAGGPSVFLFPPKPKDTLMISRTPEVTCVVVDVSHEDPEVK*
*FNWYVDGVEVHNAKTKPREEQYNSTYRVVSVLTVLHQDWLNGKEYKCKVSNKALPA*
*PIEKTISKAKGQPREPQVYTLPPSRDELTKNQVSLTCLVKGFYPSDIAVEWESNGQPE*
*NNYKTTPPVLDSDGSFFLYSKLTVDKSRWQQGNVFSCSVMHEALHNHYTQKSLSLSP*
*GK*

> the light chain of JNJ7564-huIgG1 (SEQ ID NO: 76):

DIQMTQSPSSLSASVGDRVTITCKASQNVATHVGWYQQKPGKAPKRLIYSASY
RYSGVPSRFSGSGSGTEFTLTISNLQPEDFATYYCQQYNRYPYTFGQGTKLEIK*R*
*TVAAPSVFIFPPSDEQLKSGTASVVCLLNNFYPREAKVQWKVDNALQSGNSQESVTE*
*QDSKDSTYSLSSTLTLSKADYEKHKVYACEVTHQGLSSPVTKSFNRGEC*

**Test Examples-Assays on Bispecific Antibodies Specifically Binding to GPRC5D and CD3**

**Test Example 1. FACS Assay for Affinity of GPRC5D/CD3 Bispecific Antibodies for GPRC5D**

To test the ability of the GPRC5D/CD3 bispecific antibodies of the present disclosure to bind to GPRC5D, a flow cytometry assay was used in this test example to determine the ability of the GPRC5D/CD3 bispecific antibodies to bind to the human multiple myeloma cell line MM.1R (ATCC® CRL-2975), which expresses GPRC5D, and to a stably transfected cell strain CHOK1-cynoGPRC5D, which expresses cynomolgus monkey GPRC5D. The specific procedure was as follows: The cells described above were cultured in complete culture medium containing 10% FBS, incubated in a 5% $CO_2$ incubator at 37 °C for 2 days, and added to a cell plate at $1 \times 10^5$ cells/well. The plate was centrifuged at 300 g for 5 min and washed once with 1% BSA. The antibodies were serially diluted to 8 concentrations and added to the cell plate at 100 $\mu$L/well. The plate was incubated at 4 °C for 1 h and washed once with 1% BSA. A dilution of FITC-Goat Anti-human IgG Fc fluorescent secondary antibody (1:400) was added at 100 $\mu$L/well, and the plate was incubated at 4 °C for 1 h. The plate was washed three times with 1% BSA, PBS was added at 100 $\mu$L/well, and plate reading was performed. The $EC_{50}$ (nM) for the binding of each antibody to the corresponding cells is shown in the table below.

Table 9. Determination of the ability of chimeric antibodies to bind to GPRC5D

| Antibody | MM.1R | | CynoGPRC5D-CHOK1 | |
|---|---|---|---|---|
| | Max | $EC_{50}$ (nM) | Max | $EC_{50}$ (nM) |
| Chi-1 | 5214 | 1.43 | 7878 | 1.20 |
| Chi-24 | 4998 | 2.81 | 9633 | 3.96 |
| JNJ7564-huIgG 1 | 1632 | 109 | - | - |
| Note: JNJ7564-huIgG1 is an antibody obtained by combining the variable region sequence of the humanized anti-GPRC5D antibody from JNJ-64407564 with a heavy chain constant region set forth in SEQ ID NO: 45 and a light chain constant region set forth in SEQ ID NO: 46; "-" indicates no detectable binding. | | | | |

The results show that the GPRC5D chimeric antibodies of the present disclosure exhibited good binding ability to membrane surface GPRC5D and relatively good cross-binding activity with CynoGPRC5D. JNJ-64407564 exhibited a weaker ability to bind to humanGPRC5D than the antibodies of the present disclosure did, which is characterized by a very low binding response and an affinity for cynoGPRC5D that was so weak that almost no binding could be detected.

**Test Example 2. ELISA Assay for Epitope of GPRC5D to Which GPRC5D/CD3 Bispecific Antibodies Bind**

To determine the epitope of GPRC5D to which the GPRC5D/CD3 bispecific antibodies of the present disclosure bind, 4 extracellular domain peptides of human GPRC5D were synthesized, including an amino-terminal peptide (N-terminal peptide), an E2 peptide, an E3 peptide, and an E4 peptide (synthesized by GenScript). The peptides were biotinylated and made into two forms: one lacked intramolecular disulfide bonds and the other included intramolecular disulfide bonds. The sequences are shown in Table 10. An Elisa plate (corning 3590) was coated with streptavidin (sigma, SA101), left to stand at 37 °C for 2 h, and then washed. The non-specific binding sites were blocked with PBS containing 5% skim milk, and the plate was left to stand at 37 °C for 2 h and then washed. The peptides, diluted in PBS containing 1% BSA, were added at 100 $\mu$L/well, and the plate was left to stand at 37 °C for 1 h and then washed. Subsequently, the GPRC5D/CD3 antibodies, diluted in PBS containing 1% BSA to 0.003 nM-300 nM, were added at 100 $\mu$L/well, and the plate was left to stand at 37 °C for 1 h and then washed. Goat anti-human IgG (H+L)-HRP (Jackson ImmunoResearch, 209-035-088) was added to the plate, and the plate was left to stand at 37 °C for 1 h and then washed. TMB (KPL, 5120-0077) was added at 100 $\mu$L/well. After 5 min of color development, 1 M $H_2SO_4$ was added to stop the reaction, and the $OD_{450}$ was measured. The specific results are shown in Table 11.

Table 10. Sequences of 4 extracellular domain peptides of human GPRC5D

| Peptide | Sequence and sequence No. |
|---|---|
| N-terminal peptide | MYKDCIESTGDYFLLCDAEGPWGIILE (SEQ ID NO: 77) |
| E2 peptide | NQQTAPVRYC (SEQ ID NO: 78) |
| E3 peptide | TEYVTLIMTRGMMFVNMTPCQLN (SEQ ID NO: 79) |
| E4 peptide | RGNPQFQRQPQWDDC (SEQ ID NO: 80) |

Table 11. Assay for ability of F2-0G4S-1 and F4-0G4S-1 to bind to the 4 extracellular domain peptides of GPRC5D

| Antibody | N-terminal peptide | | E2 peptide | | E3 peptide | | E4 peptide | |
|---|---|---|---|---|---|---|---|---|
| | $EC_{50}$ (nM) | Max $OD_{450}$ | $EC_{50}$ (nM) | Max $OD_{450}$ | $EC_{50}$ (nM) | Max $OD_{450}$ | $EC_{50}$ (nM) | Max $OD_{450}$ |
| F2-0G4S-1 | 0.392 | 2.97 | - | - | - | - | - | - |
| F4-0G4S-1 | 0.597 | 3.12 | - | - | - | - | - | - |
| Note: "-" indicates no detectable binding. | | | | | | | | |

The results show that the GPRC5D/CD3 bispecific antibodies F2-0G4S-1 and F4-0G4S-1 of the present disclosure bound to the amino-terminal peptide sequence of human GPRC5D, regardless of whether intramolecular disulfide bonds form in the peptide. Meanwhile, neither of the test antibodies bound to the E2, E3, or E4 peptide. These results indicate that the epitope of GPRC5D to which the GPRC5D/CD3 bispecific antibodies F2-0G4S-1 and F4-0G4S-1 of the present disclosure bind is in the amino-terminal region of human GPRC5D.

**Test Example 3. FACS Assay for Cross-Binding of GPRC5D/CD3 Bispecific Antibodies to GPCRA and GPCRB Family Members**

To determine whether the GPRC5D/CD3 bispecific antibodies of the present disclosure cross-bind to the GPCRA and GPCRB family members, a flow cytometry assay was used in this test example to determine the ability of the GPRC5D/CD3 bispecific antibodies to bind to CHOK1-human CCR8 cells expressing human CCR8 (the GPCR A family) (constructed with reference to Example 1) and CHOK1-human GIPR cells expressing human GIPR (the GPCR B family) (constructed with reference to Example 1). The specific procedure was as follows: The cells described above were cultured in DMEF/12K culture medium containing 10% FBS, incubated in a 5% $CO_2$ incubator at 37 °C for 2 days, and added to a cell plate at $1 \times 10^5$ cells/well. The plate was centrifuged at 300 g for 5 min and washed once with 1% BSA. The antibodies were serially diluted to 8 concentrations and added to the cell plate at 100 μL/well. The plate was incubated at 4 °C for 1 h and washed once with 1% BSA. A dilution of FITC-Goat Anti-human IgG Fc fluorescent secondary antibody (1:400) was added at 100 μL/well, and the plate was incubated at 4 °C for 1 h. The plate was washed three times with 1% BSA, PBS was added at 100 μL/well, and plate reading was performed.

> Human CCR8 antigen (SEQ ID NO: 81):

MDYTLDLSVTTVTDYYYPDIFSSPCDAELIQTNGKLLLAVFYCLLFVFSLLGNSL
VILVLVVCKKLRSITDVYLLNLALSDLLFVFSFPFQTYYLLDQWVFGTVMCKVV
SGFYYIGFYSSMFFITLMSVDRYLAVVHAVYALKVRTIRMGTTLCLAVWLTAI

MATIPLLVFYQVASEDGVLQCYSFYNQQTLKWKIFTNFKMNILGLLIPFTIFMFC
YIKILHQLKRCQNHNKTKAIRLVLIVVIASLLFWVPFNVVLFLTSLHSMHILDGC
SISQQLTYATHVTEIISFTHCCVNPVIYAFVGEKFKKHLSEIFQKSCSQIFNYLGR
QMPRESCEKSSSCQQHSSRSSSVDYIL

> Human GIPR antigen (SEQ ID NO: 82):

RAETGSKGQTAGELYQRWERYRRECQETLAAAEPPSGLACNGSFDMYVCWDY
AAPNATARASCPWYLPWHHHVAAGFVLRQCGSDGQWGLWRDHTQCENPEK
NEAFLDQRLILERLQVMYTVGYSLSLATLLLALLILSLFRRLHCTRNYIHINLFTS
FMLRAAAILSRDRLLPRPGPYLGDQALALWNQALAACRTAQIVTQYCVGANY
TWLLVEGVYLHSLLVLVGGSEEGHFRYYLLLGWGAPALFVIPWVIVRYLYENT
QCWERNEVKAIWWIIRTPILMTILINFLIFIRILGILLSKLRTRQMRCRDYRLRLAR
STLTLVPLLGVHEVVFAPVTEEQARGALRFAKLGFEIFLSSFQGFLVSVLYCFIN
KEVQSEIRRGWHHCRLRRSLGEEQRQLPERAFRALPSGSGPGEVPTSRGLSSGT
LPGPGNEASRELESYC

The results show that none of the GPRC5D/CD3 bispecific antibodies F2-0G4S-1 and F4-0G4S-1 of the present disclosure bound to human CCR8 or human GIPR, indicating their good specificity.

**Test Example 4.** *In Vitro* **Cytotoxic Activity of Bispecific Antibodies of the Present Disclosure**

In this test example, the killing activity of the bispecific antibodies of the present disclosure as T cell engager molecules against tumor cells was investigated. The target-specific cytotoxic activity of the bispecific antibodies of the present disclosure was evaluated using the cell line H929, which expresses GPRC5D, and the cell line Daudi, which does not express GPRC5D, as target cells. Fresh PBMCs (purchased from Milestone) were centrifuged at 300 g for 10 min, and the supernatant was discarded. The cells were resuspended in 1640 + 10% FBS containing 200 U IL2 (solution A) to form a 2E6/mL cell suspension, and the cell suspension was cultured overnight in a culture flask. The overnight-activated PBMCs were collected, centrifuged, resuspended in phenol red-free 1640 + 4% FBS, centrifuged again, resuspended, counted, adjusted to a cell concentration of 6.25E5 cells/mL, and added at 60 μL/well (37500 PBMCs per well). Target cells NCI-H929 (ATCC, CRL-9068) were collected, centrifuged at 1000 rpm for 3 min, adjusted to a cell concentration of 1.25E5 cells/mL, and added at 60 μL/well (7500 target cells per well) to ensure an E:T ratio of 5:1. The antibodies were serially diluted 10-fold in phenol red-free complete culture medium to 9 gradients with an initial concentration of 500 nM (5× final concentration), and added at 30 μL/well. The cells were then incubated in a 5% $CO_2$ incubator at 37 °C for 48 h. Before measurement, 10 μL of culture medium was pipetted from two wells containing only target cells, and 10 μL of Lysis Solution (10×) was then added. After 45 min of lysis, the culture plate was taken out and centrifuged at 1000 rpm for 3 min, and 50 μL of the supernatant was transferred to a new 96-well plate (#3590). According to a 1:1 ratio, 50 μL of dissolved CytoTox 96® Reagent was added. After 0.5 h of incubation at room temperature, 50 μL of stop solution was added, and the absorbance (490 nm) was measured using a FlexStation 3 (Molecular Devices). The specific results are shown in the table below.

Table 12. Cytotoxic activity of antibodies against NCI-H929

| Antibody | $EC_{50}$ ratio (relative to JNJ-64407564) | Lysis rate% |
|---|---|---|
| Experiment 1 | | |
| F2-0G4S-1 | 0.57 | 91.49 |
| JNJ-64407564 | 1.00 | 115.61 |
| Experiment 2 | | |
| F4-0G4S-1 | 0.09 | 135.57 |
| JNJ-64407564 | 1.00 | 153.42 |
| Note: The $EC_{50}$ ratio (relative to JNJ-64407564) = $EC_{50}$ (test antibody)/$EC_{50}$ (JNJ-64407564). | | |

The results show that the GPRC5D/CD3 bispecific antibodies of the present disclosure exhibited stronger cytotoxic activity against NCI-H929 cells than JNJ-64407564 did; furthermore, the cytotoxic activity of the GPRC5D/CD3 bispecific antibodies F2-0G4S-1 and F4-0G4S-1 of the present disclosure was specific to the GPRC5D target, as no killing activity was detected in the Daudi cell line, which does not express GPRC5D, suggesting that the GPRC5D/CD3 bispecific antibodies of the present disclosure are safer.

**Test Example 5. Cytokine Release Levels of GPRC5D/CD3 Bispecific Antibodies**

CD3 T cell engager molecules can induce cytokine storms. Therefore, when developing CD3 T cell engager molecules, it is necessary to be able to maintain low levels of cytokines, especially IL6, which is unrelated to efficacy but can cause side effects. In this test example, the release levels of the cytokines IFN$\gamma$ and IL6 of the bispecific antibodies of the present disclosure were investigated. Fresh PBMCs (purchased from Milestone) were centrifuged at 300 g for 10 min, and the supernatant was discarded. The cells were resuspended in 1640 + 10% FBS containing 200 U IL2 (solution A) to form a 2E6/mL cell suspension, and the cell suspension was cultured overnight in a culture flask. The overnight-activated PBMCs were collected, centrifuged, resuspended in phenol red-free 1640 + 4% FBS, centrifuged again, resuspended, counted, adjusted to a cell concentration of 6.25E5 cells/mL, and added at 60 $\mu$L/well (37500 PBMCs per well). Target cells NCI-H929 (ATCC, CRL-9068) or RPMI-8226 (ATCC, CRM-CCL-155) were collected, centrifuged at 1000 rpm for 3 min, adjusted to a cell concentration of 1.25E5 cells/mL, and added at 60 $\mu$L/well (7500 target cells per well) to ensure an E:T ratio of 5:1. The antibodies were serially diluted 10-fold in phenol red-free complete culture medium to 9 gradients with an initial concentration of 500 nM (5$\times$ final concentration), and added at 30 $\mu$L/well. The cells were then cultured in a 5% $CO_2$ incubator at 37 °C for 48 h for later use.

The IL6 assay was performed using the ELISA method. The cell sample described above was centrifuged at 1000 rpm for 3 min, and 50 $\mu$L of the supernatant was collected and diluted 6-fold with a general specimen diluent. The IL6 standard was also diluted 6-fold. The diluted samples or the standards at different concentrations were added to an assay plate (100 $\mu$L/well), and the reaction wells were sealed with plate-sealing adhesive paper. The plate was incubated at 37 °C for 90 min and then washed 5 times. A biotinylated antibody working solution was added (100 $\mu$L/well), and the reaction wells were sealed with new plate-sealing adhesive paper. The plate was incubated at 37 °C for 60 min and then washed 5 times. An enzyme conjugate working solution was added (100 $\mu$L/well), and the reaction wells were sealed with new plate-sealing adhesive paper. The plate was incubated at 37 °C for 30 min and then washed 5 times. The chromogenic substrate (TMB) was added at 100 $\mu$L/well, and the plate was incubated in the absence of light at 37 °C for 8 min. The reaction stop solution was added at 100 $\mu$L/well, and immediately after thorough mixing, the $OD_{450}$ values were measured (within 3 min).

The IFN$\gamma$ assay was performed using the HTRF method. The cell sample described above was centrifuged at 1000 rpm for 3 min, and 50 $\mu$L of the supernatant was collected. The assay kit was equilibrated to room temperature, and the two assay antibodies in the kit were diluted (20-fold) with the assay buffer. 16 $\mu$L of the 20-fold diluted sample and the IFN$\gamma$ standard were added to a 384-well plate, and 4 $\mu$L of the corresponding diluted assay antibody was added. A sealing material was attached, and the plate was shaken for thorough mixing, centrifuged at 1000 rpm for 1 min, incubated overnight at room temperature, and then centrifuged at 1000 rpm for 1 min. The sealing material was removed. The absorbance values at 665 nm and 620 nm were measured using a PHERAstar multi-mode microplate reader, and data were processed and analyzed using Graphpad Prism 5. The specific results are shown in the table below.

Table 13-1. IL-6 release in the RPMI-8226 cytotoxic activity experiment

| Antibody concentration (pM) | F4-0G4S-1 | JNJ-64407564 |
|---|---|---|
| | IL-6 (pg/mL) | |
| 100000 | - | 359.1 |
| 10000 | - | 331.3 |
| 1000 | - | 155.8 |

Note: "-" indicates that the level was undetectable as it was below the lower limit of detection. This applies hereinafter.

Table 13-2. IL-6 release in the RPMI-8226 cytotoxic activity experiment

| Antibody concentration (pM) | F2-0G4S-1 | JNJ-64407564 |
|---|---|---|
| | IL-6 (pg/mL) | |
| 100000 | 11.03 | 818.1 |
| 10000 | - | 727.6 |
| 1000 | - | 456.1 |
| 100 | 0.92 | 10.7 |

Table 13-3. IFNγ release in the RPMI-8226 cytotoxic activity experiment

| Antibody concentration (pM) | F4-0G4S-1 | JNJ-64407564 |
|---|---|---|
| | IFNγ (pg/mL) | |
| 100000 | 273.25 | 861.36 |
| 10000 | - | 643.74 |
| 1000 | - | - |

Table 13-4. IFNγ release in the RPMI-8226 cytotoxic activity experiment

| Antibody concentration (pM) | F2-0G4S-1 | JNJ-64407564 |
|---|---|---|
| | IFNγ (pg/mL) | |
| 100000 | 375.79 | 2318.22 |
| 10000 | - | 4553.04 |
| 1000 | - | 967.36 |

Table 13-5. IL-6 release in the NCI-H929 cytotoxic activity experiment

| Antibody concentration (pM) | F4-0G4S-1 | JNJ-64407564 |
|---|---|---|
| | IL-6 (pg/mL) | |
| 100000 | - | 430.74 |
| 10000 | - | 328.41 |
| 1000 | - | 184.23 |

Table 13-6. IL-6 release in the NCI-H929 cytotoxic activity experiment

| Antibody concentration (pM) | F2-0G4S-1 | JNJ-64407564 |
|---|---|---|
| | IL-6 (pg/mL) | |
| 100000 | 47.1 | 1166.29 |
| 10000 | 21.1 | 1027.14 |
| 1000 | 9.6 | 580.21 |
| 100 | 5.3 | 36.57 |

The results show that the GPRC5D/CD3 bispecific antibodies of the present disclosure caused lower levels of release of IL6 and IFNγ, particularly IL6, which was almost undetectable, than JNJ-64407564 did. This suggests that the GPRC5D/CD3 bispecific antibodies of the present disclosure are safer.

## Biological Evaluations of *In Vivo* Activity

### Test Example 6. Efficacy of GPRC5D/CD3 Bispecific Antibodies in NCIH929 Subcutaneous Graft Tumor Model

In the present disclosure, the anti-tumor activity of the GPRC5D/CD3 bispecific antibodies was evaluated using a human myeloma NCI-H929 cell human PBMC NOG mouse xenograft tumor model.

NOG mice, female, 8-10 weeks old, were purchased from Beijing Vital River Laboratory Animal Technology Co., Ltd.

The human multiple myeloma cell strain NCI-H929 was purchased from Shanghai MingJing Biotech, Co., Ltd.

NCI-H929 cells were added to RPMI-1640 culture medium containing 10% FBS and 0.05 mM 2-mercaptoethanol and cultured in a 5% $CO_2$ incubator at 37 °C with saturated humidity. NCI-H929 cells in the logarithmic growth phase were collected and resuspended in RPMI-1640 culture medium containing 50% matrigel, and the cell concentration was adjusted to $2 \times 10^7$/mL. Under sterile conditions, 0.1 mL of the cell suspension was inoculated subcutaneously into the right side of the back of the mice, with an inoculation concentration of $2 \times 10^6$/0.1 mL/mouse. One day after tumor cell inoculation, hPBMCs cryopreserved in liquid nitrogen were thawed, cultured in PRMI-1640 culture medium containing 10% HIFBS (FBS, inactivated at 56 °C for 30 min), and incubated for 6 h in a 5% $CO_2$ incubator at 37 °C. After incubation, the hPBMCs were collected and resuspended in PBS buffer, and the cell concentration was adjusted to $2.5 \times 10^7$/mL. Under sterile conditions, 0.2 mL of the cell suspension was intraperitoneally injected into the mice, with an injection concentration of $5 \times 10^6$/0.2 mL/mouse. When the mean tumor volume reached about 100 mm$^3$, the animals were randomly grouped according to tumor volume, with the difference in tumor volume between groups being less than 10% of the mean. The day of grouping was denoted by day 0, and administration was started according to animal body weight. Administration was performed immediately after grouping. Intraperitoneal administration was performed once a week for 2 consecutive weeks (IP, qW $\times$ 2), and tumor measurements were recorded.

$$\text{Tumor growth inhibition rate (\%)} = 1 - \text{T/C (\%)}.$$

$$\text{T/C (\%)} = (\text{T} - \text{T0})/(\text{C} - \text{C0}) \times 100\%.$$

Tumor volume (V) = $1/2 \times a \times b^2$, where a and b represent length and width, respectively. T and C are the tumor volumes of a treatment group and the control group at the end of the experiment, and T0 and C0 are the tumor volumes at the beginning of the experiment. The specific results are shown in the table below. The tumor growth inhibition rates calculated in the tables are tumor growth inhibition rates of the last day.

Table 14. Efficacy of F4-0G4S-1 and F2-0G4S-1 in the NCIH929 subcutaneous graft tumor model

| Tumor volume (mm$^3$) | | | | | | | |
|---|---|---|---|---|---|---|---|
| Days after administration | | Dose 0.05 mpk | | | Dose 0.2 mpk | | |
| | PBS | JNJ-64407564 | F4-0G4S-1 | F2-0G4S-1 | JNJ-64407564 | F4-0G4S-1 | F2-0G4S-1 |
| 0 | 108.1 | 110.7 | 109.8 | 106.1 | 109.3 | 105.8 | 107.7 |
| 3 | 256.5 | 288.9 | 195.6 | 235.9 | 268 | 189.2 | 188.9 |
| 6 | 493.7 | 489.8 | 173.1 | 342.5 | 370.2 | 136.7 | 110.6 |
| 9 | 935.4 | 809.4 | 150 | 487.9 | 406.2 | 57.1 | 31.5 |
| 12 | 1587.6 | 1471.8 | 212.8 | 864 | 644.6 | 5.2 | 4.5 |
| Tumor growth inhibition rate (%) | | | | | | | |
| | 0.0% | 8.0% | 93.0% | 48.8% | 63.8% | 106.8% | 107.0% |

The experimental results show that the GPRC5D/CD3 bispecific antibodies F2-0G4S-1 and F4-0G4S-1 of the present disclosure exhibited higher *in vivo* tumor growth inhibition activity at the doses than JNJ-64407564 did at the corresponding doses. No drug-related animal deaths or other significant drug-related toxic and side effects were observed during the experiment.

**Preparation Examples-Formulations of Bispecific Antibody Specifically Binding to GPRC5D and CD3**

**SEC molecular exclusion chromatography:**

This is an analytical method that separates a solute based on the relative relationship between the pore size of the gel pores and the coil size of the polymer sample molecule. SEC% (SEC monomer content percentage) = A monomer/A total $\times$ 100% (A monomer represents the peak area of the main peak monomer in the sample, and A total represents the sum of all peak areas). $\Delta$SEC% = SEC% of formulation before stability experiment - SEC% of formulation after stability experiment.

Instrument for SEC analysis: Agilent HPLC 1260;
Column: Waters, XBridge® BEH200Å SEC 3.5 $\mu$m 7.8 $\times$ 300 mm Column.

**NR-CE capillary gel electrophoresis:**

This is a type of electrophoresis in which a gel is moved into a capillary as a support medium and a method that achieves separation under a certain voltage based on the molecular weight of the sample.

NR-CE% (NR-CE main peak content percentage) = A main peak/A total × 100% (A main peak represents the peak area of the main peak in the sample, and A total represents the sum of all peak areas). ΔNR-CE% = NR-CE% of formulation before stability experiment - NR-CE% of formulation after stability experiment.

Instrument for CE analysis: Beckman capillary electrophoresis system; model: PA800 plus.

**IEC ion exchange chromatography:**

This is a chromatographic method that uses an ion exchange resin or chemically bonded ion exchanger as the stationary phase and achieves separation based on differences in ion exchange ability or selectivity coefficient among the components to be separated.

IEC% (IEC neutral peak content percentage) = A neutral peak area/A total area × 100% (A total area represents the sum of areas of acidic, neutral, and basic peaks). ΔIEC% = IEC% of formulation before stability experiment - IEC% of formulation after stability experiment.

Instrument for IEC analysis: Agilent HPLC 1260.

**iCIEF whole column imaging capillary isoelectric focusing electrophoresis:**

This is a technique for separation according to the difference in isoelectric points (pI) of proteins.

iCIEF% (iCIEF neutral peak content percentage) = A neutral peak area/A total area × 100% (A total area represents the sum of areas of acidic, neutral, and basic peaks). ΔiCIEF% = iCIEF% of formulation before stability experiment - iCIEF% of formulation after stability experiment.

Instrument for iCIEF analysis: simple protein, model: Muarice.

**ELSD anion exchange and reversed-phase adsorption chromatography:**

This is a method for quantifying polysorbate 80 by separating solutes based on the principles of anion exchange and reversed-phase adsorption, followed by analysis using charged aerosol detection technique.

PS80 degradation rate% = [PS80 concentration of formulation before stability experiment (mg/mL) - PS80 concentration of formulation after stability experiment (mg/mL)]/PS80 concentration of formulation before stability experiment (mg/mL).

Instrument for ELSD analysis: Agilent HPLC 1260.

**Osmolality determination:**

The freezing point method is used for measuring osmolality. On the basis of the directly proportional relationship between the freezing point depression value and the molar concentration of a solution, the freezing point of the solution is determined using a highly sensitive temperature-sensing element and then converted into the osmolality through electric quantity.

Instrument for osmolality determination: Loser; model: OM815.

**Protein**

The protein used in the following formulation examples was the GPRC5D-CD3 bispecific antibody F4-0G4S-1 described above (hereinafter referred to as "protein").

Instrument for protein concentration determination: ultraviolet-visible spectrophotometer; model: Nano Drop oneC; optical path length: 1 mm.

**Formulation Example 1. pH and Buffer System Screening**

Formulations containing 100 mg/mL protein, 80 mg/mL sucrose, and 0.4 mg/mL polysorbate 80 (PS80) were prepared using the buffer systems shown in Table 15-1. Samples were subjected to a forced degradation study (storage at 40 °C for 2 weeks), and the effect of the different pHs and buffer systems on protein stability was evaluated based on SEC, NR-CE, and iCIEF.

The results are shown in Table 15-1. The SEC data show that after storage at 40 °C for 2 weeks, the formulations with a pH value of 5.5-6.0 showed better monomer purity than the other groups did. The NR-CE data show that ΔNR-CE was within a fluctuation range of 0.4%-1.4%, and the difference between groups was small. The iCIEF data show that the neutral peak purity of the formulations containing AA, His, and CA systems was better than that of the other groups.

Table 15-1. pH and buffer system screening results-1

| Group | Buffer system | Conditions | ΔSEC% | ΔNR-CE% | ΔiCIEF% |
|---|---|---|---|---|---|
| 1 | 20 mM AA pH5.0 | 40 °C W2 | 2.2 | 1.3 | 15.3 |
| 2 | 20 mM AA pH5.5 | 40 °C W2 | 1.6 | 0.5 | 13.0 |
| 3 | 20 mM His-HCl pH5.5 | 40 °C W2 | 1.5 | 1.1 | 12.8 |
| 4 | 20 mM His-HCl pH6.0 | 40 °C W2 | 1.3 | 0.7 | 12.2 |
| 5 | 20 mM His-HCl pH6.5 | 40 °C W2 | 3.2 | 0.7 | 14.3 |
| 6 | 20 mM SA pH5.5 | 40 °C W2 | 1.6 | 0.4 | 25.7 |
| 7 | 20 mM CA pH5.5 | 40 °C W2 | 1.5 | 1.0 | 12.8 |
| 8 | 20 mM PB pH6.5 | 40 °C W2 | 2.5 | 0.8 | 21.9 |
| 9 | 20 mM PB pH7.0 | 40 °C W2 | 5.2 | 1.4 | 29.3 |

Note: AA represents acetic acid-sodium acetate; His-HCl represents histidine-histidine hydrochloride; SA represents succinic acid-sodium succinate; CA represents citric acid-sodium citrate; PB represents disodium hydrogen phosphate-sodium dihydrogen phosphate; 40 °C W2: stored at 40 °C for 2 weeks. This applies hereinafter.

Samples were subjected to a forced degradation study (shaking at 300 rpm at 25 °C for 7 days), and the effect of different pHs and buffer systems on protein stability was evaluated based on appearance.

The results are shown in Table 15-2. The appearance results show that after 7 days of shaking, fine particles were observed in the appearance of the formulations containing SA and CA.

Table 15-2. pH and buffer system screening results-2

| Group | Buffer system | Conditions | Appearance |
|---|---|---|---|
| 1 | 20 mM AA pH5.0 | Shaking D7 | Clear and transparent |
| 2 | 20 mM AA pH5.5 | Shaking D7 | Clear and transparent |
| 3 | 20 mM His-HCl pH5.5 | Shaking D7 | Clear and transparent |
| 4 | 20 mM His-HCl pH6.0 | Shaking D7 | Clear and transparent |
| 5 | 20 mM His-HCl pH6.5 | Shaking D7 | Clear and transparent |
| 6 | 20 mM SA pH5.5 | Shaking D7 | A small number of fine particles |
| 7 | 20 mM CA pH5.5 | Shaking D7 | Fine particles, slightly opalescent |
| 8 | 20 mM PB pH6.5 | Shaking D7 | Slightly opalescent |
| 9 | 20 mM PB pH7.0 | Shaking D7 | Opalescent |

Note: shaking D7: shaking for 7 days; this applies hereinafter.

In summary, the data from the forced degradation studies (storage at 40 °C for 2 weeks; shaking at 300 rpm at 25 °C for 7 days) show that the formulations at pH 5.5-6.0 using the AA and His-HCl systems were better than the other groups.

**Formulation Example 2. Buffer System Ionic Strength Screening**

Formulations containing 100 mg/mL protein, 80 mg/mL sucrose, 0.4 mg/mL PS80, and 0.1 mg/mL edetate disodium (CAS No.: 6381-92-6, purchased from SIGMA, this applies hereinafter) were prepared using the buffer systems with different ionic strengths shown in Table 16. Samples were subjected to a forced degradation study (storage at 40 °C for 4 weeks), and the effect of the different buffer system ionic strengths on protein stability was evaluated based on appearance, SEC, NR-CE, and IEC.

The results are shown in Table 16. The data show that after storage at 40 °C for 4 weeks, the formulations containing buffer systems with different ionic strengths showed no significant difference.

Table 16. Buffer system ionic strength screening results

| Group | Buffer system ionic strength | Storage conditions | Appearance | ΔSEC % | ΔNR-CE% | ΔIE C% |
|---|---|---|---|---|---|---|
| 1 | 5 mM His-HCl 5.5 | 40 °C W4 | Clear and transparent | 6.6 | 4.2 | 25.2 |
| 2 | 20 mM His-HCl 5.5 | 40 °C W4 | Clear and transparent | 6.6 | 4.2 | 27.0 |
| 3 | 50 mM His-HCl 5.5 | 40 °C W4 | Clear and transparent | 6.4 | 4.5 | 26.2 |

**Formulation Example 3. High Protein Concentration Screening**

Formulations containing 80 mg/mL sucrose, 0.4 mg/mL PS80, and the protein at concentrations shown in Table 17 were prepared using 20 mM His-HCl buffer (pH 5.5). Samples were subjected to a forced degradation study (storage at 40 °C for 4 weeks) and subjected to storage at 2-8 °C for 6 months. Due to the high risk of SEC aggregate formation at high protein concentrations, the effect of different protein concentrations on protein stability was evaluated based on SEC.

The results are shown in Table 17. The data show that as the protein concentration increased, ΔSEC tended to increase slightly, but the differences between the groups were small, and the samples showed good stability.

Table 17. Protein concentration screening results

| Group | Protein concentration (mg/mL) | Storage conditions | ΔSEC% |
|---|---|---|---|
| 1 | 60 | 40 °C W4 | 3.2 |
| | | 2-8 °C M6 | 0.7 |
| 2 | 100 | 40 °C W4 | 3.9 |
| | | 2-8 °C M6 | 1.3 |
| 3 | 150 | 40 °C W4 | 4.4 |
| | | 2-8 °C M6 | 1.3 |
| Note: 40 °C W4: stored at 40 °C for 4 weeks; 2-8 °C M6: stored at 2-8 °C for 6 months. This applies hereinafter. | | | |

**Formulation Example 4. Low Protein Concentration Screening**

Formulations containing 80 mg/mL sucrose, 0.4 mg/mL PS80, 0.1 mg/mL edetate disodium, and the protein at concentrations shown in Table 18 were prepared using 20 mM His-HCl buffer (pH 5.5). Samples were subjected to a forced degradation study (storage at 40 °C for 4 weeks). Due to a low risk of SEC aggregate formation at low protein concentrations, the effect of different protein concentrations on protein stability was evaluated based on appearance, NR-CE, and IEC.

The results are shown in Table 18. The data show that after storage at 40 °C for 4 weeks, the formulation groups containing protein at different concentrations showed relatively small differences in NR-CE and IEC.

Table 18. Protein concentration screening results

| Group | Protein concentration (mg/mL) | Storage conditions | Appearance | ΔNR-CE% | ΔIEC % |
|---|---|---|---|---|---|
| 1 | 1 | 40 °C W4 | Clear and transparent | 3.8 | 25.0 |
| 2 | 5 | 40 °C W4 | Clear and transparent | 3.7 | 26.0 |
| 3 | 100 | 40 °C W4 | Clear and transparent | 4.2 | 27.0 |

**Formulation Example 5. Protein Concentration Confirmation**

Formulations containing 80 mg/mL sucrose, 0.4 mg/mL PS80, 0.1 mg/mL edetate disodium, and the protein at concentrations shown in Table 19 were prepared using 20 mM His-HCl buffer (pH 5.5). Samples were subjected to a forced degradation study (storage at 40 °C for 4 weeks) and a long-term stability study at 2-8 °C, and the effect of different protein concentrations on protein stability was evaluated based on appearance, SEC, NR-CE, and IEC.

The results are shown in Table 19. The data show that after storage at 40 °C for 4 weeks, the groups showed relatively small difference in purity. After storage at 2-8 °C for 3 months, the 2 mg/mL and 60 mg/mL protein concentration groups showed no significant change in purity; after storage at 2-8 °C for 12 months, the 100 mg/mL protein concentration group showed no significant change in NR-CE and showed slight reductions in SEC and IEC. This suggests that samples containing proteins at different concentrations all exhibit relatively good stability.

Table 19. Protein concentration screening results

| Group | Protein concentration (mg/mL) | Storage conditions | Appearance | ΔSEC% | ΔNR-CE% | ΔIEC % |
|---|---|---|---|---|---|---|
| 1 | 2 | 40 °C W4 | Clear and transparent | 3.4 | 3.2 | 25.7 |
| | | 2-8 °C M3 | Clear and transparent | 0.5 | 0.1 | 1.0 |
| 2 | 60 | 40 °C W4 | Clear and transparent | 5.1 | 3.2 | 26.8 |
| | | 2-8 °C M3 | Clear and transparent | 0.2 | 0.2 | 1.4 |
| 3 | 100 | 40 °C W4 | Clear and transparent | 5.7 | 4.2 | 27.0 |
| | | 2-8 °C M12 | Clear and transparent | 0.8 | 0.1 | 5.5 |
| Note: 2-8 °C M12: stored at 2-8 °C for 12 months. 2-8 °C M3: stored at 2-8 °C for 3 months. | | | | | | |

## Formulation Example 6. Auxiliary Material Screening

Formulations containing 100 mg/mL protein, edetate disodium (added or omitted according to Table 20-1), 80 mg/mL sucrose, and 0.4 mg/mL PS80 were prepared using 20 mM His-HCl buffer (pH 5.5).

Table 20-1. Screening of different auxiliary materials

| Group | Buffer | Auxiliary material | Sucrose concentration | Protein concentration | Surfactant |
|---|---|---|---|---|---|
| 1 | 20 mM His-HCl 5.5 | N/A | 80 mg/mL | 100 mg/mL | 0.4 mg/mL PS80 |
| 2 | | 0.1 mg/mL edetate disodium | | | |

Samples were subjected to a forced degradation study (storage at 40 °C for 4 weeks), and the effect of different auxiliary materials on formulation stability was evaluated based on the PS80 degradation rate. The results are shown in Table 20-2. The PS80 degradation rate data show that after storage at 40 °C for 4 weeks, the PS80 degradation rate of the formulation containing edetate disodium was significantly lower than that of the formulation without edetate disodium.

Table 20-2. Auxiliary material screening results

| Group | Auxiliary material | Storage conditions | PS80 degradation rate% |
|---|---|---|---|
| 1 | N/A | 40 °C W4 | 84.3 |
| 2 | 0.1 mg/mL edetate disodium | 40 °C W4 | 16.2 |

## Formulation Example 7. Edetate Disodium Concentration Screening

Formulations containing 100 mg/mL protein, edetate disodium at different concentrations shown in Table 21, 80 mg/mL sucrose, and 0.4 mg/mL PS80 were prepared using 20 mM His-HCl buffer (pH 5.5). Samples were subjected to a forced degradation study (storage at 40 °C for 4 weeks), and the effect of different auxiliary materials on formulation stability was evaluated based on SEC, NR-CE, IEC, and PS80 degradation rate.

The results are shown in Table 21, and the data show that after storage at 40 °C for 4 weeks, the formulations containing edetate disodium at different concentrations showed no significant difference.

Table 21. Edetate disodium concentration screening results

| Group | Edetate disodium concentration (mg/mL) | Storage conditions | ΔSEC % | ΔNR CE% | ΔIEC % | PS80 degradatio n rate% |
|---|---|---|---|---|---|---|
| 1 | 0.01 | 40 °C W4 | 6.8 | 4.5 | 27.5 | 1.2 |
| 2 | 0.1 | 40 °C W4 | 6.6 | 4.2 | 27.0 | 1.3 |
| 2 | 1.0 | 40 °C W4 | 6.0 | 4.4 | 26.1 | 0.0 |

**Formulation Example 8. Sugar Concentration Screening**

Sugar is used as a common auxiliary material for biological drugs. In the present disclosure, sucrose was used as an osmotic pressure regulator and a stabilizer for formula screening, which can meet the formulation requirements.

Formulations containing 100 mg/mL protein, 0.4 mg/mL PS80, 0.1 mg/mL edetate disodium, and sucrose at different concentrations shown in Table 22 were prepared using 20 mM His-HCl buffer (pH 5.5). Samples were subjected to a forced degradation study (storage at 40 °C for 4 weeks), and the effect of the different sucrose concentrations on protein stability was evaluated based on appearance, SEC, NR-CE, and IEC.

The results are shown in Table 22. The data show that after storage at 40 °C for 4 weeks, the formulations containing sucrose at different concentrations showed no significant difference.

Table 22. Sucrose concentration screening results

| Group | Sucrose concentration (mg/mL) | Storage conditions | Appearance | ΔSEC % | ΔNR-CE% | ΔIEC % |
|---|---|---|---|---|---|---|
| 1 | 30 | 40 °C W4 | Clear and transparent | 6.3 | 4.5 | 27.0 |
| 2 | 80 | 40 °C W4 | Clear and transparent | 6.6 | 4.2 | 27.0 |
| 3 | 100 | 40 °C W4 | Clear and transparent | 5.9 | 4.3 | 28.1 |

**Formulation Example 9. Surfactant Type Screening**

Formulations containing 100 mg/mL protein, 80 mg/mL sucrose, and different types of surfactants shown in Table 23 were prepared using 20 mM His-HCl buffer (pH 5.5). Samples were subjected to a forced degradation study (storage at 40 °C for 4 weeks), and the effect of the different types of surfactants on protein stability was evaluated based on SEC, NR-CE, and IEC.

The results are shown in Table 23. The data show that after storage at 40 °C for 4 weeks, the formulations containing different types of surfactants showed no significant difference.

Table 23. Surfactant type screening results

| Group | Surfactant | Storage conditions | ΔSEC% | ΔNR-CE% | ΔiCIEF% |
|---|---|---|---|---|---|
| 1 | 0.4 mg/mL PS80 | 40 °C W4 | 3.9 | 2.4 | 20.3 |
| 2 | 2.0 mg/mL PF68 | 40 °C W4 | 3.5 | 2.0 | 18.7 |

**Formulation Example 10. Surfactant Concentration Screening-1**

Formulations containing 100 mg/mL protein, 80 mg/mL sucrose, 0.1 mg/mL edetate disodium, and PS80 at different concentrations shown in Table 24 were prepared using 20 mM His-HCl buffer (pH 5.5). Samples were subjected to a forced degradation study (storage at 40 °C for 4 weeks, shaking for 7 days), and the effect of surfactant at different concentrations on formulation stability was evaluated based on SEC.

The results are shown in Table 24. The appearance and SEC data show that after storage at 40 °C for 4 weeks and 7 days of shaking, the formulations containing PS80 at different concentrations showed no significant difference.

Table 24. Edetate disodium concentration screening results

| Group | PS80 concentration (mg/mL) | Storage conditions | ΔSEC% |
|---|---|---|---|
| 1 | 0.1 | 40 °C W4 | 5.9 |
| | | Shaking D7 | 0.3 |
| 2 | 1.0 | 40 °C W4 | 5.9 |
| | | Shaking D7 | 0.5 |

**Formulation Example 11. Surfactant Concentration Screening-2**

Formulations containing 100 mg/mL protein, 80 mg/mL sucrose, 0.1 mg/mL edetate disodium, and PS80 at different concentrations shown in Table 25 were prepared using 20 mM His-HCl buffer (pH 5.5). Samples were subjected to a forced degradation study (storage at 40 °C for 4 weeks), and the effect of surfactant at different concentrations on protein stability was evaluated based on appearance, SEC, NR-CE, and IEC.

The results are shown in Table 25. The data show that after storage at 40 °C for 4 weeks, the formulations containing PS80 at different concentrations showed no significant difference.

Table 25. Surfactant screening results

| Group | Surfactant | Storage conditions | Appearance | ΔSEC% | ΔNR-CE% | ΔIEC% |
|---|---|---|---|---|---|---|
| 1 | 0.2 mg/mL PS80 | 40 °C W4 | Clear and transparent | 5.4 | 2.9 | 25.8 |
| 2 | 0.4 mg/mL PS80 | 40 °C W4 | Clear and transparent | 5.2 | 3.2 | 27.4 |
| 3 | 0.6 mg/mL PS80 | 40 °C W4 | Clear and transparent | 5.5 | 3.0 | 26.5 |

## Claims

1.  A pharmaceutical composition, comprising a bispecific antibody specifically binding to GPRC5D and CD3 and a buffer, wherein:

the bispecific antibody specifically binding to GPRC5D and CD3 comprises one antigen-binding moiety 1 specifically binding to GPRC5D and one antigen-binding moiety 2 specifically binding to CD3, the antigen-binding moiety 1 specifically binding to GPRC5D comprises a heavy chain variable region GPRC5D-VH and a light chain variable region GPRC5D-VL, and the antigen-binding moiety 2 specifically binding to CD3 comprises a heavy chain variable region CD3-VH and a light chain variable region CD3-VL, wherein:

(i) the GPRC5D-VH comprises: a GPRC5D-HCDR1 comprising the amino acid sequence of SEQ ID NO: 11, a GPRC5D-HCDR2 comprising the amino acid sequence of SEQ ID NO: 12, and a GPRC5D-HCDR3 comprising the amino acid sequence of SEQ ID NO: 13; and the GPRC5D-VL comprises: a GPRC5D-LCDR1 comprising the amino acid sequence of SEQ ID NO: 43, 14, 41, 42, or 44, a GPRC5D-LCDR2 comprising the amino acid sequence of SEQ ID NO: 15, and a GPRC5D-LCDR3 comprising the amino acid sequence of SEQ ID NO: 16; or
the GPRC5D-VH comprises: a GPRC5D-HCDR1 comprising the amino acid sequence of SEQ ID NO: 5, a GPRC5D-HCDR2 comprising the amino acid sequence of SEQ ID NO: 31, 6, or 30, and a GPRC5D-HCDR3 comprising the amino acid sequence of SEQ ID NO: 7; and the GPRC5D-VL comprises: a GPRC5D-LCDR1 comprising the amino acid sequence of SEQ ID NO: 27, 8, 26, 28, or 29, a GPRC5D-LCDR2 comprising the amino acid sequence of SEQ ID NO: 9, and a GPRC5D-LCDR3 comprising the amino acid sequence of SEQ ID NO: 10; and
(ii) the CD3-VH comprises: a CD3-HCDR1 comprising the amino acid sequence of SEQ ID NO: 51, a CD3-HCDR2 comprising the amino acid sequence of SEQ ID NO: 52, and a CD3-HCDR3 comprising the amino acid sequence of SEQ ID NO: 53; and the CD3-VL comprises: a CD3-LCDR1 comprising the amino acid sequence of SEQ ID NO: 54, a CD3-LCDR2 comprising the amino acid sequence of SEQ ID NO: 55, and a CD3-LCDR3 comprising the amino acid sequence of SEQ ID NO: 56;

the buffer is a histidine buffer, an acetate buffer, a citrate buffer, a succinate buffer, or a phosphate buffer; preferably, the buffer is a histidine-histidine hydrochloride buffer or an acetic acid-sodium acetate buffer; more preferably, the buffer is a histidine-histidine hydrochloride buffer.

2. The pharmaceutical composition according to claim 1, wherein the pharmaceutical composition has a pH of 5.0 to 7.0; preferably, the pharmaceutical composition has a pH of 5.0 to 6.5; further preferably, the pharmaceutical composition has a pH of 5.0 to 6.0; more preferably, the pharmaceutical composition has a pH of 5.5 to 6.0; most preferably, the pharmaceutical composition has a pH of about 5.5.

3. The pharmaceutical composition according to claim 1 or 2, wherein the bispecific antibody specifically binding to GPRC5D and CD3 is at a concentration of 1 mg/mL to 250 mg/mL;

preferably, the bispecific antibody specifically binding to GPRC5D and CD3 is at a concentration of 1 mg/mL to 200 mg/mL;

further preferably, the bispecific antibody specifically binding to GPRC5D and CD3 is at a concentration of 1 mg/mL to 150 mg/mL;

more preferably, the bispecific antibody specifically binding to GPRC5D and CD3 is at a concentration of 60 mg/mL to 150 mg/mL;

still further preferably, the bispecific antibody specifically binding to GPRC5D and CD3 is at a concentration of 80 mg/mL to 120 mg/mL;

most preferably, the bispecific antibody specifically binding to GPRC5D and CD3 is at a concentration of about 2 mg/mL, about 60 mg/mL, or about 100 mg/mL.

4. The pharmaceutical composition according to any one of claims 1 to 3, wherein the pharmaceutical composition comprises a surfactant; preferably, the surfactant is polysorbate or poloxamer; more preferably, the surfactant is polysorbate 80 or poloxamer 188; most preferably, the surfactant is polysorbate 80.

5. The pharmaceutical composition according to claim 4, wherein the surfactant is at a concentration of 0.01 mg/mL to 1.0 mg/mL; preferably, the surfactant is at a concentration of 0.1 mg/mL to 0.8 mg/mL; further preferably, the surfactant is at a concentration of 0.2 mg/mL to 0.6 mg/mL; more preferably, the surfactant is at a concentration of 0.32 mg/mL to 0.48 mg/mL; most preferably, the surfactant is at a concentration of about 0.4 mg/mL.

6. The pharmaceutical composition according to any one of claims 1 to 5, wherein the pharmaceutical composition comprises a sugar; preferably, the sugar is sucrose, trehalose, mannitol, or sorbitol; more preferably, the sugar is sucrose.

7. The pharmaceutical composition according to claim 6, wherein the sugar is at a concentration of 10 mg/mL to 100 mg/mL; preferably, the sugar is at a concentration of 30 mg/mL to 100 mg/mL; more preferably, the sugar is at a concentration of 64 mg/mL to 96 mg/mL; most preferably, the sugar is at a concentration of about 80 mg/mL.

8. The pharmaceutical composition according to any one of claims 1 to 7, wherein the buffer is at a concentration of 5 mM to 100 mM; preferably, the buffer is at a concentration of 5 mM to 50 mM; further preferably, the buffer is at a concentration of 5 mM to 30 mM; more preferably, the buffer is at a concentration of 8 mM to 20 mM; most preferably, the buffer is at a concentration of about 14 mM or about 20 mM.

9. The pharmaceutical composition according to any one of claims 1 to 8, wherein the pharmaceutical composition further comprises an auxiliary material; preferably, the auxiliary material is an ethylenediaminetetraacetic acid disodium salt or a hydrate thereof, arginine hydrochloride, glycine, methionine, proline, histidine, phenylalanine, glutamic acid, aspartic acid, sodium chloride, or calcium chloride; more preferably, the auxiliary material is ethylenediaminetetraacetic acid disodium salt dihydrate.

10. The pharmaceutical composition according to claim 9, wherein the auxiliary material is at a concentration of 0.01 mg/mL to 1 mg/mL; preferably, the auxiliary material is at a concentration of 0.01 mg/mL to 0.2 mg/mL; more preferably, the auxiliary material is at a concentration of 0.08 mg/mL to 0.12 mg/mL; most preferably, the auxiliary material is at a concentration of about 0.1 mg/mL.

11. The pharmaceutical composition according to any one of claims 1 to 10, wherein in the bispecific antibody specifically binding to GPRC5D and CD3,

(i) the GPRC5D-VH comprises: a GPRC5D-HCDR1 comprising the amino acid sequence of SEQ ID NO: 11, a GPRC5D-HCDR2 comprising the amino acid sequence of SEQ ID NO: 12, and a GPRC5D-HCDR3 comprising the amino acid sequence of SEQ ID NO: 13; and the GPRC5D-VL comprises: a GPRC5D-LCDR1 comprising the amino acid sequence of SEQ ID NO: 43, a GPRC5D-LCDR2 comprising the amino acid sequence of SEQ ID NO: 15, and a GPRC5D-LCDR3 comprising the amino acid sequence of SEQ ID NO: 16; or

the GPRC5D-VH comprises: a GPRC5D-HCDR1 comprising the amino acid sequence of SEQ ID NO: 5, a GPRC5D-HCDR2 comprising the amino acid sequence of SEQ ID NO: 31, and a GPRC5D-HCDR3 comprising the amino acid sequence of SEQ ID NO: 7;
and the GPRC5D-VL comprises: a GPRC5D-LCDR1 comprising the amino acid sequence of SEQ ID NO: 27, a GPRC5D-LCDR2 comprising the amino acid sequence of SEQ ID NO: 9, and a GPRC5D-LCDR3 comprising the amino acid sequence of SEQ ID NO: 10; and

(ii) the CD3-VH comprises: a CD3-HCDR1 comprising the amino acid sequence of SEQ ID NO: 51, a CD3-HCDR2 comprising the amino acid sequence of SEQ ID NO: 52, and a CD3-HCDR3 comprising the amino acid sequence of SEQ ID NO: 53; and the CD3-VL comprises: a CD3-LCDR1 comprising the amino acid sequence of SEQ ID NO: 54, a CD3-LCDR2 comprising the amino acid sequence of SEQ ID NO: 55, and a CD3-LCDR3 comprising the amino acid sequence of SEQ ID NO: 56.

12. The pharmaceutical composition according to claim 11, wherein in the bispecific antibody specifically binding to GPRC5D and CD3:

(i) the GPRC5D-VH comprises the amino acid sequence of SEQ ID NO: 34, 32, or 33, or an amino acid sequence having at least 90% sequence identity to SEQ ID NO: 34, 32, or 33, and the GPRC5D-VL comprises the amino acid sequence of SEQ ID NO: 39, 35, 36, 37, 38, or 40, or an amino acid sequence having at least 90% sequence identity to SEQ ID NO: 39, 35, 36, 37, 38, or 40; or

the GPRC5D-VH comprises the amino acid sequence of SEQ ID NO: 3 or an amino acid sequence having at least 90% sequence identity to SEQ ID NO: 3, and the GPRC5D-VL comprises the amino acid sequence of SEQ ID NO: 4 or an amino acid sequence having at least 90% sequence identity to SEQ ID NO: 4; the CD3-VH comprises the amino acid sequence of SEQ ID NO: 57 or an amino acid sequence having at least 90% sequence identity to SEQ ID NO: 57, and the CD3-VL comprises the amino acid sequence of SEQ ID NO: 58 or an amino acid sequence having at least 90% sequence identity to SEQ ID NO: 58; or

(ii) the GPRC5D-VH comprises the amino acid sequence of SEQ ID NO: 19, 17, or 18, or an amino acid sequence having at least 90% sequence identity to SEQ ID NO: 19, 17, or 18, and the GPRC5D-VL comprises the amino acid sequence of SEQ ID NO: 23, 20, 21, 22, 24, or 25, or an amino acid sequence having at least 90% sequence identity to SEQ ID NO: 23, 20, 21, 22, 24, or 25; or

the GPRC5D-VH comprises the amino acid sequence of SEQ ID NO: 1 or an amino acid sequence having at least 90% sequence identity to SEQ ID NO: 1, and the GPRC5D-VL comprises the amino acid sequence of SEQ ID NO: 2 or an amino acid sequence having at least 90% sequence identity to SEQ ID NO: 2; and the CD3-VH comprises the amino acid sequence of SEQ ID NO: 57 or an amino acid sequence having at least 90% sequence identity to SEQ ID NO: 57, and the CD3-VL comprises the amino acid sequence of SEQ ID NO: 58 or an amino acid sequence having at least 90% sequence identity to SEQ ID NO: 58; preferably,

(i) the GPRC5D-VH comprises the amino acid sequence of SEQ ID NO: 34, and the GPRC5D-VL comprises the amino acid sequence of SEQ ID NO: 39; and the CD3-VH comprises the amino acid sequence of SEQ ID NO: 57, and the CD3-VL comprises the amino acid sequence of SEQ ID NO: 58; or
(ii) the GPRC5D-VH comprises the amino acid sequence of SEQ ID NO: 19, and the GPRC5D-VL comprises the amino acid sequence of SEQ ID NO: 23; and the CD3-VH comprises the amino acid sequence of SEQ ID NO: 57, and the CD3-VL comprises the amino acid sequence of SEQ ID NO: 58.

13. The pharmaceutical composition according to any one of claims 1 to 12, wherein the antigen-binding moiety specifically binding to GPRC5D is a Fab, and the antigen-binding moiety specifically binding to CD3 is a substituted Fab; the substituted Fab results from substitution of the original CH1 and CL of a Fab with an Obscurin chain and a Titin chain, respectively;

preferably, the Obscurin chain comprises the amino acid sequence of SEQ ID NO: 83, and the Titin chain comprises the amino acid sequence of SEQ ID NO: 84.

14. The pharmaceutical composition according to any one of claims 1 to 13, wherein the bispecific antibody specifically binding to GPRC5D and CD3 comprises one first chain set forth in SEQ ID NO: 61, one second chain set forth in SEQ ID NO: 60, one third chain set forth in SEQ ID NO: 67, and one fourth chain set forth in SEQ ID NO: 68; or comprises one first chain set forth in SEQ ID NO: 65, one second chain set forth in SEQ ID NO: 66, one third chain set forth in SEQ ID NO: 59, and one fourth chain set forth in SEQ ID NO: 60.

15. The pharmaceutical composition according to any one of claims 1 to 14, comprising the following components:

(a) 1 mg/mL to 250 mg/mL said bispecific antibody specifically binding to GPRC5D and CD3,
(b) 0.01 mg/mL to 1.0 mg/mL surfactant,
(c) 10 mg/mL to 100 mg/mL sugar,
(d) 0.01 mg/mL to 1 mg/mL ethylenediaminetetraacetic acid disodium salt or a hydrate thereof, and
(e) 5 mM to 100 mM buffer, the pharmaceutical composition having a pH of 5.0 to 6.5;
preferably, the pharmaceutical composition comprises the following components:

(a) 1 mg/mL to 200 mg/mL said bispecific antibody specifically binding to GPRC5D and CD3,
(b) 0.1 mg/mL to 0.8 mg/mL polysorbate 80,
(c) 30 mg/mL to 100 mg/mL sucrose,
(d) 0.01 mg/mL to 1 mg/mL ethylenediaminetetraacetic acid disodium salt dihydrate, and
(e) 5 mM to 50 mM histidine-histidine hydrochloride buffer or acetic acid-sodium acetate buffer, the pharmaceutical composition having a pH of 5.0 to 6.0;

further preferably, the pharmaceutical composition comprises the following components:

(a) 60 mg/mL to 150 mg/mL said bispecific antibody specifically binding to GPRC5D and CD3,
(b) 0.2 mg/mL to 0.6 mg/mL polysorbate 80,
(c) 64 mg/mL to 96 mg/mL sucrose,
(d) 0.01 mg/mL to 0.2 mg/mL ethylenediaminetetraacetic acid disodium salt dihydrate, and
(e) 5 mM to 30 mM histidine-histidine hydrochloride buffer or acetic acid-sodium acetate buffer, the pharmaceutical composition having a pH of 5.0 to 6.0;

more preferably, the pharmaceutical composition comprises the following components:

(a) 80 mg/mL to 120 mg/mL said bispecific antibody specifically binding to GPRC5D and CD3,
(b) 0.32 mg/mL to 0.48 mg/mL polysorbate 80,
(c) 64 mg/mL to 96 mg/mL sucrose,
(d) 0.08 mg/mL to 0.12 mg/mL ethylenediaminetetraacetic acid disodium salt dihydrate, and
(e) 8 mM to 20 mM histidine-histidine hydrochloride buffer, the pharmaceutical composition having a pH of 5.5 to 6.0;

most preferably, the pharmaceutical composition comprises the following components:

(a) about 2 mg/mL, or about 60 mg/mL, or about 100 mg/mL said bispecific antibody specifically binding to GPRC5D and CD3,
(b) about 0.4 mg/mL polysorbate 80,
(c) about 80 mg/mL sucrose,
(d) about 0.1 mg/mL ethylenediaminetetraacetic acid disodium salt dihydrate, and
(e) about 20 mM histidine-histidine hydrochloride buffer, the pharmaceutical composition having a pH of about 5.5; or
(a) about 2 mg/mL, or about 60 mg/mL, or about 100 mg/mL said bispecific antibody specifically binding to GPRC5D and CD3,
(b) about 0.4 mg/mL polysorbate 80,
(c) about 80 mg/mL sucrose,
(d) about 0.1 mg/mL ethylenediaminetetraacetic acid disodium salt dihydrate, and
(e) about 14 mM histidine-histidine hydrochloride buffer, the pharmaceutical composition having a pH of about 5.5.

16. A freeze-dried formulation, wherein the freeze-dried formulation is capable of forming the pharmaceutical composition according to any one of claims 1 to 15 after being reconstituted.

17. A method for preparing a freeze-dried formulation, comprising a step of lyophilizing the pharmaceutical composition according to any one of claims 1 to 15.

18. A freeze-dried formulation, wherein the formulation is obtained by the method according to claim 17.

19. The pharmaceutical composition according to any one of claims 1 to 15, wherein the pharmaceutical composition is a formulation for intravenous, subcutaneous, intraperitoneal, or intramuscular injection, preferably a formulation for subcutaneous injection.

20. A method for treating or preventing a disease, comprising administering to a subject a therapeutically effective amount of the pharmaceutical composition according to any one of claims 1 to 15 or the freeze-dried formulation according to claim 16 or 18, wherein preferably, the disease is selected from the group consisting of a tumor, a cancer, and an autoimmune disease;
more preferably, the disease is selected from the group consisting of multiple myeloma, relapsed/refractory multiple myeloma, smoldering multiple myeloma, monoclonal gammopathy, acute lymphoblastic leukemia, diffuse large B-cell lymphoma, Burkitt's lymphoma, follicular lymphoma, mantle cell lymphoma, Waldenstrom's macroglobulinemia, plasma cell leukemia, light chain amyloidosis, precursor B-cell lymphoblastic leukemia, acute myelogenous leukemia, myelodysplastic syndrome, chronic lymphocytic leukemia, B-cell malignancy, chronic myelogenous leukemia, hairy cell leukemia, blastic plasmacytoid dendritic cell neoplasm, Hodgkin's lymphoma, non-Hodgkin's lymphoma, marginal zone B-cell lymphoma, mucosa-associated lymphoid tissue lymphoma, plasma cell leukemia, anaplastic large cell lymphoma, ovarian cancer, lung cancer, gastric cancer, prostate cancer, renal cancer, liver cancer, colorectal cancer, esophageal cancer, bladder cancer, cervical cancer, and melanoma.

FIG. 1

FIG. 2

## INTERNATIONAL SEARCH REPORT

| International application No. |
| --- |
| **PCT/CN2024/118675** |

**A. CLASSIFICATION OF SUBJECT MATTER**

C07K16/28(2006.01)i; A61K39/395(2006.01)i

According to International Patent Classification (IPC) or to both national classification and IPC

**B. FIELDS SEARCHED**

Minimum documentation searched (classification system followed by classification symbols)

IPC: C07K.A61K

Documentation searched other than minimum documentation to the extent that such documents are included in the fields searched

Electronic data base consulted during the international search (name of data base and, where practicable, search terms used)

CNTXT, DWPI, ENTXT, ENTXTC, VEN, WPABS, WPABSC, PUBMED, ISI WEB OF SCIENCE, GenBank, STN: 江苏恒瑞医药股份有限公司, VH, VL, CDR, cancer, tumor, 肿瘤, 癌症, SEQ ID NOs: 1-56, 抗体, antibody, CD3, GPRC5D, titin, obscurin, 多发性骨髓瘤, MM

**C. DOCUMENTS CONSIDERED TO BE RELEVANT**

| Category* | Citation of document, with indication, where appropriate, of the relevant passages | Relevant to claim No. |
| --- | --- | --- |
| A | CN 109715667 A (JANSSEN PHARMACEUTICA NV) 03 May 2019 (2019-05-03) claims 1-65, and description, paragraphs 12-18, 22-25 and 47 | 1-20 |
| A | WO 2020114478 A1 (JIANGSU HENGRUI MEDICINE CO., LTD. et al.) 11 June 2020 (2020-06-11) claims 1-15, description, page 5, lines 2-20, and SEQ ID NOs. 31 and 36 | 1-20 |
| A | WO 2022174813 A1 (INNOVENT BIOLOGICS (SUZHOU) CO., LTD.) 25 August 2022 (2022-08-25) claims 1-26 | 1-20 |
| A | WO 2023143537 A1 (CARSGEN LIFE SCIENCES CO., LTD.) 03 August 2023 (2023-08-03) abstract | 1-20 |
| A | HOSNY, M. et al. "Current State of the Art and Prospects of T Cell-Redirecting Bispecific Antibodies in Multiple Myeloma" *Journal of Clinical Medicine*, Vol. vol. 10, 06 October 2021 (2021-10-06), pp. 1-19 | 1-20 |

☑ Further documents are listed in the continuation of Box C.     ☑ See patent family annex.

| | |
| --- | --- |
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **17 December 2024** | **23 December 2024** |

| Name and mailing address of the ISA/CN | Authorized officer |
| --- | --- |
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2024/118675** |

### C. DOCUMENTS CONSIDERED TO BE RELEVANT

| Category* | Citation of document, with indication, where appropriate, of the relevant passages | Relevant to claim No. |
|---|---|---|
| A | MANCIA, S.S. et al. "Characterization and Management of Oral and Dermatological Toxicities in Patients Receiving the CD3 X GPRC5D Bispecific Antibody Talquetamab (JNJ-64407564) for the Treatment of Relapsed and/or Refractory Multiple Myeloma" *Blood*, Vol. vol. 138, No. Supplement 1, 05 November 2021 (2021-11-05), abstract | 1-20 |
| A | KODAMA, T. et al. "Anti-GPRC5D/CD3 Bispecific T-Cell-Redirecting Antibody for the Treatment of Multiple Myeloma" *MOLECULAR CANCER THERAPEUTICS,* Vol. 18, No. (9), 30 September 2019 (2019-09-30), pp. 1555-1564 | 1-20 |

Form PCT/ISA/210 (second sheet) (July 2022)

**INTERNATIONAL SEARCH REPORT**

International application No.

**PCT/CN2024/118675**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

| International application No. |
| --- |
| **PCT/CN2024/118675** |

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **20**
   because they relate to subject matter not required to be searched by this Authority, namely:

   Claim 20 relates to a method for preventing or treating a disease, that is, claim 20 relates to subject matter for which no search is required by the International Searching Authority as defined in PCT Rule 39.1: (iv) – methods for treatment of the human or animal body by surgery or therapy, as well as diagnostic methods. However, a search is still carried out on the basis of the pharmaceutical use thereof.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
|---|
| **PCT/CN2024/118675** |

| Patent document cited in search report | | | Publication date (day/month/year) | Patent family member(s) | | | Publication date (day/month/year) |
|---|---|---|---|---|---|---|---|
| CN | 109715667 | A | 03 May 2019 | CR | 20190025 | A | 14 March 2019 |
| | | | | AR | 109499 | A1 | 19 December 2018 |
| | | | | CL | 2024000444 | A1 | 23 August 2024 |
| | | | | JP | 2024054176 | A | 16 April 2024 |
| | | | | ZA | 201901066 | B | 28 October 2020 |
| | | | | BR | 112019001055 | A2 | 01 October 2019 |
| | | | | NI | 201900005 | A | 10 May 2019 |
| | | | | TW | 201809005 | A | 16 March 2018 |
| | | | | TWI | 781108 | B | 21 October 2022 |
| | | | | UY | 37340 | A | 31 January 2018 |
| | | | | KR | 20190028534 | A | 18 March 2019 |
| | | | | KR | 102572091 | B1 | 31 August 2023 |
| | | | | MX | 2024001615 | A | 28 February 2024 |
| | | | | EP | 3487882 | A2 | 29 May 2019 |
| | | | | ECSP | 19012075 | A | 28 February 2019 |
| | | | | PH | 12019500152 | A1 | 14 October 2019 |
| | | | | US | 2023227548 | A1 | 20 July 2023 |
| | | | | US | 11884722 | B2 | 30 January 2024 |
| | | | | MX | 2023006449 | A | 15 June 2023 |
| | | | | US | 2023084967 | A1 | 16 March 2023 |
| | | | | US | 11685777 | B2 | 27 June 2023 |
| | | | | KR | 20230128409 | A | 04 September 2023 |
| | | | | CA | 3031472 | A1 | 25 January 2018 |
| | | | | CL | 2019000146 | A1 | 26 April 2019 |
| | | | | EA | 201990346 | A1 | 28 June 2019 |
| | | | | SG | 11201900468 | YA | 27 February 2019 |
| | | | | AU | 2017299673 | A1 | 07 February 2019 |
| | | | | AU | 2017299673 | B2 | 01 August 2024 |
| | | | | IL | 264334 | A | 28 February 2019 |
| | | | | IL | 264334 | B1 | 01 January 2024 |
| | | | | IL | 264334 | B2 | 01 May 2024 |
| | | | | MA | 45712 | A | 29 May 2019 |
| | | | | IL | 298277 | A | 01 January 2023 |
| | | | | US | 2024343796 | A1 | 17 October 2024 |
| | | | | WO | 2018017786 | A2 | 25 January 2018 |
| | | | | WO | 2018017786 | A3 | 01 March 2018 |
| | | | | AU | 2024205021 | A1 | 15 August 2024 |
| | | | | JP | 2019527061 | A | 26 September 2019 |
| | | | | JP | 7292200 | B2 | 16 June 2023 |
| | | | | JP | 2023011774 | A | 24 January 2023 |
| | | | | JP | 7469432 | B2 | 16 April 2024 |
| | | | | US | 2018037651 | A1 | 08 February 2018 |
| | | | | US | 10562968 | B2 | 18 February 2020 |
| | | | | PE | 20190392 | A1 | 13 March 2019 |
| | | | | CO | 2019001114 | A2 | 19 February 2019 |
| | | | | MX | 2019000825 | A | 04 September 2019 |
| | | | | PE | 20240884 | A1 | 24 April 2024 |
| WO | 2020114478 | A1 | 11 June 2020 | None | | | |
| WO | 2022174813 | A1 | 25 August 2022 | JP | 2024510098 | A | 06 March 2024 |
| | | | | CA | 3211407 | A1 | 25 August 2022 |

Form PCT/ISA/210 (patent family annex) (July 2022)

International application No.

**PCT/CN2024/118675**

| Patent document cited in search report | Publication date (day/month/year) | Patent family member(s) | | Publication date (day/month/year) |
|---|---|---|---|---|
| | | TW | 202237661 A | 01 October 2022 |
| | | TWI | 830151 B | 21 January 2024 |
| | | AU | 2022223152 A1 | 31 August 2023 |
| | | EP | 4296286 A1 | 27 December 2023 |
| WO 2023143537 A1 | 03 August 2023 | EP | 4471065 A1 | 04 December 2024 |
| | | WO | 2023143537 A9 | 20 June 2024 |
| | | KR | 20240137090 A | 19 September 2024 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2023081237 W
- CN 202210245308
- CN 2019123548 W
- WO 2018017786 A

**Non-patent literature cited in the description**

- *J. Biol. Chem.*, 1968, vol. 243, 3558
- **ARAN F. LABRIJN et al.** *Nature Reviews Drug Discovery*, 2019, vol. 18, 585-608
- **CHEN S1 et al.** *J Immunol Res.*, 11 February 2019, vol. 2019, 4516041
- **KABAT et al.** Sequences of Proteins of Immunological Interest. Public Health Service, National Institutes of Health, 1991
- Protein Sequence and Structure Analysis of Antibody Variable Domains[J. **MARTIN**. ACR. 2001
- **LEFRANC, M.P. et al.** *Dev. Comp. Immunol*, 2003, vol. 27, 55-77
- *Front Immunol*, 16 October 2018, vol. 9, 2278
- *CHEMICAL ABSTRACTS*, 6381-92-6